# EUROPEAN PATENT SPECIFICATION

(11) **EP 1 993 673 B1**
(45) Date of publication and mention of the grant of the patent: **28.06.2017**
(21) Application number: 07718112.1
(22) Date of filing: 19.01.2007
(51) Int. Cl.: A61P 27/02, A61K 38/08, A61K 38/10, A61K 38/12, A61K 9/00, A61F 9/00, A61K 39/00, A61K 38/00, A61K 49/00, A61K 47/06, A61K 39/395, A61K 31/00, A61K 38/17

(54) **INJECTABLE COMBINATION THERAPY FOR EYE DISORDERS**
INJIZIERBARE KOMBINATIONSTHERAPIE FÜR AUGENERKRANKUNGEN
TRAITEMENT COMBINATOIRE INJECTABLE DESTINE A DES TROUBLES OPHTALMIQUES

(30) Priority: 19.01.2006 US 760974 P; 06.10.2006 US 544389
(43) Date of publication of application: 26.11.2008
(73) Proprietor: Potentia Pharmaceuticals, Inc., Louisville KY 40202 (US)
(72) Inventor: DESCHATELETS, Pascal, Louisville, Kentucky 40241 (US); OLSON, Paul, Louisville, KY 40204 (US); FRANCOIS, Cedric, Louisville, kentucky 40202 (US)
(74) Representative: Lee, Nicholas John
(86) International application number: PCT/US2007/001649
(87) International publication number: WO 2007/084765

(56) References cited:
- WO-A-96/30046
- US-A- 6 051 698
- US-A1- 2005 281 861
- US-B1- 6 319 897
- "Anti-VEGF Agents Useful in Age-Related Macular Degeneration" INTERNET CITATION, [Online] 15 October 2005 (2005-10-15), XP002414529 Retrieved from the Internet: URL:http://www.rsinewsrxreportingfrom.com/ content.asp?myid=40&tid=365> [retrieved on 2007-01-12]
- MICHELS S ET AL: "TREATMENT OF NEOVASCULAR AGE-RELATED MACULAR DEGENERATION WITH RANIBIZUMAB/LUCENTIS THERAPIE DER NEOVASKULAEREN ALTERSBEDINGTEN MAKULADEGENERATION MIT RANIBIZUMAB/LUCENTIS" KLINISCHE MONATSBLAETTER FUER AUGENHEILKUNDE, FERDINAND ENKE VERLAG, STUTTGART, DE, vol. 222, no. 6, June 2005 (2005-06), pages 480-484, XP009078844 ISSN: 0023-2165
- NG EUGENE W M ET AL: "TARGETING ANGIOGENESIS, THE UNDERLYING DISORDER IN NEOVASCULAR AGE-RELATED MACULAR DEGENERATION" CANADIAN JOURNAL OF OPHTHALMOLOGY, XX, XX, vol. 40, no. 3, June 2005 (2005-06), pages 352-368, XP009078843 ISSN: 0008-4182 cited in the application
- MURAT V KALAYOGLU: "Emerging Treatment Strategies For Age-Related Macular Degeneration" INTERNET CITATION, [Online] 30 June 2004 (2004-06-30), XP002414528 Retrieved from the Internet: URL:http://www.medcompare.com/spotlight.as p?spotlightid=62> [retrieved on 2007-01-10]
- "Anti-vascular endothelial growth factor therapy for subfoveal choroidal neovascularization secondary to age-related macular degeneration: Phase II study results" OPHTHALMOLOGY, J. B. LIPPINCOTT CO., PHILADELPHIA, PA, US, vol. 110, no. 5, May 2003 (2003-05), pages 979-986, XP002366840 ISSN: 0161-6420
- GRAGOUDAS EVANGELOS S ET AL: "Pegaptanib for neovascular age-related macular degeneration." THE NEW ENGLAND JOURNAL OF MEDICINE 30 DEC 2004, vol. 351, no. 27, 30 December 2004 (2004-12-30), pages 2805-2816, XP002472472 ISSN: 1533-4406
- AMBATI J ET AL: "Age-related macular degeneration: Etiology, pathogenesis, and therapeutic strategies" SURVEY OF OPHTHALMOLOGY, SURVEY OF OPHTHALMOLOGY INC, XX, vol. 48, no. 3, 2003, pages 257-293, XP002341326 ISSN: 0039-6257
- ROSENFELD P: "An Update on Bevacizumab" INTERNET CITATION, [Online] 1 December 2005 (2005-12-01), XP002414532 Retrieved from the Internet: URL:http://www.revophth.com/index.asp?page =1_857.htm> [retrieved on 2007-01-10]
- MAKRIDES S C: "THERAPEUTIC INHIBITION OF THE COMPLEMENT SYSTEM" PHARMACOLOGICAL REVIEWS, WILLIAMS AND WILKINS INC., BALTIMORE, MD,, US, vol. 50, no. 1, 1998, pages 59-87, XP001055179 ISSN: 0031-6997
- JOHNSON L V ET AL: "Complement activation and inflammatory processes in drusen formation and age related macular degeneration" EXPERIMENTAL EYE RESEARCH, ACADEMIC PRESS LTD., LONDON, GB, vol. 73, no. 6, December 2001 (2001-12), pages 887-896, XP002386744 ISSN: 0014-4835
- DATABASE BIOSIS [Online] BIOSCIENCES INFORMATION SERVICE, PHILADELPHIA, PA, US; 2005, RAISLER B J ET AL: "Drusen complement components C3a and C5a promote choroidal neovascularization" XP002472473 Database accession no. PREV200600053073 & IOVS, vol. 46, no. Suppl. S, 2005, page 1214, ANNUAL MEETING OF THE ASSOCIATION-FOR-RESEARCH-IN-VISION-AND-OPH THALM OLOGY; FT LAUDERDALE, FL, USA; MAY 01 -05, 2005 ISSN: 0146-0404
- MORIKIS O ET AL: "Improvement of the anti-C3 activity of compstatin using rational and combinatorial approaches" BIOCHEMICAL SOCIETY TRANSACTIONS, COLCHESTER, ESSEX, GB, vol. 32, no. 1, February 2004 (2004-02), pages 28-32, XP002455138 ISSN: 0300-5127

## Description

### Background of the Invention

Macular degeneration is a term that refers to and describes a number of different diseases characterized by degenerative changes in the macula, all of which lead to a loss of central vision. The macula is a small area in the retina of the eye, approximately 3 to 5 millimeters in size, adjacent to the optic nerve. It is the most sensitive area of the retina and contains the fovea, a depressed region that allows for high visual acuity and contains a dense concentration of cones, the photoreceptors that are responsible for color vision. Age-related macular degeneration (ARMD) is the most common cause of functional blindness in developed countries for those over 50 years of age. The disease is characterized by progressive degeneration of the retina, retinal pigment epithelium (RPE), and underlying choroid (the highly vascular tissue that lies beneath the RPE, between the retina and the sclera). Cells in the RPE recycle visual pigment (rhodopsin), phagocytose photoreceptor tips daily as part of rod and cone regeneration, and transport fluid across the membrane to the choroid. Central vision deteriorates when cells in the RPE cease to function properly. Despite extensive investigation, the pathogenesis of ARMD is not fully understood. Oxidative stress, inflammation, genetic background, and environmental or behavioral factors such as smoking and diet may contribute.

A clinical hallmark of ARMD is the appearance of drusen, localized deposits of lipoproteinaceous material that accumulate in the space between the RPE and Brunch's membrane. Drusen are typically the earliest clinical finding in ARMD, and the existence, location, and number of drusen are used in classifying the disease into stages and monitoring progression (Ambati, J., et al., Surv. Ophthalmol., 48(3): 257-293, 2003; "Preferred Practice Pattern: Age-Related Macular Degeneration", American Academy of Ophthalmology, 2003).

ARMD has been classified into "dry" and "wet" (exudative, or neavascular) forms. Dry ARMD is much more common than wet, but the dry form can progress to the wet form, and the two occur simultaneously in a significant number of cases. Dry ARMD is typically characterized by progressive apoptosis of cells in the RPE, overlying photoreceptor cells, and frequently also the underlying cells in the choroidal capillary layer. Confluent areas (e.g., at least 175 µm in minimum diameter) of RPE cell death accompanied by overlying photoreceptor atrophy are referred to as geographic atrophy. Patients with this form experience a slow and progressive deterioration in central vision. Wet ARMD is characterized by bleeding and/or leakage of fluid from abnormal vessels that have grown from the choroidal vessels (choriocapillaris) beneath the RPE and macula. This can be responsible for sudden and disabling vision loss. Much of the vision loss that patients experience is due to such choroidal neovascularization (CNV) and its complications. A subtype of neovascular ARMD in which angiomatous proliferation originates from the retina and extends posteriorly into the subretinal space, eventually communicating in some cases with choroidal new vessels has been identified (Yannuzzi, L.A., et al., Retina, 21(5):416-34, 2001). This form ofneovascular ARMD, termed retinal angiomatous proliferation (RAP) can be particularly severe. The existence of macular drusen is a strong risk factor for development of both wet and dry forms of ARMD.

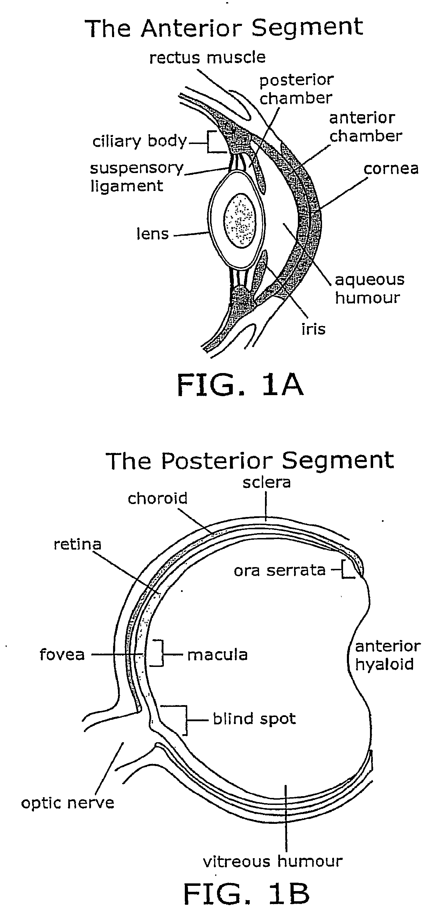

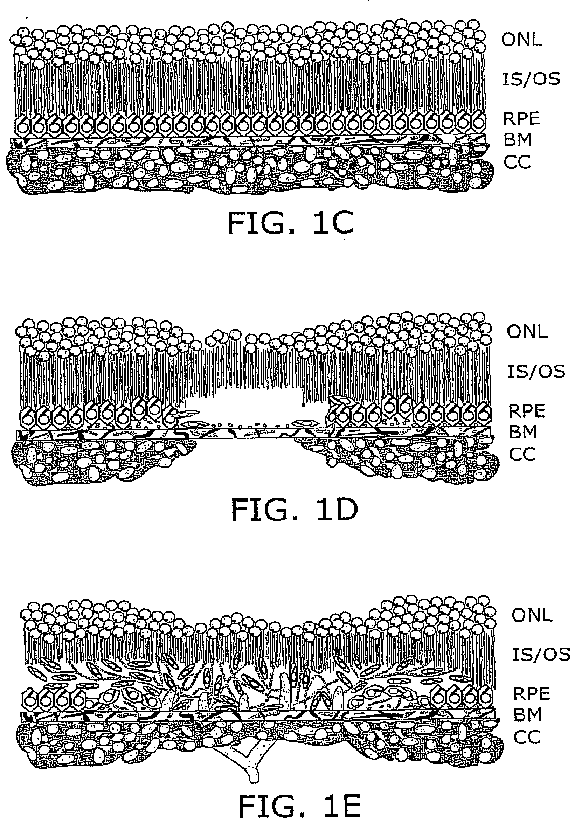

The panels of Figure 1 show structures present in a normal eye and some of the processes that occur in ARMD. Figures 1A and 1B show structures present in the anterior and posterior segments of the eye. Figures 1C-1E depict the outer layers of a normal eye (1C), an eye suffering from dry ARMD (1D), and an eye suffering from wet ARMD (1E). The outer nuclear layer (ONL) contains nuclei of rod and cone photoreceptors. Each photoreceptor contains an inner segment (IS) and outer segment (OS), the latter of which contains the pigment rhodopsin, which initiates the phototransduction cascade following exposure to light. The RPE lies below the photoreceptors and above Bruch's membrane. As shown in Figures 1D and 1E, the normal structure of the retina is disrupted in a variety of ways as in patients with ARMD.

Macular edema is associated with a variety of eye disorders including ARMD, diabetic retinopathy, inflammatory conditions such as anterior or posterior uveitis, etc. The macula becomes thickened as a result of the accumulation of fluid that leaks from weakened or otherwise abnormal blood vessels into nearby tissues. Leakage of blood or other fluids and the resulting increase in macular thickness can lead to acute alterations in visual acuity, color perception, etc. Thus macular edema can contribute to the visual disturbances and loss experienced by individuals suffering from ARMD and a variety of other eye disorders.

Development of pharmacological therapies for ARMD and other ocular disorders associated with neovascularization in the eye is an area of active investigation. Much effort has focused on methods for destroying or sealing abnormal blood vessels and/or inhibiting their development. Photodynamic therapy involves systemic intravenous administration of a light-sensitive dye (verteporfin) which is activated in the eye by a laser, resulting in formation of toxic products within the abnormal blood vessels. Local administration of angiogenesis inhibitors to the eye shows considerable promise. Pegaptanib sodium (Macugen®; Pfizer/Eyetech) was approved by the U.S. Food and Drug Administration for treatment of wet age-related macular degeneration in late 2004. Macugen is an aptamer that binds to an isoform of vascular endothelial growth factor (VEGF), a protein that acts as a signal in triggering the abnormal blood vessel growth, increased permeability, and consequent leakage that characterize wet ARMD. Binding of Macugen to VEGF prevents it from binding to VEGF receptors, thereby inhibiting its activity. Other angiogenesis inhibitors for the treatment of exudative ARMD include monoclonal antibodies such as ranibizumab (Lucentis®; Genentech) that bind to VEGF and block its interaction with VEGF receptors.

Angiogenesis inhibitors that interfere with signal transduction pathways that play a fundamental role in angiogenesis, such as the VEGF pathway, offer a powerful approach to controlling neovascularization. However, therapy with angiogenesis inhibitors alone has a number of disadvantages. Clinical trials of angiogenesis inhibitors that interfere with the VEGF pathway have involved their administration in solution by intravitreal injection at intervals of 4-6 weeks. Unfortunately this procedure is associated with a significant risk of complications such as traumatic lens injury, retinal detachment, and endophalmitis associated with either trauma or intraocular infection. With an overall risk of 1%, over the course of a year a dosing interval of 6 weeks would result in an overall risk of about 9% per eye, while a dosing interval of 4 weeks would result in an overall risk of about 13% per eye. For these and other reasons, current approaches to the use of angiogenesis inhibitors remain a less than optimal solution to treating wet ARMD. There remains a need in the art for improved approaches to treating ARMD. There also remains a need for improved approaches to treating other conditions characterized by macular degeneration, choroidal neovascularization, retinal neovascularization, retinal angiomatous proliferation, and/or blood vessel leakage in the eye.

### Summary of the Invention

The invention is as defined in the claims. In a first aspect the present invention provides first and second therapeutic agents for use in treating an eye disorder characterized by macular degeneration, choroidal neovascularisation or retinal neovascularisation, wherein
effective amounts of the first and second therapeutic agents are administered to the subject's eye, optionally in a single procedure;
the first therapeutic agent is an anti-VEGF agent selected from the group consisting of antibodies or antibody fragments that bind to VEGF, a fusion protein containing extracellular domains of two VEGF receptors connected to the Fc region of an antibody, and nucleic acids that bind to VEGF; and
the second therapeutic agent is selected from the group consisting of a compstatin analog having at least 5-fold higher complement inhibiting activity than compstatin, a monoclonal antibody or monoclonal antibody fragment that binds to C3 and an antibody or antibody fragment that binds to C5, factor B or factor D.

Certain preferred features of the invention are set out in the dependent claims herein. In certain embodiments of the invention at least a portion of the first therapeutic agent, optionally essentially the entire administered dose of the first therapeutic agent, is provided as a component of a sustained release formulation. The first and second therapeutic agents may be provided as components of a single sustained release formulation or as components of separate sustained release formulations.

In certain embodiments of the invention the procedure is an injection procedure, e.g., an intravitreal injection. In certain embodiments the procedure is an injection procedure in which, prior to administration, the first therapeutic agent is contained in a syringe and the sustained release formulation comprising the second therapeutic agent is contained in a needle attached to the syringe. For example, the first therapeutic agent may be dissolved in a liquid medium located in the syringe and the sustained formulation of the second therapeutic agent may comprise an ocular implant located in the needle.

In-any embodiment of the invention the sustained release formulation may comprise an ocular implant. In any embodiment of the invention the sustained release formulation may comprise a polymer and one or more therapeutic agents.

In another aspect, the invention provides an article of manufacture comprising the first and second therapeutic agents of the first aspect, wherein the article of manufacture comprises a needle containing the second therapeutic agent, wherein the article of manufacture optionally further comprises a syringe, such as a syringe that contains the first therapeutic agent and/or wherein the article of manufacture contains a unit dosage form of the first therapeutic agent and/or a unit dosage form of the second therapeutic agent.

The article of manufacture may further comprise a syringe, wherein the article of manufacture contains at least one compartment and the syringe and needle are housed in a single compartment of the article of manufacture and/or wherein the syringe and needle are attached to one another or wherein the article of manufacture contains at least two compartments, and wherein the syringe and needle are housed in individual compartments.

In another aspect, the invention provides an article of manufacture comprising the first and second therapeutic agents of the first aspect, wherein each therapeutic agent is contained in an individual syringe and wherein the article of manufacture optionally further comprises a needle.

In any embodiment of the present invention, the eye disorder can be a macular degeneration related condition, diabetic retinopathy, retinopathy or prematurity, or any condition featuring CNV, RNV, or RAP.

The first therapeutic agent may be selected from the group consisting of: Macugen® (pegaptanib sodium) or another VEGF aptamer or nucleic acid ligand; Lucentis® (ranibizumab), Avastin® (bevacizumb) or another antibody or antibody fragment that specifically binds to VEGF; combretastatin or a derivative or prodrug thereof such as Combretastatin A4 Prodrug (CA4P); VEGF-Trap; EVIZON™ (squalamine lactate); AG-013958 (Pfizer, Inc.); JSM6427 (Jerini AG), β2-glycoprotein 1 (β2-GP1), and a short interfering RNA (siRNA) or short hairpin RNA (shRNA) that inhibits expression of one or more VEGF isoforms, or inhibits expression of a VEGF receptor. In certain embodiments of the invention the therapeutic agent is not a steroid.

Those of skill in the art will appreciate that certain compounds encompassed by the structures herein may exhibit tautomerism, conformational isomerism, geometric isomerism and/or stereoisomerism. It should be understood that the invention encompasses use of any tautomeric, conformational isomeric, enantiomeric and/or geometric isomeric forms of the compounds described herein. Any references herein employing nomenclature that corresponds to illustrated structural formulae that represent only one of several tautomeric forms (or resonance structures) are not intended to limit the scope of the compounds described herein. Those of skill in the art also will recognize that the compounds disclosed as of use in the invention may exist in many different protonation states, depending on, among other things, the pH of their environment. Where structural formulae provided herein depict the compounds in only one of several possible protonation states, it will be understood that these structures are illustrative only, and that the invention is not limited to any particular protonation state--any and - all protonated forms are intended to fall within the scope of the invention.

Compounds of use in this invention may, in certain embodiments, bear multiple positive or negative charges and may have appropriate counter ions associated therewith. The identity of the associated counter ions are may be governed by the synthesis and/or isolation methods by which the compounds are obtained. Counter ions include, but are not limited to, chloride and other halides, acetate, trifluoroacetate, citrate, sulfate, phosphate, etc., and mixtures thereof. It will be understood that the identity of any associated counter ion is not a critical feature and that the invention encompasses the compounds in association with any type of counter ion. Moreover, as the compounds can exists in a variety of different forms, the invention is intended to encompass not only forms that are in association with counter ions (e.g., dry salts), but also forms that are not in association with counter ions (e.g., aqueous or organic solutions).

Unless otherwise stated or otherwise clearly evident from the context, the invention makes use of standard methods of molecular biology, cell culture, animal maintenance, ophthalmologic examination, and administration of therapeutic agents to subjects, etc., and uses art-accepted meanings of terms. This application refers to various patents and publications.

In addition, the following publications are referred to:
Current Protocols in Molecular Biology, Current Protocols in Immunology, Current Protocols in Protein Science, and Current Protocols in Cell Biology, all John Wiley & Sons, N.Y., edition as of July 2002; Sambrook, Russell, and Sambrook, Molecular Cloning: A Laboratory Manual, 3rd ed., Cold Spring Harbor Laboratory Press, Cold Spring Harbor, 2001; Kuby Immunology, 4th ed, Goldsby, R.A., Kindt, T.J., and Osborne, B. (eds.)', W.H. Freeman, 2000, Goodman and Gilman's The Pharmacological Basis of Therapeutics, 10th Ed. .McGraw Hill, 2001, Katzung, B. (ed.) Basic and Clinical Pharmacology, McGraw-Hill/Appleton & Lange; 9th edition (December 2003), Ophthalmic Surgery: Principles and Practice, 3rd ed., W.B. Saunders Company, 2002; Albert, DM and Lucarelli, MJ (eds.), Clinical Atlas of Procedures in Ophthalmic Surgery, American Medical Association, 2003. In the event of a conflict or inconsistency between any of the incorporated references and the instant specification, the specification shall control, it being understood that the determination of whether a conflict or inconsistency exists is within the discretion of the inventors and can be made at any time.

### Brief Description of the Drawing

***Figures 1A-1E*** show schematic representations of the anterior and posterior segments of the eye (1A and 1B) and the outer layers of the eye (1C-1E). Figure 1C depicts a normal eye. Figure ID depicts an eye suffering from dry ARMD. Figure IE depicts an eye suffering from exudative ARMD. ONL = outer nuclear layer; IS = inner segment; OS = outer segment; RPE = retinal pigment epithelial layer; BM = Bruch's membrane; CC = choriocapillaris. From Tezel, T., et al., Trends in Molecular Medicine, 10(9), 417-420, 2004.
***Figure 2*** shows a consensus sequence for a short consensus repeat (SCR), a module found in complement control proteins. From Smith, SA, et al., J. Virol. 74(12), 5659-5666, 2000.
***Figures 3A and 3B*** show sequences of vaccinia virus complement control protein precursor (SEQ ID NO: 33) and the mature vaccinia virus complement control protein (SEQ ID NO: 34).
***Figure 4*** shows a sequence comparison of mature complement control proteins from a variety of orthopoxvirus isolates (SEQ ID NO: 35 - 42). The corresponding genetic loci are listed. Modified from Smith, SA, et al., J. Virol. 74(12), 5659-5666, 2000.
***Figure 5*** shows a comparison of the SCR domain structure of a number of complement control proteins and fragments thereof, the number of K+R residues, %K+R residues, pI, number of putative heparin binding sites, and ability to inhibit hemolysis and/or bind to heparin. Modified from Smith, SA, et al., J. Virol. 74(12), 5659-5666, 2000. The domains are SCR modules. Thus, for example, rVCP SCR (2, 3, 4), is a recombinantly produced polypeptide containing SCRs 2, 3, and 4 from VCP.
***Figure 6*** shows the amino acid sequence of SPICE (SEQ ID NO: 44).

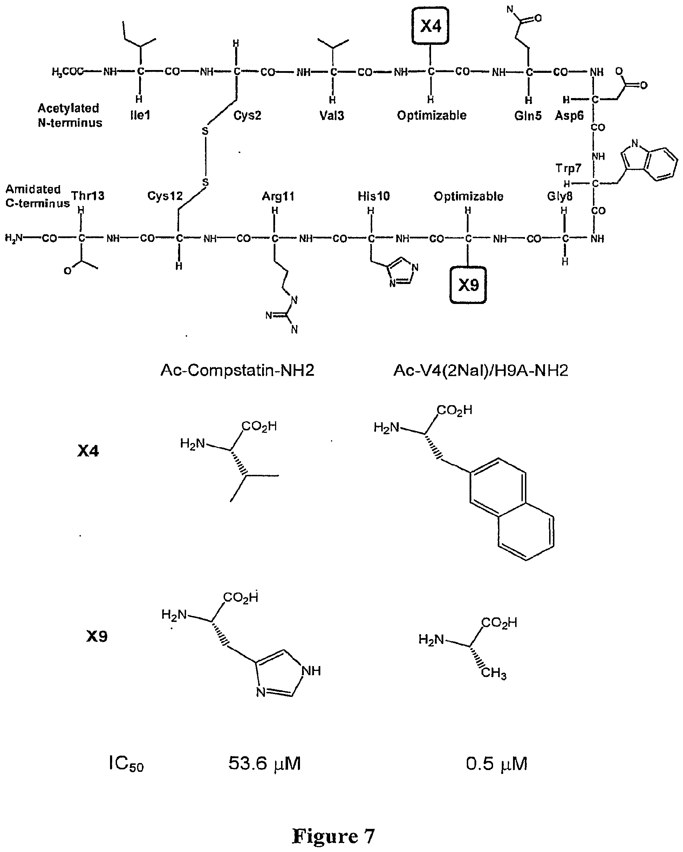

***Figure 7*** shows the structure of compstatin and the structure of a compstatin analog showing increased complement inhibiting activity relative to compstatin. The figure also shows the IC50 of compstatin and the compstatin analog for inhibition of human complement. Amino acids 4 and 9 in the peptide chain depicted in the upper portion of the figure are as shown on the lower left for compstatin and as shown on the lower right for the compstatin analog. Thus the boxes labeled "X4" and "X9" in the peptide chain represent the side chains of the amino acids X4 and X9 shown in the lower portion of the figure for compstatin (left) and the compstatin analog (right) respectively.
***Figure 8*** shows an exemplary compound for use in the invention.
***Figure 9*** shows a needle/syringe assembly loaded with first and second therapeutic agents.

### Definitions

"Activity period" refers to the time period over which a subject experiences an improvement in one or more symptoms and/or signs of a disorder following administration of a therapeutic agent, relative to a baseline condition or state existing prior to administration of the therapeutic agent. The activity period begins when the subject first experiences improvement and ends when the subject's condition or state returns to a baseline that existed prior to administration of the agent.

"Angiogenesis" or "angiogenic" refer to formation, growth, and/or development of new blood vessels.

The terms "angiogenesis inhibitor" and "antiangiogenic agent" are used interchangeably herein to refer to agents that are capable of inhibiting or reducing one or more processes associated with angiogenesis including, but not limited to, endothelial cell proliferation, endothelial cell survival, endothelial cell migration, differentiation of precursor cells into endothelial cells, and capillary tube formation.

"Antibody", as used herein, refers to an immunoglobulin or portion thereof that binds to an antigen. An antibody may be natural or wholly or partially synthetically produced. An antibody may be derived from natural sources, e.g., purified from an animal such as a rodent, rabbit, or chicken, that has been immunized with an antigen or a construct that encodes the antigen. An antibody may be a member of any immunoglobulin class, including any of the human classes: IgG, IgM, IgA, IgD, and IgE. An antibody of use in this invention may be an antibody fragment such as an Fab', F(ab')₂, scFv (single-chain variable) or other fragment that retains an antigen binding site, or a recombinantly produced scFv fragment, including recombinantly produced fragments that comprise an immunoglobulin antigen binding domain. See, e.g., Allen, T., Nature Reviews Cancer, Vol.2, 750-765, 2002, and references therein. Antibody fragments which contain the idiotype of the antibody molecule can be generated by known techniques. For example, F(ab')₂ fragments can be produced by pepsin digestion of the antibody molecule, Fab' fragments can be produced by reducing the disulfide bridges of the F(ab')₂ fragment, or by treating the antibody molecule with papain and a reducing agent. An antibody can be an antibody multimer or a multimer of antibody fragments. Antibodies, antibody fragments, and/or protein domains comprising an antigen binding site may be generated and/or selected *in vitro,* e.g., using techniques such as phage display (Winter, G. et al., Annu. Rev. Immunol. 12:433-455, 1994), ribosome display (Hanes, J., and Pluckthun, A. Proc. Natl. Acad. Sci. USA. 94:4937-4942, 1997), etc.

An antibody may be polyclonal (e.g., an affinity-purified polyclonal antibody) or monoclonal. A "monoclonal antibody" as used herein refers to a population of substantially homogeneous antibodies or a member of such a population, i.e., the individual antibodies comprising the population are identical except for possible naturally occurring mutations that can be present in minor amounts. In contrast to polyclonal antibody preparations that typically include different antibodies directed against different determinants (epitopes), each monoclonal antibody is directed against a single determinant on the antigen and is therefore highly specific. The modifier "monoclonal" indicates the character of the antibody as being obtained from a substantially homogeneous population of antibodies, and is not to be construed as requiring production of the antibody by any particular method. For example, monoclonal antibodies to be used in accordance with the present invention can be made by the hybridoma method first described by Kohler & Milstein, Nature 256: 495, 1975, or alternatively can be made by recombinant DNA methods (see e.g., U.S. Pat. No. 4,816,567).

An antibody may be a "chimeric" antibody in which for example, a variable domain of rodent origin is fused to a constant domain of human origin, thus retaining the specificity of the rodent antibody. The domain of human origin need not originate directly from a human in the sense that it is first synthesized in a human being. Instead, "human" domains may be generated in rodents whose genome incorporates human immunoglobulin genes. Such an antibody is considered at least partially "humanized". The degree to which an antibody is "humanized" can vary. Thus part or most of the variable domain of a rodent antibody may be replaced by human sequences, e.g., by site-directed mutagenesis of a polynucleotide that encodes the antibody or a portion thereof. According to one approach rodent, e.g., murine, complementarity-determining regions (CDRs) are grafted onto the variable light (VL) and variable heavy (VH) frameworks of human immunoglobulin molecules, while retaining only those rodent framework residues deemed essential for the integrity of the antigen-binding site. See Gonzales NR, Tumour Biol. Jan-Feb;26(1):31-43, 2005 for a review of various methods of minimizing antigenicity of a monoclonal antibody. Such human or humanized chimeric antibodies are often preferred for use in therapy of human diseases or disorders, since the human or humanized antibodies are less likely than to induce an immune response.

A variety of methods are known for determining whether or not an antibody reacts with, or specifically binds to, an antigen such and for determining the affinity of such binding if desired. Examples include enzyme-linked immunosorbent assays (ELISA), radioimmunoassays (RIA), and the like. Binding of an antibody to a target molecule such as a protein may inhibit or interfere with the activity of the target molecule. For example, binding of an antibody to ligand such as a growth factor may interfere with the binding of the ligand to its receptor(s); binding of an antibody to a receptor may interfere with the binding of the receptor to its ligand(s).

The terms "approximately" or "about" in reference to a number include numbers that fall within a range of 5% in either direction (greater than or less than) of the number unless otherwise stated or otherwise evident from the context (except where such number would exceed 100% of a possible value).

"Biocompatible" refers to a material that is substantially nontoxic to a recipient's cells in the quantities and at the location used, and does not elicit or cause a significant deleterious or untoward effect on the recipient's body at the location used, e.g., an unacceptable immunological or inflammatory reaction, unacceptable scar tissue formation, etc. A material that is biocompatible with the eye does not substantially interfere with the physiology or function of the eye.

"Biodegradable" means that a material is capable of being broken down physically and/or chemically within cells or within the body of a subject, e.g., by hydrolysis under physiological conditions and/or by natural biological processes such as the action of enzymes present within cells or within the body, and/or by processes such as dissolution, dispersion, etc., to form smaller chemical species which can typically be metabolized and, optionally, used by the body, and/or excreted or otherwise disposed of. Preferably a biodegradable compound is biocompatible. A polymer whose molecular weight decreases over time *in vivo* due to a reduction in the number of monomers is considered biodegradable.

A "biological macromolecule" is a large molecule composed of smaller subunits of a type that are found in biological systems. Examples include polypeptides, nucleic acids, and polysaccharides. Typically a biological macromolecule contains at least 3 subunits (e.g., amino acids, nucleosides, monosaccharides, etc.). The biological macromolecule may be a naturally occurring polypeptide, nucleic acid, or polysaccharide. The biological macromolecule may be modified, e.g., it may be conjugated to a nonbiological molecule such as synthetic polymer, etc.

The phrases "characterized by macular degeneration, choroidal neovascularization, retinal neovascularization, or any combination of the foregoing" and "characterized by macular degeneration, choroidal neovascularization, or retinal neovascularization" are intended to indicate that macular degeneration, CNV, and/or RNV, is a characteristic (i.e., typical) feature of the disorder. Macular degeneration, CNV, and/or RNV may be a defining and/or diagnostic feature of the disorder.

"Choroidal neovascularization" (CNV) refers to the abnormal development, proliferation, and/or growth of blood vessels arising from the choriocapillaris. The blood vessels typically extend through Bruch's membrane, RPE layer, and/or subretinal space.

A "complement component" or "complement protein" is a molecule that is involved in activation of the complement system or participates in one or more complement-mediated activities. Components of the classical complement pathway include, e.g., C1q, C1r, C1s, C2, C3, C4, C5, C6, C7, C8, C9, and the C5b-9 complex, also referred to as the membrane attack complex (MAC) and active fragments or enzymatic cleavage products of any of the foregoing (e.g., C3a, C3b, C4a, C4b, C5a, etc.). Components of the alternative pathway include, e.g., factors B, D, H, and I, and properdin.

The terms "deliver" or "delivery", in the context of a drug delivery device or sustained release formulation refers to release of a therapeutic agent into its surrounding environment in the body.

A "drug delivery device" refers to a device, structure, or element that contains and/or delivers a therapeutic agent to a subject. Release of the drug may, but need not, occur as a result of degradation of the drug delivery device within the body. The term "drug delivery device" is used herein to refer to devices that contain a therapeutic agent and to devices that have not yet been loaded with the therapeutic agent. An ocular implant is a drug delivery device that has appropriate dimensions and structure for placement within the eye. Preferably an ocular drug delivery device does not substantially interfere with the physiology and/or functioning of the eye, e.g., the device causes minimal or no disruption of vision.

An "effective amount" of an active agent refers to the amount of the active agent sufficient to elicit a desired biological response. As will be appreciated by those of ordinary skill in this art, the absolute amount of a particular agent that is effective may vary depending on such factors as the desired biological endpoint, the agent to be delivered, the target tissue, *etc.* Those of ordinary skill in the art will further understand that an "effective amount" may be administered in a single dose, or may be achieved by administration of multiple doses. For example, an effective amount of a therapeutic agent for the treatment of an eye disorder may be an amount sufficient to treat the disorder, e.g., an amount sufficient to achieve one or more of the following: (i) inhibit or prevent drusen formation; (ii) cause a reduction in drusen number and/or size (drusen regression); (iii) cause a reduction in or prevent lipofuscin deposits; (iv) inhibit or prevent visual loss or slow the rate of visual loss; (v) inhibit choroidal neovascularization or slow the rate of choroidal neovascularization; (vi) cause a reduction in size and/or number of lesions characterized by choroidal neovascularization; (vii) inhibit choroidal neovascularization or slow the rate of retinal neovascularization; (viii) cause a reduction in size and/or number of lesions characterized by-retinal neovascularization; (ix) improve visual acuity and/or contrast sensitivity; (x) reduce macular edema and/or reduce abnormal macular thickness; (xi) inhibit or prevent photoreceptor or RPE cell atrophy or apoptosis, or reduce the rate of photoreceptor or RPE cell atrophy or apoptosis; (xii) inhibit or prevent progression of non-exudative macular degeneration to exudative macular degeneration.

"Eye disorder", which is used interchangeably herein with "ocular disorder" refers to any disease, disorder, or condition that involves or affects the eye or one or more portions, structures, or parts of the eye. The eye includes the eyeball, the periocular muscles, and the portion of the optic nerve which is within or adjacent to the eyeball.

"Exudative" macular degeneration is used herein synonymously with "wet" type macular degeneration, as those terms are generally understood in the art, i.e., to refer to a macular degeneration related condition such as ARMD characterized by neovascularization and/or the presence of an exudate.

"Identity" refers to the extent to which the sequence of two or more nucleic acids or polypeptides is the same. The percent identity between a sequence of interest and a second sequence over a window of evaluation, e.g., over the length of the sequence of interest, may be computed by aligning the sequences, determining the number of residues (nucleotides or amino acids) within the window of evaluation that are opposite an identical residue allowing the introduction of gaps to maximize identity, dividing by the total number of residues of the sequence of interest or the second sequence (whichever is greater) that fall within the window, and multiplying by 100. By gap is meant a portion of a sequence that is not occupied by a residue. For example, the sequence A K L - - - S I G (SEQ ID NO: 43) contains a gap of three residues. When computing the number of identical residues needed to achieve a particular percent identity, fractions are to be rounded to the nearest whole number. Percent identity can be calculated with the use of a variety of computer programs known in the art. For example, computer programs such as BLAST2, BLASTN, BLASTP, Gapped BLAST, etc., generate alignments and provide percent identity between a sequence of interest and sequences in any of a variety of public databases. The algorithm of Karlin and Altschul (Karlin and Altschul, Proc. Natl. Acad. Sci. USA 87:22264-2268, 1990) modified as in Karlin and Altschul, Proc. Natl. Acad. Sci. USA 90:5873-5877, 1993 is incorporated into the NBLAST and XBLAST programs of Altschul et al. (Altschul, et al., J. Mol. Biol. 215:403-410, 1990). To obtain gapped alignments for comparison purposes, Gapped BLAST is utilized as described in Altschul et al. (Altschul, et al. Nucleic Acids Res. 25: 3389-3402, 1997). When utilizing BLAST and Gapped BLAST programs, the default parameters of the respective programs are used. A PAM250 or BLOSUM62 matrix may be used. See the Web site having URL www.ncbi.nlm.nih.gov for these programs. In a specific embodiment, percent identity of a sequence of interest and a second sequence is calculated using BLAST2 with default parameters.

"Invasive therapy" as used herein, is therapy that involves insertion of an instrument or device into the eye or orbit, e.g, entrance into or penetration of the eyeball or entry into the orbit by an instrument such as a needle, trocar, catheter, or the like.

"Liposomes" are artificial microscopic spherical particles formed by a lipid bilayer (or multilayers) enclosing an aqueous compartment. Liposomes can be used for delivering certain of the compositions of the invention.

The term "long-acting therapeutic agent" refers to a therapeutic agent that has an activity period of at least 3 months when administered in medically acceptable quantities. A "medically acceptable quantity" refers to an amount that does not cause unacceptable toxicity or adverse effects under the conditions of administration.

"Macular degeneration related condition" refers to any of a number of disorders and conditions in which the macula degenerates or loses functional activity. The degeneration or loss of functional activity can arise as a result of, for example, cell death, decreased cell proliferation, and/or loss of normal biological function. Macular degeneration can lead to and/or manifest as alterations in the structural integrity of the cells and/or extracellular matrix of the macula, alteration in normal cellular and/or extracellular matrix architecture, and/or the loss of function of macular cells. The cells can be any cell type normally present in or near the macula including RPE cells, photoreceptors, and/or capillary endothelial cells. ARMD is the major macular degeneration related condition. Others include Best macular dystrophy, Sorsby fundus dystrophy, Mallatia Leventinese and Doyne honeycomb retinal dystrophy.

"Non-exudative" macular degeneration is used herein synonymously with "dry" type macular degeneration as those terms are generally used in the art, to refer to a macular degeneration related condition, e.g., ARMD, in which neovascularization and/or exudation that would be detectable using standard methods such as fluorescein angiography has not occurred.

"Ocular implant" refers to a device or structure that has appropriate dimensions, shape, and/or configuration and is made of appropriate materials so that it may be placed in the eye without causing unacceptable interference with the physiology or functioning of the eye. Preferably placement of an ocular implant does not significantly disrupt vision. An ocular implant is typically a solid or semi-solid article of manufacture and is typically macroscopic, i.e., visible with the naked eye.

"Plurality" means more than one.

"Polypeptide", as used herein, refers to a polymer of amino acids, optionally including one or more amino acid analogs. A protein is a molecule composed of one or more polypeptides. A peptide is a relatively short polypeptide, typically between about 2 and 60 amino acids in length. The terms "protein", "polypeptide", and "peptide" may be used interchangeably. Polypeptides used herein may contain amino acids such as those that are naturally found in proteins, amino acids that are not naturally found in proteins, and/or amino acid analogs that are not amino acids. A large number of art-recognized analogs of the 20 amino acids commonly found in proteins (the "standard" amino acids) are known. As used herein, an "analog" of an amino acid may be a different amino acid that structurally resembles the amino acid (a "non-standard" amino acid) or a compound other than an amino acid that structurally resembles the amino acid. One or more of the amino acids in a polypeptide may be modified, for example, by the addition of a chemical entity such as a carbohydrate group, a phosphate group, a farnesyl group, an isofarnesyl group, a fatty acid group, a linker for conjugation, functionalization, or other modification, etc. Certain non-limiting suitable analogs and modifications are described in WO2004026328. The polypeptide may be acetylated, e.g., at the N-terminus and/or amidated, e.g., at the C-terminus.

The natural or other chemical modifications such as those described above can occur anywhere in a polypeptide, including the peptide backbone, the amino acid side-chains and the amino or carboxyl termini. A given polypeptide may contain many types of modifications. Polypeptides may be branched or they may be cyclic, with or without branching. Polypeptides may be conjugated with, encapsulated by, or embedded within a polymer or polymeric matrix, dendrimer, nanoparticle, microparticle, liposome, or the like. Polypeptides of use in this invention may, for example, be purified from natural sources, produced *in vitro* or *in vivo* in suitable expression systems using recombinant DNA technology (e.g., by recombinant host cells or in transgenic animals or plants), synthesized through chemical means such as conventional solid phase peptide synthesis and/or methods involving chemical ligation of synthesized peptides (see, e.g., Kent, S., J Pept Sci., 9(9):574-93, 2003 and U.S. Pub. No. 20040115774), or any combination of these. The term "polypeptide sequence" or "amino acid sequence" as used herein can refer to the polypeptide material itself and is not restricted to the sequence information (i.e. the succession of letters or three letter codes chosen among the letters and codes used as abbreviations for amino acid names) that biochemically characterizes a polypeptide. A polypeptide sequence presented herein is presented in an N-terminal to C-terminal direction unless otherwise indicated.

"Poxvirus" refers to a family of complex, double-stranded DNA viruses constituting the family *Poxviridae.* The family includes the orthopoxviruses, a genus of the family *Poxviridae,* subfamily *Chordopoxvirinae,* comprising many species infecting mammals. Poxviruses are described in Fields, BN, et al., Fields Virology, 3rd ed., Lippincott Williams & Wilkins, 2001. Orthopoxviruses include vaccinia virus, variola virus major, variola virus minor, cowpox virus, monkeypox virus, camelpox virus, swinepox virus, and ectromelia virus.

"Poxvirus complement control protein" refers to members of a family of homologous proteins encoded by a number of different poxviruses that bind to one or more complement pathway proteins and inhibit the classical pathway of complement activation, the alternative pathway of complement activation, the lectin pathway, or any combination of these. Poxvirus complement control proteins are members of the complement control protein (CCP), also called regulators of complement activation (RCA) superfamily (Reid, KBM and Day, AJ, Immunol Today, 10:177-80, 1989).

"Posterior segment of the eye" refers to the portion of the eye behind the lens, including the vitreous, choroid, and retina (including the macula).

"Rapid improvement in the condition of a subject's eye" refers to a clinically significant improvement in one or more symptoms and/or signs of an ocular disorder that occurs within two weeks, or preferably within one week, following administration of a therapeutic agent. Rapid improvement in the condition of a subject's eye can include, without limitation, any one or more of the following: increased visual acuity (e.g., gaining two or more lines of vision on a visual acuity chart), decreased visual distortion, increased contrast sensitivity, decreased retinal vessel leakage, decreased macular thickness (e.g., a decrease in macular thickness of at least 50% from a baseline value). In general, "rapid" as used herein in reference to a therapeutic or other biological effect, means occurring within two weeks or less following a reference event (e.g., administration of a therapeutic agent). In some embodiments, "rapid" means occurring within one week or less following a reference event.

"Retinal neovascularization" refers to the abnormal development, proliferation, and/or growth of blood vessels on or in the retina, e.g., on the retinal surface (i.e., as will be evident to one of ordinary skill in the art, the abnormal proliferation, and/or growth originates from blood vessels already present on or in the surface). "Retinal" here refers to the source of the neovascularization.

"Single procedure" means a procedure, i.e., a process or series of steps or acts that involves a single entrance into or penetration of the eyeball or entry into the orbit by an instrument such as a needle, trocar, catheter, or the like. For example, an eye injection, e.g., an intravitreal injection, is a single procedure provided that the tip of the needle, once having been inserted into the eyeball, is not reintroduced into the eyeball once having been withdrawn therefrom. A single procedure may or may not involve multiple penetrations of one or more structures of the eye, e.g., the vitreous, provided that only a single entrance into or penetration of the eyeball takes place.

"Small molecule" refers to organic compounds, whether naturally-occurring or artificially created (e.g., via chemical synthesis) that have relatively low molecular weight and that are not proteins, polypeptides, or nucleic acids. Typically, small molecules have a molecular weight of less than about 1500 g/mol and multiple carbon-carbon bonds.

"Specific binding" generally refers to a physical association between a target polypeptide (or, more generally, a target molecule) and a binding molecule such as an antibody or ligand. The association is typically dependent upon the presence of a particular structural feature of the target such as an antigenic determinant or epitope recognized by the binding molecule. For example, if an antibody is specific for epitope A, the presence of a polypeptide containing epitope A or the presence of free unlabeled A in a reaction containing both free labeled A and the binding molecule that binds thereto, will reduce the amount of labeled A that binds to the binding molecule. It is to be understood that specificity need not be absolute but generally relates to the context in which the binding occurs. For example, it is well known in the art that numerous antibodies cross-react with other epitopes in addition to those present in the target molecule. Such cross-reactivity may be acceptable depending upon the application for which the antibody is to be used. One of ordinary skill in the art will be able to select antibodies or ligands having a sufficient degree of specificity to perform appropriately in any given application (e.g., for detection of a target molecule, for therapeutic purposes, etc). It is also to be understood that specificity may be evaluated in the context of additional factors such as the affinity of the binding molecule for the target versus the affinity of the binding molecule for other targets, e.g., competitors. If a binding molecule exhibits a high affinity for a target molecule that it is desired to detect and low affinity for nontarget molecules, the antibody will likely be an acceptable reagent. Once the specificity of a binding molecule is established in one or more contexts, it may be employed in other, preferably similar, contexts without necessarily re-evaluating its specificity. Binding of two or more molecules may be considered specific if the affinity (equilibrium dissociation constant, Kd) is 10⁻³ M or less, preferably 10⁻⁴ M or less, more preferably 10⁻⁵ M or less, e.g., 10⁻⁶ M or less, 10⁻⁷ M or less, 10⁻⁸ M or less, or 10⁻⁹ M or less under the conditions tested, e.g., under physiological conditions.

"Stabilize", as used herein in reference to a eye disorder, means to reduce the rate of progression of the disorder and/or to prevent or reduce the likelihood of a rapid and noticeable deterioration in the condition of an eye afflicted with the disorder.

"Subject", as used herein, refers to an individual to whom an agent is to be delivered, e.g., for experimental, diagnostic, and/or therapeutic purposes. Preferred subjects are mammals, e.g., , primates, or humans. A subject under the care of a physician or other health care provider may be referred to as a "patient".

"Substantial sequence homology" as applied to a sequence means that the sequence displays at least approximately 60% identity, desirably at least approximately 70% identity, more desirably at least approximately 80% identity, and most desirably at least approximately 90% identity relative to a reference sequence. When two or more sequences are compared, any of them may be considered the reference sequence. % identity can be calculated using a FASTA, BLASTN, or BLASTP algorithm. Default parameters may be used. A PAM250 or BLOSUM62 matrix may be used.

A "sustained release formulation" or "sustained delivery formulation" is a composition of matter that comprises a therapeutic agent as one of its components and further comprises or has one or more components, elements, or structures effective to provide sustained release of the therapeutic agent, optionally in part as a consequence of the physical structure of the formulation. In some embodiments the structure is provided at least in part by the therapeutic agent itself and, optionally, one or more substances present at the site of administration. Sustained release is release or delivery that occurs either continuously or intermittently over a period of time e.g., at least 1, 2, 4, or 6 weeks, at least 1, 2, 3, 4, 6, 8, 10, 12, 15, 18, or 24 months, or longer.

"Therapeutic agent" is used interchangeably herein with "drug", to refer to any pharmaceutically active agent useful for treating a disorder. The term includes any pharmaceutically acceptable salt, prodrug, salt of a prodrug, and such derivatives of an active agent as are known in the art or readily produced using standard methods known in the art. "Prodrug" refers to a precursor of a drug, wherein the prodrug is not itself pharmacologically active (or has a lesser or different activity than the desired activity of the drug) but is converted, following administration (e.g., by metabolism) into the pharmaceutically active drug. A therapeutic agent can be, without limitation, a small molecule or a biological macromolecule such as a protein (e.g., an antibody) or nucleic acid such as an aptamer, siRNA, etc.

"Treating", as used herein, refers to providing treatment, i.e, providing any type of medical or surgical management of a subject in order to reverse, alleviate, inhibit the progression of, prevent or reduce the likelihood of a disease, disorder, or condition, or in order to reverse, alleviate, inhibit or prevent the progression of, prevent or reduce the likelihood of one or-more symptoms or manifestations of a disease, disorder or condition. "Prevent" refers to causing a disease, disorder, condition, or symptom or manifestation of such not to occur. Treating can include administering an agent to the subject following the development of one or more symptoms or manifestations indicative of a condition such as macular degeneration or diabetic retinopathy, e.g., in order to reverse, alleviate, reduce the severity of, and/or inhibit or prevent the progression of the condition and/or to reverse, alleviate, reduce the severity of, and/or inhibit or one or more symptoms or manifestations of the condition. A composition of this invention can be administered to a subject who has developed an eye disorder such as exudative or non-exudative ARMD or diabetic retinopathy or is at increased risk of developing such a disorder relative to a member of the general population. A composition of this invention can be administered prophylactically, i.e., before development of any symptom or manifestation of then condition. Typically in this case the subject will be at risk of developing the condition.

"Unit dosage form" as used herein refers to physically discrete units suited as unitary dosages for the subject to be treated (e.g., for a single eye); each unit containing a predetermined quantity of an active agent selected to produce the desired therapeutic effects, optionally together with a pharmaceutically acceptable carrier, which may be provided in a predetermined amount The unit dosage form may be, for example, a volume of liquid (e.g,. a pharmaceutically acceptable carrier) containing a predetermined quantity of a therapeutic agent, a predetermined amount of a therapeutic agent in solid form, an ocular implant containing a predetermined amount of a therapeutic agent, a plurality of nanoparticles or microparticles that collectively contain a predetermined amount of a therapeutic agent, etc. It will be appreciated that a unit dosage form may contain a variety of components in addition to the therapeutic agent. For example, pharmaceutically acceptable carriers, diluents, stabilizers, buffers, preservatives, etc., may be included.

### Detailed Description of Certain Embodiments of the Invention

### I. Overview

The present invention provides therapeutic agents for use in the treatment of an eye disorder characterized by macular degeneration, CNV, or RNV. The invention also provides articles of manufacture comprising the therapeutic agents. Exemplary disorders that can be treated according to the invention include, but are not limited to, macular degeneration related conditions, diabetic retinopathy, and retinopathy of prematurity.. While the concepts underlying the invention are described herein with particular reference totreatment of wet ARMD or other conditions characterized by CNV and/or RNV, they apply to a range of different ocular (and other) disorders.

The invention encompasses the recognition that eye care providers, e.g., ophthalmologists, are often relucant to administer a therapeutic agent, e.g., an angiogenesis inhibitor, using an invasive procedure such as intravitreal injection that is associated with the risk of a severe complication unless there is a significant likelihood that the therapy will cause rapid improvement in the condition of the patient's eye. There can be reluctance to use an invasive procedure to administer therapy that may possibly prevent or delay future deterioration or destabilization in an eye that is, at least from a symptomatic standpoint, relatively stable. Similarly, patients are often reluctant to undergo administration of a therapeutic agent using an invasive procedure associated with the risk of a severe complication unless they have recently experienced noticeable deterioration or destabilization in the condition of their eye(s) and there is a significant likelihood that administration of the therapeutic agent will result in rapid and/or noticeable improvement in the condition. Patients with an eye in a relatively stable condition are often reluctant to undergo an invasive procedure to administer a therapeutic agent that may halt or slow progress of the disorder if the procedure is associated with the risk of a severe complication.

As a consequence, when administration of a therapeutic agent involves an invasive procedure associated with a risk of a severe complication, patients may be treated on a symptom driven, case-by-case basis, rather than according to a predetermined, recommended dosing schedule that would at least in part involve administering the agent while the patient's condition is apparently stable, at least from a symptomatic standpoint. This appears to be the case even though following the predetermined, recommended dosing schedule may have the potential to delay or inhibit future destabilization or deterioration. It was observed that in one well known eye clinic, an angiogenesis inhibitor was administered to patients with exudative ARMD on a symptom driven basis, in response to a sudden deterioration or destabilization in the condition of a patient's eye or in response to the presence of exudation (e.g., hemorrhage), rather than according to a predetermined dosing schedule.

Clinical trials have demonstrated that certain angiogenesis inhibitors, e.g., Macugen and Lucentis, are of benefit in terms of important parameters such as visual acuity when administered as recommended, i.e., by repeated intravitreal administration of a solution containing the agent. For example, administration of certain angiogenesis inhibitors slows the rate of visual loss and may lead to at least temporary improvement in visual acuity. Based on - clinical trials, the recommended dosing interval for. Lucentis is 4 weeks (see, e.g., Heier, J.S., et al., Invest Opthalmol Vis Sci, 44:e-abstract 972, 2003), while the recommended dosing interval for Macugen is 6 weeks (see, e.g., Gragoudas ES, N Engl J Med., 351(27):2805-16, 2004). Avastin, while currently not approved for treatment of eye disorders by the U.S. Food and Drug Administration has been approved for the treatment of certain cancers and is available for use in the eye. Since Lucentis and Avastin act in a similar manner by binding to VEGF isoforms, these agents may have similar therapeutic effects.

Repeated administration of angiogenesis inhibitors according to the dosing intervals described above could result in sustained improvement in the condition of the subject's eye by inhibiting further neovascularization and blood vessel leakage. However, given the risk associated with intravitreal injection, ophthalmologists appear reluctant to administer a therapy associated with significant risk while the patient's symptoms remain substantially stable. Similarly, patients appear reluctant to submit to a procedure with significant risk when their symptoms remain substantially stable.

Therefore, as a result of the desire to avoid intravitreal injections, angiogenesis inhibitors may not be administered according to the recommended or predetermined dosing intervals. Instead, in practice these agents may be administered on a symptomatic basis, e.g., after a subject has experienced a deterioration or destabilization in the condition of the eye such as an acute loss of visual acuity and/or presence of exudation relative to a baseline condition, e.g., relative to the improved condition that resulted following administration of the previous dose of the angiogenesis inhibitor. Such deterioration or destabilization can occur at a variable and unpredictable time following the initial improvement. Instead of retreating the patient with an angiogenesis inhibitor when the patient is symptomatically stable in an effort to prevent future deterioration or destabilization, at the possible risk of causing a severe complication, treatment may be postponed until the subject has actually experienced deterioration or destabilization such that treatment would be likely to result in a symptomatic improvement in addition to any possible preventive effect. Thus the desire to avoid intravitreal injection of an eye that is symptomatically stable has a significant and heretofore unappreciated effect on clinical practice. In essence, the risk/benefit ratio as perceived by opthalmologists and patients may dictate that intravitreal administration of angiogenesis inhibitors should be performed on an individualized basis, following deterioration or destabilization of a patient's eye, rather than according to a predetermined dosing interval of approximately 4 or 6 weeks. It is unclear whether treatment on a symptomatic basis will have a greater, lesser, or equivalent efficacy on a long-term basis (e.g., over periods of a year or more) relative to treatment according to the recommended, predetermined dosing schedule. However, in view of the definite and known risk of complications associated with intravitreal injection, ophthalmologists and patients may be willing to forego the possible long-term increased benefit that could result from administering anti-angiogenic therapy at predetermined intervals of 4-6 weeks instead of on a symptomatic basis.

In summary, the inventors' observations suggest that ophthalmologists and patients are reluctant to accept the risks associated with intravitreal injection unless there is a significant likelihood that the therapy thus administered will result in a rapid improvement in the condition of the patient's eye. However, this mode of administration may not be optimal in terms of providing long term improvement in and/or stabilization of the condition of the subject's eye.

The present invention encompasses the recognition that symptom driven treatment may be less than optimal for preventing or delaying further deterioration in the patient's condition. Furthermore, a formulation of a therapeutic agent that is preferred or optimal for producing rapid improvement in the condition of a patient's eye may not be preferred or optimal for achieving long-term improvement and/or stabilization. For example, in the case of eye disorders, rapid improvement may best be achieved by locally administering a therapeutic agent in solution or in a form in which it is quickly released, so as to rapidly achieve a high concentration of the agent at the location where activity is desired. However, long-term benefit may best be achieved by locally administering a sustained release formulation of the therapeutic agent that provides a lower, yet still therapeutically effective concentration of the therapeutic agent. Alternately or additionaly, therapeutic agent(s) that are most appropriate for relieving particular symptoms may be less appropriate for providing long-term benefit.

"Administering" or "administration" generally refers to introducing a therapeutic agent, composition, formulation, etc., to a desired site or location on or within the body of a subject, e.g., a site or location within the eye. Adminstration may be performed, e.g., by a health care provider. For purposes of convenience, the present specification refers generally toophthalmologists. However, the methods described herein, including both the methods of the invention and other methods (e.g., methods for diagnosing and/or monitoring an eye disorder) may be practeiced by any qualified health care provider.

The present invention provides therapeutic agents and articles of manufacture that address the preferences of ophthalmologists and patients for avoiding procedures associated with a risk of severe complications while the condition of the subject's eye is relatively stable and at the same time offer the potential benefits associated with therapeutic agents and/or formulations that may be preferred for long term improvement, stabilization, and/or reduced progression of the condition. In some embodiments the invention provides agents for use in treating an eye disorder characterized by macular degeneration, CNV, or RNV comprising the step of administering the first and second therapeutic agents to the subject's eye in a single procedure, wherein the first therapeutic agent provides rapid improvement in the condition of the subject's eye and the second therapeutic agent is a long-acting therapeutic agent or is administered as a component of a sustained release formulation. One of skill in the art will appreciate that not all patients will exhibit an improvement in the condition of the eye. It will also be appreciated that the time to response (where "response" refers to improvement in the condition of the eye) may be an average time to response among patients who exhibit a response. In some embodiments of the invention, at least 10%, 20%, 30%, 40%, 50%, 60%, 70%, 80%, or 90% of patients exhibit a rapid improvement. In some embodiments the response rate falls between 10% and 100%, or any intervening range such as between 30% and 80%, etc., exhibit a rapid improvement.

According to certain embodiments of the invention a procedure is used to administer a first therapeutic agent that provides rapid improvement in the condition of the subject's eye. The agent may be administered, e.g., following a sudden deterioration in the condition of the subject's eye such as may be caused by retinal hemorrhage or vessel leakage. In the course of the same procedure, a second therapeutic agent (which may be the same as or different from the first therapeutic agent) is also administered at little or no additional risk to the patient. The second therapeutic agent is a long-acting agent or is a component of a sustained release formulation (or both). The second therapeutic agent may provide a long-term benefit to the patient, preferably prolonging the time interval before the patient experiences destabilization or perceives significant deterioration in the condition of the eye.

The first and second therapeutic agents may be administered sequentially or they may be administered substantially at the same time. If administered sequentially, they may be administered in either order. The order may be selected based on the identity and formulation of the agents. In certain embodiments of the invention the first and second therapeutic agents are administered no more than 5, 10, 15, 30, or 45 seconds apart, or no more than 1, 2, 3, 4, 5, 10, 15, or 30 minutes apart. In other words, the time interval between completing administration of the first agent and completing administration of the second agent is no more than 5, 10, 15, 30, or 45 seconds or no more than 1, 2, 3, 4, 5, 0, 15, or 30 minutes apart in various embodiments of the invention. In certain embodiments of the invention administration of the first and second therapeutic agents is completed within 5, 10, 15, 30, or 45 seconds or within 1, 2, 3, 4, 5, 10, 15, or 30 minutes from the time at which any amount of either the first or second therapeutic agent leaves the confines of any medical or surgical instrument or device (e.g., needle, syringe, trocar, catheter, cannula or other device that may enclose or contain a solution or solid formulation such as an implant and may be used to introduce such solution or solid formulation into the eye) and comes into contact with tissues or fluids of the subject's eye. Administration is said to be "complete" when the entire dose of an agent to be administered has left the confines of any medical or surgical instrument or device that is used to introduce the therapeutic agent into the subject's eye, it being understood that such instrument or device may retain a residual amount of the agent. The time interval starting from the time at which any amount of either the first or second therapeutic agent leaves the confines of any medical or surgical instrument or device and comes into contact with tissues or fluids of the subject's eye and the time at which administration of both first and second therapeutic agents is complete is referred to herein as the "time window" of administration. If more than two therapeutic agents are administered, the time window of administration is the time interval starting from the time at which any amount of any therapeutic agent leaves the confines of any medical or surgical instrument or device and comes into contact with tissues or fluids of the subject's eye and the time at which administration of all therapeutic agents is complete. The short time window of administration of the first and second therapeutic agents is an additional feature of embodiments of the invention. Thus disclosed herein is a method of treating an eye disorder characterised by macular degeneration, CNV, or RNV comprising the step of administering first and second therapeutic agents to the subject's eye within a short time window, wherein the first therapeutic agent provides rapid improvement in the condition of the subject's eye and the second therapeutic agent is a long-acting therapeutic agent or is administered as a component of a sustained release formulation. In the method the time window may be no more than 5, 10, 15, 30 or 45 seconds or no more than 1, 2, 3, 4, 5, 10, 15, or 30 minutes.

Also disclosed herein is a method of treating an eye disorder characterized by macular degeneration, CNV, or RNV comprising the step of administering first and second therapeutic agents to the subject's eye, wherein the first therapeutic agent is an angiogenesis inhibitor and the second therapeutic agent is a long-acting therapeutic agent or is administered as a component of a sustained release formulation. The second therapeutic agent may, but need not be, a complement inhibitor. In certain embodiments the second agent is a compstatin analog. Optionally the first and second therapeutic agents are administered in a single procedure.

Also disclosed is a method of treating an eye disorder characterized by macular degeneration, CNV, or RNV comprising the step of administering first and second therapeutic agents to the subject's eye, wherein the second therapeutic agent is a complement inhibitor that is either a long-acting complement inhibitor or is administered as a component of a sustained release formulation. The first therapeutic agent may, but need not be, an angiogenesis inhibitor. Optionally the first and second therapeutic agents are administered in a single procedure.

The therapeutic agent that provides rapid improvement in the condition of the subject's eye may be administered in a liquid medium. In some embodiments the agent is administered at least in part in a formulation that releases the agent over time in sufficient amounts and sufficiently quickly to provide a rapid improvement in the condition of the subject's eye. For example, particles that degrade or otherwise release an effective amount of the agent within the first 24, 48, 72, or 96 hours, within the first week, or within the first 2 weeks following administration, could be used. In some embodiments a first portion of the agent is provided in solution and a second portion is provided in a formulation that provides for release over time. Optionally the second portion is a component of the sustained release preparation of the second agent.

By administering the first and second therapeutic agents in a single procedure, certain embodiments of the present invention minimize the overall risk of complications and make administration of the second therapeutic agent, which may primarily delay progression, inhibit further deterioration or destabilization, and/or provide slow rather than rapid improvement in the condition of the subject's eye more acceptable to ophthalmologists and/or patients. The combination therapy approach of the present invention thus provides an unexpected and unappreciated improvement in the risk/benefit ratio associated with invasive therapy of eye disorders.

Also disclosed herein are pharmaceutical packs or kits and other articles of manufacture that facilitate the convenient, effective, and safe administration of multiple therapeutic agents to the eye using a single procedure.

Disclosed herein is a method of treating exudative macular degeneration with a combination of a fast-acting anti-angiogenic drug and a sustained release or long-acting complement inhibiting drug. The fast-acting anti-angiogenic drug reduces the leakage and/or promotes regression of newly-formed blood vessels in the weeks following treatment; the slow-release or long-acting complement inhibiting drug will prevent the formation of new blood vessels and promote disease regression for significant amounts of time (months or years depending on the device or formulation). Thus certain methods disclosed herein (1) inhibit/stop blood vessel formation and/or leakage in patients with exudative macular degeneration in an acute fashion, so that the retina comes closer in proximity to the choroid layer of the eye, and that thus ischemic damage to the retina is reduced, and (2) install a slow-release or long-acting complement inhibitor that will lower the inflammatory response in the retina/RPE/choroid layers of the eye and thus block a primary stimulus for blood vessel growth and, optionally, inhibit the formation of drusen deposits in addition.

In certain embodiments of any aspect of the invention, administration of the first therapeutic agent does not result in rapid improvement in the condition of the subject's eye. However, improvement takes place more gradually, e.g., within 15 days to 3 weeks, within 15 days to 4 weeks, within 15 days to 5 weeks, or within 15 days to 6 weeks.

### II. Therapeutic Agents

A variety of different therapeutic agents are of use in the present invention. Furthermore, the invention is not limited to the administration of two therapeutic agents. For example, the invention may comprise. administering a composition comprising one or more therapeutic agents in solution in a liquid medium, e.g., an aqueous medium, and also administering a composition comprising or consisting essentially of a sustained release formulation, e.g., an ocular implant, comprising one or more therapeutic agents, in the course of the same procedure. Thus certain embodiments of the invention involve administering at least two, three, or four therapeutic agents in a liquid composition and at least two, three, or four therapeutic agents in a sustained release preparation. Any of the therapeutic agents described herein may be included in either or both of the liquid composition and the sustained release formulation. The sustained release formulation may be a liquid composition as long as it possesses sustained release properties.

In certain embodiments the therapeutic agent has a targeting moiety either covalently or noncovalentily attached thereto. The targeting moiety comprises a ligand that binds to a marker present on or at the surface of a target cell or other component such as a drusen constituent present at a site of desired activity. The term "ligand" is used to refer to a moiety that specifically binds to a second moiety.

The total amount of each therapeutic agent used, and their concentrations, can vary. Exemplary, nonlimiting, doses are between .0001 mg/dose and 100 mg/dose for each eye to be treated, e.g., between .001 mg/dose and 100 mg/dose, between .01 mg/dose and 100 mg/dose, between .05 mg/dose and 50 mg/dose, between .1 mg/dose and 10 mg/dose, between 0.5 mg/dose and 5 mg/dose, between 1 mg/dose and 10 mg/dose, etc. (with all doses being approximate). Exemplary, nonlimiting concentrations of a therapeutic agent in a composition of the invention are between approximately .0001 mg and 100 mg of the therapeutic agent per milliliter of solution, e.g., the concentration may be between .001 and 100 mg/ml, between .01 and 50 mg/ml, between 0.01 and 50 mg/ml, between .1 and 10 mg/ml, etc., with all concentrations being approximate. In specific embodiments the dose of either or both the first or second therapeutic agents is, without limitation, exactly or approximately 0.1, 0.2, 0.25, 0.3, 0.4, 0.5, 0.6, 0.7, 0.75, 0.8, 0.9, 1.0, 2.0, 3.0, 4.0, or 5.0 mg or can fall within a range delimited by any two of the foregoing values. For example, in certain embodiments a sustained release formulation, e.g., an ocular implant, contains exactly or approximately 0.1, 0.2, 0.25, 0.3, 0.4, 0.5, 0.6, 0.7, 0.75, 0.9, 0.9, 1.0, 2.0, 3.0,4.0, or 5.0 mg of a therapeutic agent or an amount that falls within a range delimited by any two of the foregoing values. If the therapeutic agent is one already approved or under study for use in the disorder, a conventional dose may be used, where "conventional" means a unit dose that has been previously shown to be effective and/or is accepted in the art when used as a single agent. For example, 0.5 mg of Lucentis or 0.3 mg of Macugen may be administered. In other embodiments the dose is between 0.5 and 2 times a conventional dose.

The following sections describe a variety of therapeutic agents of use in the invention, but the invention is not limited to these agents or classes of agents or their mechanisms of action.

### A. Angiogenesis Inhibitors

Some angiogenesis inhibitors are cytotoxic agents that damage or kill target cells (e.g., endothelial cells) or trigger an immune-mediated response that results in damage to or killing of target cells. A second group includes agents that do not substantially damage or kill endothelial cells but instead inhibit their proliferation, migration, capillary tube formation, differentiation of endothelial cells from precursors thereof, or other processes associated with angiogenesis.

A variety of angiogenesis inhibitors have been developed. Vascular endothelial growth factor (VEGF) is one of the key regulators of angiogenesis. Other regulators include fibroblast growth factor 2, pigment epithelium derived growth factor (PEDF), angiopoietins, and extracellular growth factor molecules (see, e.g., Ng, E. and Adamis, A., Can. J. Ophthalmol., 40:352-68, 2005 for discussion of angiogenesis inhibitors and molecules involved in angiogenesis). Any of these regulators and/or proteins with which they interact can be a target of an angiogenesis inhibitor. VEGF-A is an endothelial cell mitogen with the ability to stimulate angiogenesis *in vivo* (Leung DW, Cachianes G, Kuang W-J, Goeddel DV, Ferrara N, Science, 246:1306-1309, 1989). Other VEGF family members include VEGF-B, VEGF-C, and VEGF-D. VEGF-A promotes endothelial cell proliferation and survival as well as vascular permeability (Ng, *supra,* and references therein). VEGF-A exists in several different isoforms containing 121, 145, 165, 189, and 208 amino acids (in humans), of which VEGF₁₆₅ maybe primarily responsible for pathological ocular neovascularization. A number of different receptors for VEGF exist, e.g., VEGFR-1, VEGFR-2, and VEGPR-3.

Agents that inhibit the activity and/or expression of VEGF, e.g., VEGF-A, or one or more VEGF receptors are referred to herein as "anti-VEGF agents". Useful agents of the invention include antibodies, antibody fragments, and nucleic acids that bind to one or more VEGF isoforms or VEGF receptors. The binding may inhibit interaction of one or more VEGF isoforms with its receptor(s). Macugen (Pfizer, Eyetech) is a VEGF nucleic acid ligand (also referred to as an aptamer) that binds to and inhibits VEGF₁₆₅ (U.S. Pat. No. 6,051,698). Lucentis (Genentech) is a humanized antibody fragment that binds and inhibits Vascular Endothelial Growth Factor A (VEGF-A) (Gaudreault, J., et al., Invest Ophthalmol. Vis. Sci. 46, 726-733 (2005) and references therein. Avastin (Genentech) is a full length humanized antibody that also binds to VEGF (reviewed in Ferrara, N. Endocr Rev., 25(4):581-611, 2004).

Another angiogenesis inhibitor of use in the invention is VEGF-Trap (Regeneron Pharmceuticals), a fusion protein containing extracellular domains and two VEGF receptors connected to the Fc region of an antibody (U.S. Pat. No. 5,844,099).

Disclosed herein are angiogenesis inhibitors that are agents that inhibit expression of one or more pro-angiogenic molecules through the cellular process referred to as RNA interference (RNAi), also referred to as "gene silencing" (Novina, C.D. and Sharp, P.A. (2004) "The RNAi revolution", Nature, 430, 161-164.). Such agents are referred to herein as RNAi agents and include siRNA and shRNA. Typically, RNAi agents are nucleic acids that include a double-stranded portion between about 17 and 29 nucleotides, e.g., 19-25, or 19 nucleotides, in length, one strand of which includes a portion (the "antisense" or "guide" strand) that is substantially or perfectly complementary (e.g., at least 70%, at least 80%, at least 90%, or 100% complementary) to a target gene over about 17-29 nucleotides, e.g., 19-25, or 19 nucleotides. Optionally the RNAi agent includes one or more single-stranded 3' overhangs. The presence of an RNAi agent in a cell typically results in sequence-specific degradation and/or translational repression of a target mRNA encoded by the target gene, thereby inhibiting its expression. RNAi agents and methods for their design and manufacture are well known in the art. See, e.g., Novina, *supra,* and references therein as well as U.S.S.N. 09/821,832 (U.S. Pub. No. 20020086356) and U.S.S.N. 10/832,248 (U.S. Pub. No. 20040229266). It will be appreciated that RNAi agents may consist entirely of nucleotides such as those found naturally in RNA and/or RNA or may comprise any of a wide variety of nucleotide analogs or may differ in other ways from the structure of naturally occurring RNA and DNA. See, e.g., U.S. Pub. Nos. 20030175950, 20040192626, 20040092470, 20050020525, 20050032733.

Disclosed herein is an angiogenesis inhibitor that is an RNAi agent, e.g., an siRNA, that inhibits expression of one or more VEGF isoforms (e.g., VEGF₁₆₅); or inhibits expression of a VEGF receptor (e.g., VEGFR1). One of ordinary skill in the art will be able to design appropriate RNAi agents based on the known sequences of these molecules (or any other target pro-angiogenic molecule including, but not limited to, angiogenin, angiopoietin, fibroblast growth factors, PEDF, etc.), which are available in public databases, e.g., GenBank.
The RNAi agent, when administered to cells, e.g., endothelial cells, *in vitro* in an appropriate amount optionally together with uptake enhancing compounds such as lipids, may inhibit expression of its target gene by at least 60%, at least 70%, at least 80%, at least 90%. The RNAi agent, when administered to the eye in an appropriate amount, may inhibit expression of its target gene by at least 60%, at least 70%, at least 80%, at least 90%, or more within at least one structure, tissue, or compartment of the eye, e.g., within the retina. Exemplary RNAi agents include, but are not limited to, the siRNA known as Cand5 (Acuity Pharmaceuticals), which inhibits expression of VEGF, Sirna-027 (Sirna Therapeutics), which inhibits expression of VEGFR-1, and siRNAs having a sequence that differs at 1, 2, or 3 positions from that of either Cand5 or Siena-027. Additional sequences and structures of RNAi agents are described in U.S.S.N. 10/294,228 (U.S. Pub. No. 20040018176), U.S.S.N. 10/764,957 (U.S. Pub. No. 20050054596). Additional nucleic acids that inhibit experssion of a target gene include antisense oligonucleotides and ribozymes (see, e.g., U.S. Pat. No. 6,818,447).

Other angiogenesis inhibitors include various endogenous or synthetic peptides such as angiostatin, arresten, canstatin, combstatin, endostatin, thrombospondin, and tumstatin. Other antiangiogenic molecules include thalidomide and its antiangiogenic derivatives such as iMiDs (Barnias A, Dimopoulos MA. Eur J Intern Med. 14(8):459-469, 2003; Bartlett JB, Dredge K, Dalgleish AG. Nat Rev Cancer. 4(4):314-22,2004).

Administration of certain angiogenesis inhibitors, e.g., anti-VEGF agents such as Avastin or Lucentis by intravitreal injection results in a rapid improvement in the condition of a subject's eye. While not wishing to be bound by any theory, this rapid improvement may at least in part occur due to diminished vessel leakage and reduced macular edema. These effects may, at least in the short term (i.e., over the first 1-2 weeks following treatment), be at least as significant as any inhibition of blood vessel development or growth that occurs during this time period. Therapeutic agents that rapidly reduce macular edema may be of particular use to cause rapid improvement in the condition of a subject's eye. Since VEGF is an inducer of vascular permeability, anti-VEGF agents may be especially effective for these purposes. Endostatin has the ability to reduce vascular permeability (see, e.g., Campochiaro, PA., Expert Opin Biol Ther., 4(9): 1395-402, 2004). In certain embodiments of the invention endostatin and an anti-VEGF agent, e.g., an antibody, antibody fragment, or aptamer that binds to VEGF or to a VEGF receptor are administered. Either or both agents may be contained in a liquid composition or a sustained release formulation.

### B. Complement Pathways and Complement Inhibitors

The complement system plays a crucial role in a number of physiological processes including the response to injury and defense against foreign entities such as infectious agents. The complement system is also known to play a role in a number of diseases (Makrides, SC, Pharm Rev., 50(1): 59-87, 1998). The complement system comprises more than 30 serum and cellular proteins that are involved in two major pathways, known as the classical and alternative pathways (Kuby Immunology, 2000).

The classical pathway is usually triggered by binding of a complex of antigen and IgM or IgG antibody to C1 (though certain other activators can also initiate the pathway). Activated C1 cleaves C4 and C2 to produce C4a and C4b, in addition to C2a and C2b. C4b and C2a combine to form C3 convertase, which cleaves C3 to form C3a and C3b. Binding of C3b to C3 convertase produces C5 convertase, which cleaves C5 into C5a and C5b. C3a, C4a, and C5a are anaphylotoxins and mediate multiple reactions in the acute inflammatory response. C3a and C5a are also chemotactic factors that attract immune system cells such as neutrophils. C3 and C5 convertase activity is controlled by a number of endogenous members of the Regulators of Complement Activation (RCA) family, also called Complement Control Protein (CCP) family, which includes complement receptor type 1 (CR1; C3b:C4b receptor), complement receptor type 2 (CR2), membrane cofactor protein (MCP; CD46), decay-accelerating factor (DAF), factor H (fH), and C4b-binding protein (C4bp). RCA proteins are described in U.S. Pat. No. 6,897,290.

The alternative pathway is initiated by microbial surfaces and various complex polysaccharides. In this pathway, C3b, resulting from cleavage of C3, which occurs spontaneously at a low level, binds to targets on cell surfaces and forms a complex with factor B, which is later cleaved by factor D, resulting in a C3 convertase. Cleavage of C3 and binding of another molecule of C3b to the C3 convertase gives rise to a C5 convertase. C3 and C5 convertases of this pathway are regulated by CR1, DAF, MCP, and fH. The mode of action of these proteins involves either decay accelerating activity (i.e., ability to dissociate convertases), ability to serve as cofactors in the degradation of C3b or C4b by factor I, or both.

The C5 convertases produced in both pathways cleave C5 to produce C5a and C5b. C5b then binds to C6, C7, and C8 to form C5b-8, which catalyzes polymerization of C9 to form the C5b-9 membrane attack complex (MAC). The MAC inserts itself into target cell membranes and causes cell lysis. Small amounts of MAC on the membrane of cells may have a variety of consequences other than cell death.

A third complement pathway, the lectin complement pathway is initiated by binding of mannose-binding lectin (MBL) and MBL-associated serine protease (MASP) to carbohydrates. In-the human lectin pathway, MASP-1 and MASP-2 are involved in the proteolysis of C4, C2 and C3, leading to a C3 convertase described above.

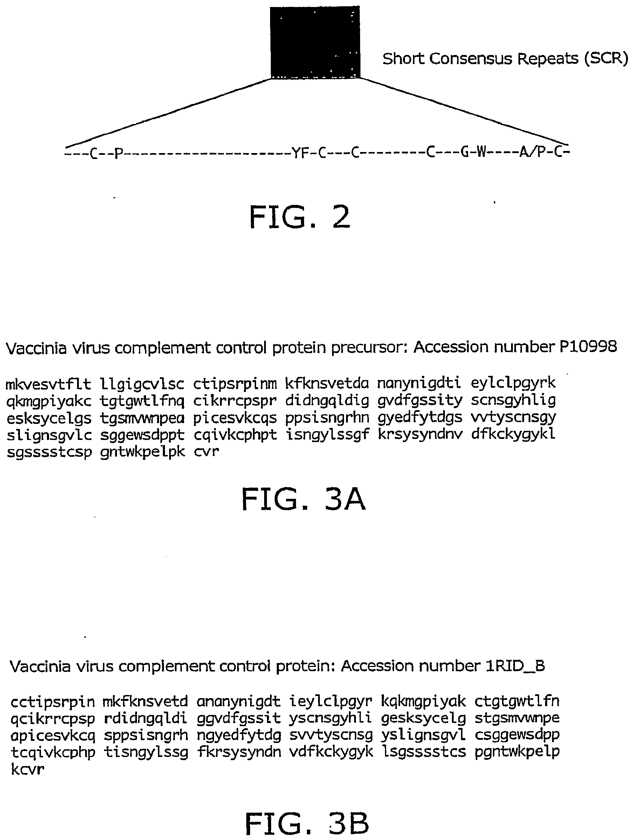

Complement activity is regulated by various mammalian proteins referred to as complement control proteins (CCPs). These proteins differ with respect to ligand specificity and mechanism(s) of complement inhibition (Lisczewski, MK and Atkinson, JP, in *The Human Complement System in Health and Disease,* eds. Volanakis, JE and Frank, MM, Decker, New York, pp. 149-66,1998). They may accelerate the normal decay of convertases and/or function as cofactors for factor I, to enzymatically cleave C3b and/or C4b into smaller fragments. CCPs are characterized by the presence of multiple (typically 4-56) homologous motifs known as short consensus repeats (SCR), complement control protein (CCP) modules, or SUSHI domains (Reid, KBM and Day, AJ, Immunol Today, 10:177-80, 1989). These domains, consisting of approximately 50-70 amino acids, typically about 60 amino acids, are characterized by a conserved motif that includes four disulfide-bonded cysteines (two disulfide bonds), proline, tryptophan, and many hydrophobic residues. Figure 2 shows an SCR consensus sequence. Any particular SCR may differ from the consensus at one or more positions.

In the present invention the second agent is a complement inhibitor selected from the group consisting of a compstatin analogue having at least a 5-fold higher complement inhibiting activity than compstatin, a monoclonal antibody or monoclonal antibody fragment that binds to C3, and an antibody or antibody fragment that binds to C5, factor B or factor D. A complement inhibitor may inhibit complement activation, e.g., inhibit activation of one or more complement proteins. For example, it may inhibit cleavage of an inactive complement protein to its active form. Complement inhibitors include, but are not limited to, (i) viral or mammalian complement control or complement inhibiting proteins as well as fragments or variants thereof that retain the ability to inhibit complement; (ii) compstatin and derivatives thereof; (iii) complement receptor antagonists. The following sections describe complement inhibitors of use in various embodiments of the invention.

### Compounds that Inhibit C3 Activation or Activity

In certain embodiments of the invention the complement inhibitor inhibits activation of C3. Exemplary compounds include compounds that bind to C3 and inhibit its cleavage. The compound may be a compstatin analog. Compstatin is a cyclic peptide identified using phage display that binds to complement component C3 and
inhibits complement activation. Compstatin inhibits cleavage of C3 to C3a and C3b by convertase. Since C3 is a central component of all three pathways of complement activation, compstatin and analogs thereof are able to inhibit activation of the converging protein of all three pathways. Without wishing to be bound by any theory, the ability of compstatin and analogs thereof to inhibit the alternative pathway of complement activation may contribute significantly to efficacy in certain of the disorders described herein.

The invention encompasses the recognition that compstatin and analogs thereof possess unique and unexpected advantages as compared with certain other complement inhibitors, particularly for sustained release, in a variety of eye disorders. The relatively low molecular weight (∼1.6 kD) and various other properties of compstatin analogs facilitate their incorporation into sustained delivery formulations and devices suitable for providing therapeutic concentrations in the eye. In certain embodiments a compstatin analog is delivered in a sustained manner over a prolonged period of time such as 1-2 weeks, 2-4 weeks, 1-3 months, 3-6 months, 6-12 months, 1-2 years, 2-5 years, or 5-10 years.

Compstatin is described in U.S. Pat. No. 6,319,897. Compstatin has the sequence Ile- [Cys-Val-Val-Gln-Asp-Trp-Gly-His-His-Arg-Cys]-Thr (SEQ ID NO: 8), with the disulfide bond between the two cysteines denoted by brackets. It is an N-terminal cyclic region of a larger peptide (SEQ ID NO: 1 in U.S. Pat. No. 6,319,897) that also shows complement inhibiting activity. A number of fragments and variants of compstatin inhibit complement have been identified. See, e.g. SEQ ID NOs: 13, 15, 20, 21, and 22 in U.S. Pat. No. 6,319,897.

A variety of compstatin analogs that have higher complement inhibiting activity than compstatin have been synthesized. See WO2004/026328 (PCT/US2003/029653), Morikis, D., et al., Biochem Soc Trans. 32(Pt 1):28-32, 2004, Mallik, B., et al., J. Med. Chem., 274-286, 2005, and/or in Katragadda, M., et al. J. Med. Chem., 49: 4616-4622,2006. Complement inhibiting peptides and peptidomimetics described therein can be used in the present invention.

As used herein, the term "compstatin analog" includes compstatin and any complement inhibiting analog thereof and is used interchangeably with "compstatin derivative". The term "compstatin analog" encompasses compstatin and other compounds designed or identified based on compstatin and whose complement inhibiting activity is at least 50% as great as that of compstatin as measured, e.g., using any complement activation assay accepted in the art or substantially similar or equivalent assays. Certain compstatin analogs and suitable assays are described in U.S. Pat. No. 6,319,897, WO2004/026328, Morikis, *supra,* Mallik, *supra,* and/or Katragadda 2006, *supra.* The assay may, for example, measure alternative pathway-mediated erythrocyte lysis or be an ELISA assay (see Examples 5 and 6 of copending applications USSN 11/544,389 and PCT/US06/39397). The invention includes embodiments in which any one or more of the compstatin analogs or compositions described herein is used. In the invention a peptide having at least 5-fold higher complement inhibiting activity than compstatin, preferably at least 10-fold higher activity, etc., is used.

Compstatin and any of its analogs may be acetylated or amidated, e.g., at the N-terminus and/or C-terminus. For example, Compstatin and any of its analogs may be acetylated at the N-terminus and amidated at the C-terminus. Consistent with usage in the art, "compstatin," as used herein, and the activities of compstatin analogs described herein relative to that of compstatin, refer to compstatin amidated at the C-terminus (Mallik, 2005, *supra*).

Concatamers or multimers of a compstatin analog thereof are also of use in the present invention. A supramolecular complex comprising a compstatin analog is of use in the Invention.

The activity of a compstatin analog may be expressed in terms of its IC₅₀ (the concentration of the compound that inhibits complement activation by 50%), e.g., at a particular plasma concentration, with a lower IC₅₀ indicating a higher activity as recognized in the art.

Certain modifications are known to reduce or eliminate complement inhibiting activity and may be explicitly excluded from any embodiment of the invention. The IC₅₀ of compstatin has been reported as 12 µM using a complement activity assay comprising measuring alternative pathway-mediated erythrocyte lysis assay (WO2004/026328). In certain embodiments of the invention the activity of the compstatin analog is between 10 and 50 times as great as that of compstatin, or between 50 and 99 times as great as that of compstatin. In certain embodiments of the invention the activity of the compstatin analog is between 99 and 264 times that of compstatin. For example, the activity may be 100, 110, 120, 130, 140, 150, 160, 170, 180, 190, 200, 210, 220, 230, 240, 250, 260, or 264 times as great as that of compstatin. In certain embodiments the activity is between 264 and 300, 300 and 350, 350 and 400, or 400 and 500 times as great as that of compstatin. The invention further contemplates compstatin analogs having activities between 500 and 1000 times that of compstatin.

The K_{d} of compstatin binding to C3 has been reported as 1.3 µM using isothermal titration calorimetry (Katragadda, et al., J. Biol. Chem., 279(53), 54987-54995,2004). Binding affinity of a variety of compstatin analogs for C3 has been correlated with their activity, with a lower K_{d} indicating a higher binding affinity, as recognized in the art. A linear correlation between binding affinity and activity was shown for certain analogs tested (Katragadda, 2004, *supra*; Katragadda 2006, *supra*)*.* In certain embodiments of the invention the compstatin analog binds to C3 with a K_{d} of between 0.05 µM and 0.1 µM, between 0.025 µM and 0.05 µM, between 0.015 µM and 0.025 µM, between 0.01 µM and 0.015 µM, or between 0.001 µM and 0.01µM. In certain embodiments the IC₅₀ of the compstatin analog is between about 0.2 µM and about 0.5 µM. In certain embodiments the IC₅₀ of the compstatin analog is between about 0.1 µM and about 0.2 µM. In certain embodiments the IC₅₀ of the compstatin analog is between about 0.05 µM and about 0.1 µM. In certain embodiments the IC₅₀ of the compstatin analog is between about 0.001 µM and about 0.05 µM.

Compounds "designed or identified based on compstatin" include, but are not limited to, compounds that comprise an amino acid chain whose sequence is obtained by (i) modifying the sequence of compstatin (e.g., replacing one or more amino acids of the sequence of compstatin with a different amino acid or amino acid analog, inserting one or more amino acids or amino acid analogs into the sequence of compstatin, or deleting one or more amino acids from the sequence of compstatin); (ii) selection from a phage display peptides library in which one or more amino acids of compstatin is randomized, and optionally further modified according to method (i); or (iii) identified by screening for compounds that compete with compstatin or any analog thereof obtained by methods (i) or (ii) for binding to C3 or a fragment thereof. Many useful compstatin analogs comprise a hydrophobic cluster, a β-turn, and a disulfide bridge.

In certain embodiments of the invention the sequence of the compstatin analog comprises or consists essentially of a sequence that is obtained by making 1, 2, 3, or 4 substitutions in the sequence of compstatin, i.e., 1, 2, 3, or 4 amino acids in the sequence of compstatin is replaced by a different standard amino acid or by anon-standard amino acid. In certain embodiments of the invention the amino acid at position 4 is altered. In certain embodiments of the invention the amino acid at position 9 is altered. In certain embodiments of the invention the amino acids at positions 4 and 9 are altered. In certain embodiments of the invention only the amino acids at positions 4 and 9 are altered. In certain embodiments of the invention the amino acid at position 4 or 9 is altered, or in certain embodiments both amino acids 4 and 9 are altered, and in addition up to 2 amino acids located at positions selected from 1, 7, 10, 11, and 13 are altered. In certain embodiments of the invention the amino acids at positions 4, 7, and 9 are altered. In certain embodiments of the invention amino acids at position 2, 12, or both are altered, provided that the alteration preserves the ability of the compound to be cyclized. Such alteration(s) at positions 2 and/or 12 may be in addition to the alteration(s) at position 1, 4, 7, 9, 10, 11, and/or 13. Optionally the sequence of any of the compstatin analogs whose sequence is obtained by replacing one or more amino acids of compstatin sequence further includes up to 1, 2, or 3 additional amino acids at the C-terminus. In one embodiment, the additional amino acid is Gly. Optionally the sequence of any of the compstatin analogs whose sequence is obtained by replacing one or more amino acids of compstatin sequence further includes up to 5, or up to 10 additional amino acids at the C-terminus. It should be understood that compstatin analogs may have any one or more of the characteristics or features of the various embodiments described herein, and characteristics or features of any embodiment may additionally characterize any other embodiment described herein, unless otherwise stated or evident from the context. In certain embodiments of the invention the sequence of the compstatin analog comprises or consists essentially of a sequence shown in the upper portion of Figure 7, in which X4 and X9 represent modifiable side chains.

Certain compstatin analogs having somewhat greater activity than compstatin contain only standard amino acids ("standard amino acids" are glycine, leucine, isoleucine, valine, alanine, phenylalanine, tyrosine, tryptophan, aspartic acid, asparagine, glutamic acid, glutamine, cysteine, methionine, arginine, lysine, proline, serine, threonine and histidine). Certain compstatin analogs having improved activity incorporate one or more non-standard amino acids. Useful non-standard amino acids include singly and multiply halogenated (e.g., fluorinated) amino acids, D-amino acids, homo-amino acids, N-alkyl amino acids, dehydroamino acids, aromatic amino acids (other than phenylalanine, tyrosine and tryptophan), ortho-, meta- or para-aminobenzoic acid, phospho-amino acids, methoxylated amino acids, and α,α-disubstituted amino acids. In certain embodiments of the invention, a compstatin analog is designed by replacing one or more L-amino acids in a compstatin analog described elsewhere herein with the corresponding D-amino acid.

Exemplary non-standard amino acids of use include 2-naphthylalanine (2-NaI), 1-naphthylalanine (1-NaI), 2-indanylglycine carboxylic acid (2Ig1), dihydrotrpytophan (Dht), 4-benzoyl-L-phenylalanine (Bpa), 2-α-aminobutyric acid (2-Abu), 3-α-aminobutyric acid (3-Abu), 4-α-aminobutyric acid (4-Abu), cyclohexylalanine (Cha), homocyclohexylalanine (hCha), 4-fluoro-L-tryptophan (4fW), 5-fluoro-L-tryptophan (5fW), 6-fluoro-L-tryptophan (6fW), 4-hydroxy-L-tryptophan (4OH-W), 5-hydroxy-L-tryptophan (5OH-W), 6-hydroxy-L-tryptophan (6OH-W), 1-methyl-L-tryptophan (1MeW), 4-methyl-L-tryptophan (4MeW), 5-methyl-L-tryptophan (5MeW), 7-aza-L-tryptophan (7aW), α-methyl-L-tryptophan (αMeW), (β-methyl-L-tryptophan (βMeW), N-methyl-L-tryptophan (NMeW), ornithine (orn), citrulline, norleucine, γ-glutamic acid, etc.

In certain embodiments of the invention the compstatin analog comprises one or more Trp analogs (e.g., at position 4 and/or 7 relative to the sequence of compstatin). Exemplary Trp analogs are mentioned above. See also Beene, et. al. Biochemistry 41: 10262-10269, 2002 (describing, *inter alia*, singly- and multiply-halogenated Trp analogs); Babitzke & Yanofsky, J. Biol. Chem. 270: 12452-12456, 1995 (describing, *inter alia*, methylated and halogenated Trp and other Trp and indole analogs); and U.S. Patents 6,214,790, 6,169,057, 5,776,970,4,870,097,4,576,750 and 4,299,838. Other Trp analogs include variants substituted (e.g., by a methyl group) at the α or β carbon and, optionally, also at one or more positions of the indole ring. Amino acids comprising two or more aromatic rings, including substituted, unsubstituted, or alternatively substituted variants thereof, are of interest as Trp analogs.

In certain embodiments the Trp analog has increased hydrophobic character relative to Trp. For example, the indole ring may be substituted by one or more alkyl (e.g., methyl) groups. In certain embodiments the Trp analog participates in a hydrophobic interaction with C3. Such a Trp analog may be located, e.g., at position 4 relative to the sequence of compstatin. In certain embodiments the Trp analog comprises a substituted or unsubstituted bicyclic aromatic ring component or two or more substituted or unsubstituted monocyclic aromatic ring components.

In certain embodiments the Trp analog has increased propensity to form hydrogen bonds with C3 relative to Trp but does not have increased hydrophobic character relative to Trp. The Trp analog may have increased polarity relative to Trp and/or an increased ability to participate in an electrostatic interaction with a hydrogen bond donor on C3. Certain exemplary Trp analogs with an increased hydrogen bond forming character comprise an electronegative substituent on the indole ring. Such a Trp analog may be located, e.g., at position 7 relative to the sequence of compstatin.

In certain embodiments of the invention the compstatin analog comprises one or more Ala analogs (e.g., at position 9 relative to the sequence of compstatin), e.g., Ala analogs that are identical to Ala except that they include one or more CH₂ groups in the side chain. In certain embodiments the Ala analog is an unbranched single methyl amino acid such as 2-Abu. In certain embodiments of the invention the compstatin analog comprises one or more Trp analogs (e.g., at position 4 and/or 7 relative to the sequence of compstatin) and an Ala analog (e.g., at position 9 relative to the sequence of campstatin).

In certain embodiments of the invention the compstatin analog is a compound that comprises a peptide that has a sequence of (X'aa)ₙ- Gln - Asp - Xaa - Gly-(X"aa)ₘ, (SEQ ID NO: 2) wherein each X'aa and each X"aa is an independently selected amino acid or amino acid analog, wherein Xaa is Trp or an analog of Trp, and wherein n>1 and m>1 and n+m is between 5 and 21. The peptide has a core sequence of Gln - Asp - Xaa - Gly, where Xaa is Trp or an analog of Trp, e.g., an analog of Trp having increased propensity to form hydrogen bonds with an H-bond donor relative to Trp but, in certain embodiments, not having increased hydrophobic character relative to Trp. For example, the analog may be one in which the indole ring of Trp is substituted with an electronegative moiety, e.g., a halogen such as fluorine. In one embodiment Xaa is 5-fluorotryptophan. Absent evidence to the contrary, one of skill in the art would recognize that any non-naturally occurring peptide whose sequence comprises this core sequence and that inhibits complement activation and/or binds to C3 will have been designed based on the sequence of compstatin. In an alternative embodiment Xaa is an amino acid or amino acid analog other than a Trp analog that allows the Gln - Asp - Xaa - Gly peptide to form a β-turn.

In certain embodiments of the invention the peptide has a core sequence of X'aa-Gln - Asp - Xaa - Gly (SEQ ID NO: 3), where X'aa and Xaa are selected from Trp and analogs of Trp. In certain embodiments of the invention the peptide has a core sequence of X'aa-Gln - Asp - Xaa - Gly (SEQ ID NO: 3), where X'aa and Xaa are selected from Trp, analogs of Trp, and other amino acids or amino acid analogs comprising at least one aromatic ring. In certain embodiments of the invention the core sequence forms a β-turn in the context of the peptide. The β-turn may be flexible, allowing the peptide to assume two or more conformations as assessed for example, using nuclear magnetic resonance (NMR). In certain embodiments X'aa is an analog of Trp that comprises a substituted or unsubstituted bicyclic aromatic ring component or two or more substituted or unsubstituted monocyclic aromatic ring components. In certain embodiments of the invention X'aa is selected from the group consisting of 2-napthylalanine, 1-napthylalanine, 2-indanylglycine carboxylic acid, dihydrotryptophan, and benzoylphenylalanine. In certain embodiments of the invention X'aa is an analog of Trp that has increased hydrophobic character relative to Trp. For example, X'aa may be 1-methyltryptophan. In certain embodiments of the invention Xaa is an analog of Trp that has increased propensity to form hydrogen bonds relative to Trp but, in certain embodiments, not having increased hydrophobic character relative to Trp. In certain embodiments of the invention the analog of Trp that has increased propensity to form hydrogen bonds relative to Trp comprises a modification on the indole ring of Trp, e.g., at position 5, such as a substitution of a halogen atom for an H atom at position 5. For example, Xaa may be 5-fluorotryptophan.

In certain embodiments of the invention the peptide has a core sequence of X'aa-Gln - Asp - Xaa - Gly-X"aa (SEQ ID NO: 4), where X'aa and Xaa are each independently selected from Trp and analogs of Trp and X"aa is selected from His, Ala, analogs of Ala, Phe, and Trp. In certain embodiments of the invention X'aa is an analog of Trp that has increased hydrophobic character relative to Trp, such as 1-methyltryptophan or another Trp analog having an alkyl substituent on the indole ring (e.g., at position 1, 4, 5, or 6). In certain embodiments X'aa is an analog of Trp that comprises a substituted or unsubstituted bicyclic aromatic ring component or two or more substituted or unsubstituted monocyclic aromatic ring components. In certain embodiments of the invention X'aa is selected from the group consisting of 2-napthylalanine, 1-napthylalanine, 2-indanylglycine carboxylic acid, dihydrotryptophan, and benzoylphenylalanine. In certain embodiments of the invention Xaa is an analog of Trp that has increased propensity to form hydrogen bonds with C3 relative to Trp but, in certain embodiments, not having increased hydrophobic character relative to Trp. In certain embodiments of the invention the analog of Trp that has increased propensity to form hydrogen bonds relative to Trp comprises a modification on the indole ring of Trp, e.g., at position 5, such as a substitution of a halogen atom for an H atom at position 5. For example, Xaa may be 5-fluorotryptophan. In certain embodiments X"aa is Ala or an analog of Ala such as Abu or another unbranched single methyl amino acid. In certain embodiments of the invention the peptide has a core sequence of X'aa-Gln - Asp - Xaa - Gly-X"aa (SEQ ID NO: 4), where X'aa and Xaa are each independently selected from Trp, analogs of Trp, and amino acids or amino acid analogs comprising at least one aromatic side chain, and X"aa is selected from His, Ala, analogs of Ala, Phe, and Trp. In certain embodiments X"aa is selected from analogs of Trp, aromatic amino acids, and aromatic amino acid analogs.

In certain preferred embodiments of the invention the peptide is cyclic. The peptide may be cyclized via a bond between any two amino acids, one of which is (X'aa)ₙ and the other of which is located within (X"aa)ₘ. In certain embodiments the cyclic portion of the peptide is between 9 and 15 amino acids in length, e.g., 10-12 amino acids in length. In certain embodiments the cyclic portion of the peptide is 11 amino acids in length, with a bond (e.g., a disulfide bond) between amino acids at positions 2 and 12. For example, the peptide may be 13 amino acids long, with a bond between amino acids at positions 2 and 12 resulting in a cyclic portion 11 amino acids in length.

In certain embodiments the peptide comprises or consists of the sequence X'aa1 - X'aa2 - X'aa3 - X'aa4 -Gln-Asp-Xaa-Gly X"aa1- X"aa2- X"aa3- X"aa4- X"aa5 (SEQ ID NO: 5). In certain embodiments X'aa4 and Xaa are selected from Trp and analogs of Trp, and X'aa1, X'aa2, X'aa3, X"aa1, X"aa2, X"aa3, X"aa4, and X"aa5 are independently selected from among amino acids and amino acid analogs. In certain embodiments X'aa4 and Xaa are selected from aromatic amino acids and aromatic amino acid analogs. Any one or more of X'aa1, X'aa2, X'aa3, X"aa1, X"aa2, X"aa3, X"aa4, and X"aa5 may be identical to the amino acid at the corresponding position in compstatin. In one embodiment, X"aa1 is Ala or a single methyl unbranched amino acid. The peptide may be cyclized via a covalent bond between (i) X'aa1, X'aa2, or X'aa3; and (ii) X"aa2, X"aa3, X"aa4 or X"aa5. In one embodiment the peptide is cyclized via a covalent bond between X'aa2 and X"aa4. In one embodiment the covalently bound amino acid are each Cys and the covalent bond is a disulfide (S-S) bond. In other embodiments the covalent bond is a C-C, C-O, C-S, or C-N bond. In certain embodiments one of the covalently bound residues is an amino acid or amino acid analog having a side chain that comprises a primary or secondary amine, the other covalently bound residue is an amino acid or amino acid analog having a side chain that comprises a carboxylic acid group, and the covalent bond is an amide bond. Amino acids or amino acid analogs having a side chain that comprises a primary or secondary amine include lysine and diaminocarboxylic acids of general structure NH₂(CH₂)ₙCH(NH₂)COOH such as 2,3-diaminopropionic acid (dapa), 2,4-diaminobutyric acid (daba), and ornithine (orn), wherein n = 1 (dapa), 2 (daba), and 3 (orn), respectively. Examples of amino acids having a side chain that comprises a carboxylic acid group include dicarboxylic amino acids such as glutamic acid and aspartic acid. Analogs such as beta-hydroxy-L-glutamic acid may also be used.

In certain embodiments, the compstatin analog is a compound that comprises a peptide having a sequence:
Xaa1 - Cys - Val- Xaa2 - Gln - Asp - Xaa2* - Gly - Xaa3 - His - Arg - Cys - Xaa4 (SEQ ID NO: 6); wherein:
   Xaa1 is Ile, Val, Leu, B¹-Ile, B¹-Val, B¹-Leu or a dipeptide comprising Gly-Ile or B¹-Gly-Ile, and B¹ represents a first blocking moiety;
   Xaa2 and Xaa2* are independently selected from Trp and analogs of Trp;
   Xaa3 is His, Ala or an analog of Ala, Phe, Trp, or an analog of Trp;
   Xaa4 is L-Thr, D-Thr, Ile, Val, Gly, a dipeptide selected from Thr-Ala and Thr-Asn, or a tripeptide comprising Thr-Ala-Asn, wherein a carboxy terminal -OH of any of the L-Thr, D-Thr, Ile, Val, Gly, Ala, or Asn optionally is replaced by a second blocking moiety B²; and the two Cys residues are joined by a disulfide bond.

In other embodiments Xaa1 is absent or is any amino acid or amino acid analog, and Xaa2, Xaa2*, Xaa3, and Xaa4 are as defined above. If Xaa1 is absent, the N-terminal Cys residue may have a blocking moiety B¹ attached thereto.

In another embodiment, Xaa4 is any amino acid or amino acid analog and Xaa1, Xaa2, Xaa2*, and Xaa3 are as defined above. In another embodiment Xaa4 is a dipeptide selected from the group consisting of: Thr-Ala and Thr-Asn, wherein the carboxy terminal -OH or the Ala or Asn is optionally replaced by a second blocking moiety B².

In any of the embodiments of the compstatin analog of SEQ ID NO: 6, Xaa2 may be Trp.

In any of the embodiments of the compstatin analog of SEQ ID NO: 6, Xaa2 may be an analog of Trp comprising a substituted or unsubstituted bicyclic aromatic ring component or two or more substituted or unsubstituted monocyclic aromatic ring components. For example, the analog of Trp may be selected from 2-naphthylalanine (2-Nal), 1-naphthylalanine (1-Nal), 2-indanylglycine carboxylic acid (Ig1), dihydrotrpytophan (Dht), and 4-benzoyl-L-phenylalanine.

In any of the embodiments of the compstatin analog of SEQ ID NO: 6, Xaa2 may be an analog of Trp having increased hydrophobic character relative to Trp. For example, the analog of Trp may be selected from 1-methyltryptophan, 4-methyltryptophan, 5-methyltryptophan, and 6-methyltryptophan. In one embodiment, the analog of Trp is 1-methyltryptophan. In one embodiment, Xaa2 is 1-methyltryptophan, Xaa2* is Trp, Xaa3 is Ala, and the other amino acids are identical to those of compstatin.

In any of the embodiments of the compstatin analog of SEQ ID NO: 6, Xaa2* may be an analog of Trp such as an analog of Trp having increased hydrogen bond forming propensity with C3 relative to Trp, which, in certain embodiments, does not have increased hydrophobic character relative to Trp. In certain embodiments the analog of Trp comprises an electronegative substituent on the indole ring. For example, the analog of Trp may be selected from 5-fluorotryptophan and 6-fluorotryptophan.

In certain embodiments of the invention Xaa2 is Trp and Xaa2* is an analog of Trp having increased hydrogen bond forming propensity with C3 relative to Trp which, in certain embodiments, does not have increased hydrophobic character relative to Trp. In certain embodiments of the compstatin analog of SEQ ID NO: 6, Xaa2 is analog of Trp having increased hydrophobic character relative to Trp such as an analog of Trp selected from 1-methyltryptophan, 4-methyltryptophan, 5-methyltryptophan, and 6-methyltryptophan, and and Xaa2* is an analog of Trp having increased hydrogen bond forming propensity with C3 relative to Trp which, in certain embodiments, does not have increased hydrophobic character relative to Trp. For example, in one embodiment Xaa2 is methyltryptophan and Xaa2* is 5-fluorotryptophan.

In certain of the afore-mentioned embodiments, Xaa3 is Ala. In certain of the afore-mentioned embodiments Xaa3 is a single methyl unbranched amino acid, e.g., Abu.

In certain embodiments the invention employs a compstatin analog of SEQ ID NO: 6, as described above, wherein Xaa2 and Xaa2* are independently selected from Trp, analogs of Trp, and other amino acids or amino acid analogs that comprise at least one aromatic ring, and Xaa3 is His, Ala or an analog of Ala, Phe, Trp, an analog of Trp, or another aromatic amino acid or aromatic amino acid analog.

In certain embodiments of the invention the blocking moiety present at the N- or C-terminus of any of the compstatin analogs described herein is any moiety that stabilizes a peptide against degradation that would otherwise occur in mammalian (e.g., human or non-human primate) blood or vitreous. For example, blocking moiety B¹ could be any moiety that alters the structure of the N-terminus of a peptide so as to inhibit cleavage of a peptide bond between the N-terminal amino acid of the peptide and the adjacent amino acid. Blocking moiety B² could be any moiety that alters the structure of the C-terminus of a peptide so as to inhibit cleavage of a peptide bond between the C-terminal amino acid of the peptide and the adjacent amino acid. Any suitable blocking moieties known in the art could be used. In certain embodiments of the invention blocking moiety B¹ comprises an acyl group (i.e., the portion of a carboxylic acid that remains following removal of the -OH group). The acyl group typically comprises between 1 and 12 carbons, e.g., between 1 and 6 carbons. For example, in certain embodiments of the invention blocking moiety B¹ is selected from the group consisting of: formyl, acetyl, proprionyl, butyryl, isobutyryl, valeryl, isovaleryl, etc. In one embodiment, the blocking moiety B¹ is an acetyl group, i.e., Xaa1 is Ac-Ile, Ac-Val, Ac-Leu, or Ac-Gly-Ile.

In certain embodiments of the invention blocking moiety B² is a primary or secondary amine (-NH₂ or -NHR¹, wherein R is an organic moiety such as an alkyl group).

In certain embodiments of the invention blocking moiety B¹ is any moiety that neutralizes or reduces the negative charge that may otherwise be present at the N-terminus at physiological pH. In certain embodiments of the invention blocking moiety B² is any moiety that neutralizes or reduces the negative charge that may otherwise be present at the C-terminus at physiological pH.

In certain embodiments of the invention, the compstatin analog is acetylated or amidated at the N-terminus and/or C-terminus, respectively. A compstatin analog may be acetylated at the N-terminus, amidated at the C-terminus, and or both acetylated at the N-terminus and amidated at the C-terminus. In certain embodiments of the invention a compstatin analog comprises an alkyl or aryl group at the N-terminus rather than an acetyl group.

In certain embodiments, the compstatin analog is a compound that comprises a peptide having a sequence:

Xaa1 - Cys - Val - Xaa2 - Gln - Asp - Xaa2* - Gly - Xaa3 - His - Arg - Cys - Xaa4 (SEQ ID NO: 7); wherein:
Xaa1 is Ile, Val, Leu, Ac-Ile, Ac-Val, Ac-Leu or a dipeptide comprising Gly-Ile or Ac-Gly-Ile;
Xaa2 and Xaa2* are independently selected from Trp and analogs of Trp;
Xaa3 is His, Ala or an analog of Ala, Phe, Trp, or an analog of Trp;
Xaa4 is L-Thr, D-Thr, Ile, Val, Gly, a dipeptide selected from Thr-Ala and Thr-Asn, or a tripeptide comprising Thr-Ala-Asn, wherein a carboxy terminal -OH of any of L-Thr, D-Thr, Ile, Val, Gly, Ala, or Asn optionally is replaced by -NH₂; and
the two Cys residues are joined by a disulfide bond.

Xaa1, Xaa2, Xaa2*, Xaa3, and Xaa4 are as described above for the various embodiments of SEQ ID NO: 6. For example, in certain embodiments Xaa2* is Trp. In certain embodiments Xaa2 is an analog of Trp having increased hydrophobic character relative to Trp, e.g., 1-methyltryptophan. In certain embodiments Xaa3 is Ala. In certain embodiments Xaa3 is a single methyl unbranched amino acid.

In certain embodiments of the invention Xaa1 is Ile and Xaa4 is L-Thr.

In certain embodiments of the invention Xaa1 is Ile, Xaa2* is Trp, and Xaa4 is L-Thr.

In certain embodiments the invention utilizes a compstatin analog of SEQ ID NO: 7, as described above, wherein Xaa2 and Xaa2* are independently selected from Trp, analogs of Trp, other amino acids or aromatic amino acid analogs, and
Xaa3 is His, Ala or an analog of Ala, Phe, Trp, an analog of Trp, or another aromatic amino acid or aromatic amino acid analog.

In certain embodiments of any of the compstatin analogs described herein, Xaa3 is an analog of His.
Table 1 provides a non-limiting list of compstatin analogs useful in the present invention (excluding Ac-compstatin). The analogs are referred to in abbreviated form in the left column by indicating specific modifications at designated positions (1-13) as compared to the parent peptide, compstatin (amidated at the C-terminus). Unless otherwise indicated, peptides are amidated at the C-terminus. Bold text is used to indicate certain modifications. Activity relative to compstatin (in this case compstatin amidated at the C-terminus) is based on published data and assays described therein (WO2004/026326, Mallik, 2005; Katragadda, 2006). Where multiple publications reporting an activity were consulted, the more recently published value is used, and it will be recognized that values may be adjusted in the case of differences between assays. It will also be appreciated that the peptides listed in Table 1 are cyclized via a disulfide bond between the two Cys residues when used in the invention.

**Table 1**

| **Peptide** | **Sequence** | **SEQ ID NO:** | **Activity over compstatin** |
|---|---|---|---|
| Compstatin | *H*-ICVVQDWGHHRCT-*CONH2* | 8 | * |
| Ac-compstatin | *Ac*-ICVVQDWGHHRCT-*CONH2* | 9 | 3xmore |
| Ac-V4Y/H9A | *Ac*-ICV**Y**QDWG**A**HRCT-*CONH2* | 10 | 14xmore |
| Ac-V4W/H9A-OH | *Ac*-ICV**W**QDWG**A**HRCT-*COOH* | 11 | 27xmore |
| Ac-V4W/H9A | *Ac*-ICV**W**QDWG**A**HRCT-*CONH2* | 12 | 45xmore |
| Ac-V4W/H9A/T13dT-OH | *Ac*-ICV**W**QDWG**A**HRC**dT**-*COOH* | 13 | 55xmore |
| Ac-V4(2-Nal)/H9A | *Ac*-ICV**(2-Nal)**QDWG**A**HRCT-*CONH2* | 14 | 99xmore |
| Ac V4(2-Nal)/H9A-OH | *Ac*-ICV**(2-Nal)**QD7WG**A**HRCT-*COOH* | 15 | 38xmore |
| Ac V4(1-Nal)/H9A -OH | *Ac*-ICV**(1-Nal)**QDWG**A**HRCT-*COOH* | 16 | 30xmore |
| Ac-V42Igl/H9A | *Ac*-ICV**(2-Igl)**QDWG**A**HRCT-*CONH2* | 17 | 39more |
| Ac-V42Igl/H9A-OH | Ac-ICV**(2-Igl)**QDWG**A**HRCT-*COOH* | 18 | 37xmore |
| Ac-V4Dht/H9A -OH | *Ac*-ICV**Dht**QDWG**A**HRCT-*COOH* | 19 | 5xmore |
| Ac-V4(Bpa)/H9A -OH | *Ac*-ICV**(Bpa)**QDW**A**HRCT-*COOH* | 20 | 49xmore |
| Ac-V4(Bpa)/H9A | *Ac*-ICV**(Bpa)**QDWG**A**HCT-*CONH2* | 21 | 86xmore |
| Ac-V4(Bta)/H9A -OH | *Ac*-ICV**(Bta)**QDWG**A**HRCT-*COOH* | 22 | 65xmore |
| Ac-V4(Bta)/H9A | *Ac*-ICV**(Bta)**QDWG**A**HRCT-*CONH2* | 23 | 64xmore |
| Ac-V4W/H9(2-Abu) | *Ac*-ICV**W**QDWG(2-**Abu)**HRCT-*CONH2* | 24 | 64xmore |
| +G/V4W/H9A +AN -OH | *H*-**G**ICV**W**QDWG**A**HRC**TAN**-*COOH* | 25 | 38xmore |
| Ac-V4(5fW)/H9A | *Ac*-ICV**(5fW)**QDWG**A**HRCT- *CONH₂* | 26 | 31xmore |
| Ac-V4(5-MeW)/H9A | *Ac*-ICV**(5-methyl-W)**QDWG**A**HRCT- *CONH₂* | 27 | 67xmore |
| Ac-V4(1-MeW)/H9A | *Ac-*ICV**(1-methyl-W)**QDWG**A**HRCT*-CONH₂* | 28 | 264xmore |
| Ac-V4W/W7(5fW)/H9A | *Ac*-ICV**W**QD**(5fW)**G**A**HRCT-*CONH₂* | 29 | 121xmore |
| Ac-V4(5fW)/W7(5fW)/H9A | *Ac*-ICV**(5fW)**QD**(5fW)**G**A**HRCT- *CONH₂* | 30 | NA |
| Ac-V4(5-MeW)/W7(5fW)H9A | *Ac*-ICV**(5-methyl-W)**QD**(5fW)**G**A**HRCT-*CONH₂* | 31 | NA |
| Ac-V4(1MeW)/W7(5fW)/H9A | *Ac*-ICV**(1-methyl-W)**QD**(5fW)**G**A**HRCT-*CONH₂* | 32 | 264xmore |

| | | | |
|---|---|---|---|
| NA = not available | | | |

In certain embodiments of the invention the compstatin analog has a sequence selected from sequences 10-32. In certain embodiments of the invention the compstatin analog has a sequence selected from SEQ ID NOs: 14, 21, 28, 29, and 32. In certain embodiments of the invention the compstatin analog has a sequence selected from SEQ ID NOs: 30 and 31. In one embodiment of the invention the compstatin analog has a sequence of SEQ ID NO: 28. In one embodiment of the invention the compstatin analog has a sequence of SEQ ID NO: 32.

In other embodiments, compstatin analogs having sequences as set forth in Table 1, but where the Ac- group is replaced by an alternate blocking moiety B¹, as described above, are used. In other embodiments, compstatin analogs having sequences as set forth in Table 1, but where the -NH₂ group is replaced by an alternate blocking moiety B², as described above, are used.

In one embodiment, the compstatin analog binds to substantially the same region of the β chain of human C3 as does compstatin. In one embodiment the compstatin analog is a compound that binds to a fragment of the C-terminal portion of the β chain of human C3 having a molecular weight of about 40 kDa to which compstatin binds (Soulika, A.M., et al., Mol. Immunol., 35:160, 1998; Soulika, A.M., et al., Mol. Immunol. 43(12):2023-9, 2006). In certain embodiments the compstatin analog is a compound that binds to the binding site of compstatin as determined in a compstatin-C3 structure, e.g., a crystal structure or NMR-derived 3D structure. In certain embodiments the compstatin analog is a compound that could substitute for compstatin in a compstatin-C3 structure and would form substantially the same intermolecular contacts with C3 as compstatin. In certain embodiments the compstatin analog is a compound that binds to the binding site of a peptide having a sequence set forth in Table 1, e.g., SEQ ID NO: 14, 21, 28, 29, or 32 in a peptide-C3 structure, e.g., a crystal structure. In certain embodiments the compstatin analog is a compound that binds to the binding site of a peptide having SEQ ID NO: 30 or 31 in a peptide-C3 structure, e.g., a crystal structure. In certain embodiments the compstatin analog is a compound that could substitute for the peptide of SEQ ID NO: 9-32, e.g., SEQ ID NO: 14, 21, 28, or 32 in a peptide-C3 structure and would form substantially the same intermolecular contacts with C3 as the peptide. In certain embodiments the compstatin analog is a compound that could substitute for the peptide of SEQ ID NO: 30 or 31 in a peptide-C3 structure and would form substantially the same intermolecular contacts with C3 as the peptide.

One of ordinary skill in the art will readily be able to determine whether a compstatin analog binds to a fragment of the C-terminal portion of the β chain of C3 using routine experimental methods. For example, one of skill in the art could synthesize a photocrosslinkable version of the compstatin analog by including a photo-crosslinking amino acid such as *p*-benzoyl-L-phenylalanine (Bpa) in the compound, e.g., at the C-terminus of the sequence (Soulika, A.M., et al, *supra*)*.* Optionally additional amino acids, e.g., an epitope tag such as a FLAG tag or an HA tag could be included to facilitate detection of the compound, e.g., by Western blotting. The compstatin analog is incubated with the fragment and crosslinking is initiated. Colocalization of the compstatin analog and the C3 fragment indicates binding. Surface plasmon resonance may also be used to determine whether a compstatin analog binds to the compstatin binding site on C3 or a fragment thereof. One of skill in the art would be able to use molecular modeling software programs to predict whether a compound would form substantially the same intermolecular contacts with C3 as would compstatin or a peptide having the sequence of any of the peptides in Table 1, e.g., SEQ ID NO: 14, 21, 28,29, or 32, or in other embodiments SEQ ID NO: 30 or 31.

Compstatin analogs may be prepared by various synthetic methods of peptide synthesis known in the art *via* condensation of amino acid residues, e.g., in accordance with conventional peptide synthesis methods, may be prepared by expression *in vitro* or in living cells from appropriate nucleic acid sequences encoding them using methods known in the art. For example, peptides may be synthesized using standard solid-phase methodologies as described in Malik, *supra*, Katragadda, *supra*, and/or WO2004026328. Potentially reactive moieties such as amino and carboxyl groups, reactive functional groups, etc., may be protected and subsequently deprotected using various protecting groups and methodologies known in the art. See, e.g., "Protective Groups in Organic Synthesis", 3rd ed. Greene, T. W. and Wuts, P. G., Eds., John Wiley & Sons, New York: 1999. Peptides may be purified using standard approaches such as reversed-phase HPLC. Separation of diasteriomeric peptides, if desired, may be performed using known methods such as reversed-phase HPLC. Preparations may be lyophilized, if desired, and subsequently dissolved in a suitable solvent, e.g., water. The pH of the resulting solution may be adjusted, e.g. to physiological pH, using a base such as NaOH. Peptide preparations may be characterized by mass spectrometry if desired, e.g., to confirm mass and/or disulfide bond formation. See, e.g., Mallik, 2005, and Katragadda, 2006.

The structure of compstatin is known in the art, and NMR structures for a number of compstatin analogs having higher activity than compstatin are also known (Malik, *supra*). Structural information may be used to design compstatin mimetics. In one embodiment, the compstatin mimetic is any compound that competes with compstatin or any compstatin analog (e.g., a compstatin analog whose sequence is set forth in Table 1) for binding to C3 or a fragment thereof (such as a 40 kD fragment of the β chain to which compstatin binds) and that has an activity equal to or greater than that of compstatin. The compstatin mimetic may be a peptide, nucleic acid, or small molecule. In certain embodiments the compstatin mimetic is a compound that binds to the binding site of compstatin as determined in a compstatin-C3 structure, e.g., a crystal structure or a 3-D structure derived from NMR experiments. In certain embodiments the compstatin mimetic is a compound that could substitute for compstatin in a compstatin-C3 structure and would form substantially the same intermolecular contacts with C3 as compstatin. In embodiments the compstatin mimetic is a compound that binds to the binding site of a peptide having a sequence set forth in Table 1, e.g., SEQ ID NO: 14,21,28,29, or 32, or in certain embodiments SEQ ID NO: 30 or 31, in a peptide-C3 structure. In certain embodiments the compstatin mimetic is a compound that could substitute for a peptide having a sequence set forth in Table 1, e.g., SEQ ID NO: 14, 21, 28, 29, or 32, or in certain embodiments SEQ ID NO: 30 or 31, in a peptide-C3 structure and would form substantially the same intermolecular contacts with C3 as the peptide. In certain embodiments the compstatin mimetic has a non-peptide backbone but has side chains arranged in a sequence designed based on the sequence of compstatin.

One of skill in the art will appreciate that once a particular desired conformation of a short peptide has been ascertained, methods for designing a peptide or peptidomimetic to fit that conformation are well known. See, e.g., G.R. Marshall (1993), Tetrahedron, 49: 3547-3558; Hruby and Nikiforovich (1991), in Molecular Conformation and Biological Interactions, P. Balaram & S. Ramasehan, eds., Indian Acad. of Sci., Bangalore, PP. 429-455), Eguchi M, Kahn M., Mini Rev Med Chem., 2(5):447-62, 2002. Of particular relevance to the present invention, the design of peptide analogs may be further refined by considering the contribution of various side chains of amino acid residues, e.g., for the effect of functional groups or for steric considerations as described in the art for compstatin and analogs thereof, among others.

It will be appreciated by those of skill in the art that a peptide mimic may serve equally well as a peptide for the purpose of providing the specific backbone conformation and side chain functionalities required for binding to C3 and inhibiting complement activation. Accordingly, it is contemplated as being within the scope of the present invention to produce and utilize C3-binding, complement-inhibiting compounds through the use of either naturally-occurring amino acids, amino acid derivatives, analogs or non-amino acid molecules capable of being joined to form the appropriate backbone conformation. A non-peptide analog, or an analog comprising peptide and non-peptide components, is sometimes referred to herein as a "peptidomimetic" or "isosteric mimetic," to designate substitutions or derivations of a peptide that possesses much the same backbone conformational features and/or other functionalities, so as to be sufficiently similar to the exemplified peptides to inhibit complement activation. More generally, a compstatin mimetic is any compound that would position pharmacophores similarly to their positioning in compstatin, even if the backbone differs.

The use of peptidomimetics for the development of high-affinity peptide analogs is well known in the art. Assuming rotational constraints similar to those of amino acid residues within a peptide, analogs comprising non-amino acid moieties may be analyzed, and their conformational motifs verified, by means of the Ramachandran plot (Hruby & Nikiforovich 1991), among other known techniques. Virtual screening methods can be used to identify compstatin mimetics that bind to C3. Such methods may comprise use of suitable algorithms to computationally dock, score, and optionally rank a plurality of candidate structures. Any of a wide variety of available software programs can be used to perform the virtual screening method. Exemplary programs useful for flexible molecular docking include DOCK 4.0, FlexX 1.8, AutoDock 3.0, GOLD 1.2, ICM 2.8, and more recent versions thereof.

One of skill in the art will readily be able to establish suitable screening assays to identify additional compstatin mimetics and to select those having desired inhibitory activities. For example, compstatin or an analog thereof could be labeled (e.g., with a radioactive or fluorescent label) and contacted with C3 in the presence of different concentrations of a test compound. The ability of the test compound to diminish binding of the compstatin analog to C3 is evaluated. A test compound that significantly diminishes binding of the compstatin analog to C3 is a candidate compstatin mimetic. For example, a test compound that diminishes steady-state concentration of a compstatin analog-C3 complex, or that diminishes the rate of formation of a compstatin analog-C3 complex by at least 25%, or by at least 50%, is a candidate compstatin mimetic. One of skill in the art will recognize that a number of variations of this screening assay may be employed. Compounds to be screened include natural products, libraries of aptamers, phage display libraries, compound libraries synthesized using combinatorial chemistry, etc. The invention encompasses synthesizing a combinatorial library of compounds based upon the core sequence described above and screening the library to identify compstatin mimetics. Any of these methods could also be used to identify new compstatin analogs having higher inhibitory activity than compstatin analogs tested thus far.

Monoclonal antibodies, that bind to C3 or C3a receptors (C3aR) are of use in the invention. U.S. Pat. No. 5,942,405 discloses C3aR antagonists. Aptamers that bind to and inhibit factor B may be identified using methods such as SELEX (discussed below). U.S. Pat. Pub. No.20030191084 discloses aptamers that bind to C1q, C3 and C5.

### Compounds that Inhibit Factor B Activation or Activity

Antibodies and antibody fragments that bind to factor B are of use in the invention. Exemplary antibodies that inhibit factor B are described in U.S. Pat. Pub. No. 20050260198. In certain embodiments the isolated antibody or antigen-binding fragment selectively binds to factor B within the third short consensus repeat (SCR) domain. In certain embodiments the antibody prevents formation of a C3bBb complex. In certain embodiments the antibody or antigen-binding fragment prevents or inhibits cleavage of factor B by factor D. In certain embodiments the complement inhibitor is an antibody that binds to substantially the same binding site on factor B as an antibody described in U.S. Pat. Pub. No. 20050260198.

### Compounds that Inhibit Factor D Activity

Antibodies and antibody fragments that bind to factor D are of use in the invention. Exemplary antibodies that inhibit factor D are described in U.S. Pat. No. 7,112,327. In certain embodiments the antibody binds to substantially the same binding site on factor D as an antibody described in U.S. Pat. No. 7,112,327. Exemplary polypeptides that inhibit alternative pathway activation and are believed to inhibit factor D are disclosed in U.S. Pub. No. 20040038869.

### Viral complement control proteins (VCCPs) and viral complement inhibiting proteins (VCIP)

Poxviruses and herpesviruses are families of large, complex viruses with a linear doublestranded DNA genome, some of which infect animals and can cause a range of diseases, the most feared of which in humans is smallpox. Certain of these viruses encode a number of immunomodulatory proteins that are believed to play a role in pathogenesis by subverting one or more aspects of the normal immune response and/or fostering development of a more favorable environment in the host organism (Kotwal, GJ, Immunology Today, 21(5), 242-248, 2000). VCCPs are among these proteins. Poxvirus complement control proteins are members of the complement control protein (CCP) superfamily and typically contain 4 SCR modules. These proteins possess features that make them particularly advantageous for treatment and prevention of macular degeneration related conditions and for treatment and prevention of choroidal neovascularization. Agents are described in U.S.S.N. 60/616,983, filed October 8, 2004, in US 2006/0142191, filed Oct. 8, 2005, entitled VIRAL COMPLEMENT CONTROL PROTEINS FOR EYE DISORDERS, and/or in U.S.S.N. 60/751,771, and US 2008/0075755, filed Dec. 19, 2005, and Dec. 19, 2006, respectively, entitled VIRAL COMPLEMENT CONTROL PROTEINS FOR EYE DISORDERS CHARACTERIZED BY INFLAMMATION.

A poxvirus complement control protein (PVCCP) can comprise a sequence encoded by, e.g., vaccinia virus, variola major virus, variola minor virus, cowpox virus, monkeypox virus, ectromelia virus, rabbitpox virus, myxoma virus, Yaba-like disease virus, or swinepox virus. A herpesvirus complement control protein (HVCCP) can comprise a sequence encoded by *Macaca fuscata* rhadinovirus, cercopithecine herpesvirus 17, or human herpes virus 8. The HVCCP may comprise a sequence encoded by herpes simplex virus saimiri ORF 4 or ORF 15 (Albrecht, JC. & Fleckenstein, B., J. Virol., 66, 3937-3940, 1992; Albrecht, J., et al., Virology, 190, 527-530, 1992).

The VCCP may inhibit the classical complement pathway, the alternate complement pathway, the lectin pathway, or any combination of these. The VCCP, e.g., a PVCCP, may bind to C3b, C4b, or both. The PVCCP may comprise one or more putative heparin binding sites (K/R-X-K/R) and/or possess an overall positive charge. Preferably the PVCCP comprises at least 3 SCR modules (e.g., modules 1-3), preferably 4 SCR modules. The PVCCP protein can be a precursor of a mature PVCCP (i.e., can include a signal sequence that is normally cleaved off when the protein is expressed in virus-infected cells) or can be a mature form (i.e., lacking the signal sequence).

Vaccinia complement control protein (VCP) is a virus-encoded protein secreted from vaccinia infected cells. VCP is 244 amino acids in length, contains 4 SCRs, and is naturally produced by intracellular cleavage of a 263 amino acid precursor. VCP runs as an -35 kD protein in a 12% SDS/polyacrylamide gel under reducing conditions and has a predicted molecular mass of about 28.6 kD. VCP is described in U.S. Patent Nos. 5,157,110 and 6,140,472, and in Kotwal, GK, et al., Nature, 355, 176-178, 1988. Figures 3A and 3B show the sequence of the precursor and mature VCP proteins, respectively. VCP has been shown to inhibit the classical pathway of complement activation via its ability to bind to C3 and C4 and act as a cofactor for factor I mediated cleavage of these components as well as promoting decay of existing convertase (Kotwal, GK, et al., Science, 250, 827-830, 1990; McKenzie et al., J. Infect. Dis., 1566, 1245-1250, 1992). It has also been shown to inhibit the alternative pathway by causing cleavage of C3b into iC3b and thereby preventing formation of the alternative pathway C3 convertase (Sahu, A, et al., J. Immunol., 160, 5596-5604, 1998). VCP thus blocks complement activation at multiple steps and reduces levels of the proinflammatory chemotactic factors C3a, C4a, and C5a.

VCP also possesses the ability to strongly bind heparin in addition to heparan sulfate proteoglycans. VCP contains two putative heparin binding sites located in modules 1 and 4 (Jha, P and Kotwal, GJ, and references therein). VCP is able to bind to the surface of endothelial cells, possibly via interaction with heparin and/or heparan sulfate at the cell surface, resulting in decreased antibody binding (Smith, SA, et al., J. Virol., 74(12), 5659-5666,2000). VCP can be taken up by mast cells and possibly persist in tissue for lengthy periods of time, thereby potentially prolonging its activity (Kotwal, GJ, et al., In GP. Talwat, et al. (eds), 10th International Congress of Immunology., Monduzzi Editore, Bologna, Italy, 1998). In addition, VCP can reduce chemotactic migration of leukocytes by blocking chemokine binding (Reynolds, D, et al., in S. Jameel and L. Villareal (ed., Advances in animal virology. Oxford and IBN Publishing, New Delhi, India, 1999).

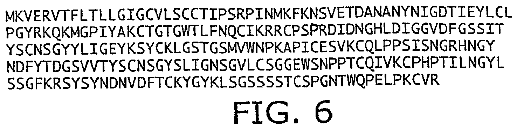

Variola virus major and minor encode proteins that are highly homologous to VCP and are referred to as smallpox inhibitor of complement enzymes (SPICE) (Rosengard, AM, et al., Proc. Natl. Acad. Sci., 99(13), 8803-8813. U.S. Pat. No. 6,551,595). SPICE from various variola strains sequenced to date differs from VCP by about 5% (e.g., about 11 amino acid differences). Similarly to VCP, SPICE binds to C3b and C4b and causes their degradation, acting as a cofactor for factor I. However, SPICE degrades C3b approximately 100 times as fast as VCP and degrades C4b approximately 6 times as fast as VCP. The amino acid sequence of SPICE is presented in Figure 6 and can be described as follows. Referring to Figure 6, a signal sequence extends from amino acid 1 to about amino acid 19. Four SCRs extend from about amino acid 20 to amino acid 263. Each SCR is characterized by four cysteine residues. The four cysteine residues form two disulfide bonds in the expressed protein. The boundaries of each SCR are best defined by the first and fourth cysteine residues in the sequence that forms the disulfide bonds of the SCR. An invariant tryptophan residue is present between cysteine 3 and cysteine 4 of each SCR. SCR1 extends from amino acid 20 or 21 to amino acid 81. Both residues are cysteines that may be involved in disulfide bonding. SCR2 extends from amino acid 86 to amino acid 143. SCR3 extends from amino acid 148 to amino acid 201. SCR4 extends from amino acid 206 to amino acid 261. The SCRs include the complement binding locations of SPICE. SPICE or any of the portions thereof that inhibit complement activation, e.g., SPICE and SPICE-related polypeptides containing four SCRs, such as those described in U.S. Pat. No. 6,551,595, are of use in the present invention.

Complement control proteins from cowpox virus (referred to as inflammation modulatory protein, IMP) and monkeypox virus (referred to herein as monkeypox virus complement control protein, MCP) have also been identified and sequenced (Miller, CG, et al., Virology, 229, 126-133, 1997 and Uvarova, EA and Shchelkunov, SN, Virus Res., 81(1-2), 39-45,2001). MCP differs from the other PVCCPs described herein in that it contains a truncation of the C-terminal portion of the fourth SCR.

It will be appreciated that the exact sequence of complement control proteins identified in different virus isolates may differ slightly.

Complement control proteins from any such isolate may be used, provided that the protein has not undergone a mutation that substantially abolishes its activity. Thus the sequence of a VCCP such as SPICE or VCP may differ from the exact sequences presented herein or under the accession numbers listed in Table 1. It will also be appreciated that a number of amino acid alterations, e.g., additions, deletions, or substitutions such as conservative amino acid substitutions, may be made in a typical polypeptide such as a VCCP without significantly affecting its activity, such that the resulting protein is considered equivalent to the original polypeptide. For example, up to about 10% of the amino acids, or up to about 20% of the amino acids may frequently be changed without significantly altering the activity. Also, of course, domains known to have similar functions can be substituted for one another. Such domains may be found within a single polypeptide (e.g., repeated domains) or within different, homologous polypeptides. The effect of any particular amino acid alteration(s) or domain substitutions can readily be determined.

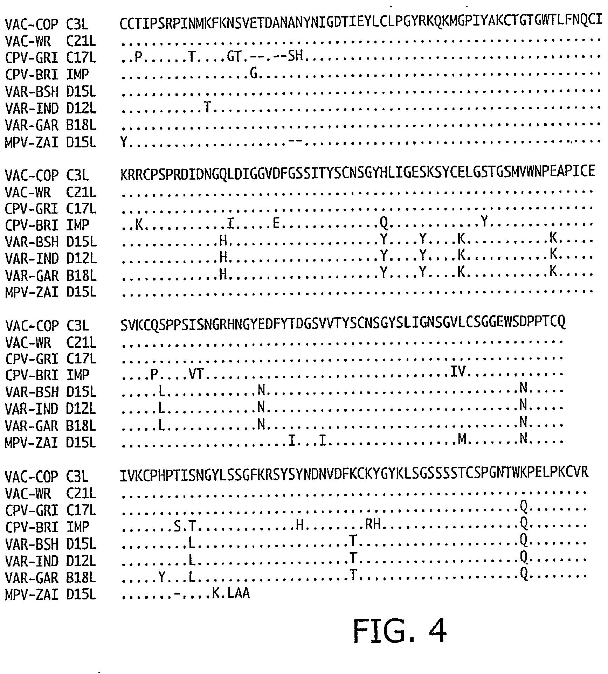

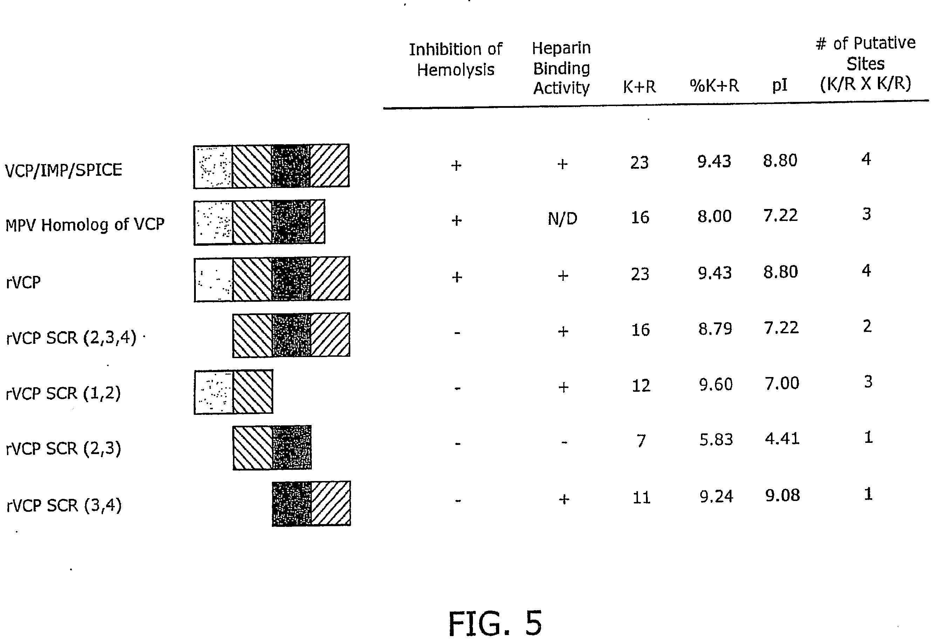

Figure 4 shows a sequence alignment of a variety of poxvirus complement control proteins from isolates of variola major and minor, vaccinia, cowpox virus, and monkeypox virus. Figure 5 shows a comparison of the SCR domain structure of a number of complement control proteins and fragments thereof, the number of K+R residues, %K+R residues, pI, number of putative heparin binding sites, and ability to inhibit hemolysis (indicative of complement inhibiting activity) and/or bind to heparin.

Without limitation, any of the viral polypeptides identified by accession number in Table 2 below is of use in various embodiments of the invention.

**Table 2: Representative Viral Complement Control Proteins**

| **Virus** | **Protein** | **Accession** | **virus Type** |
|---|---|---|---|
| Variola | D12L | NP_042056 | Orthopoxvirus |
| | D15L (SPICE) | AAA69423 | Orthopoxvirus |
| Vaccinia | VCP | AAO89304 | Orthopoxvirus |
| Cowpox | CPXV034 | AAM13481 | Orthopoxvirus |
| | C17L | CAA64102 | Orthopoxvirus |
| Monkeypox | D14L | AAV84857 | Orthopoxvirus |
| Ectromella virus | Complement control protein | CAE00484 | Orthopoxvirus |
| Rabbitpox | RPXV017 | AAS49730 | Orthopoxvirus |
| Macaca fuscata rhadinovirus | JM4 | AAS99981 | Rhadinavirus (Herpesvirus) |
| Cercopithecine herpesvirus 17 | Complement binding protein (ORF4) | NP_570746 | Herpesvirus |
| Human herpes virus 8 | Complement binding protein (ORF4) | AAB62602 | Herpesvirus |

### Compounds that Inhibit C5 Activation or Activity

Antibodies and antibody fragments that bind to C5 are of use in the invention. Exemplary antibodies are described in U.S. Pat. No. 6,534,058. Compounds that bind to and inhibit C5 are described in U.S. Pat. Pub. Nos. 20050090448 and 20060115476. In certain embodiments the antibody binds to substantially the same binding site on C5 as an antibody described in U.S. Pat. No. 6,534,058 or a peptide described in USSN 10/937,912. U.S. Pat. Pub. No. 20060105980 discloses aptamers that bind to and inhibit C5.

Exemplary C5a receptor antagonists include a variety of small cyclic peptides such as those described in U.S. Pat. No. 6,821,950; US 2009/0117171; and/or PCT/US06/08960 (WO2006/099330).

For example, the compound may be of general formula I below: 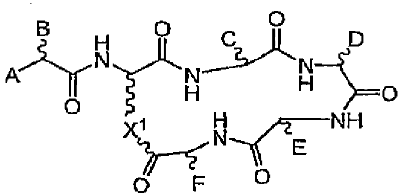 where A is H, alkyl, aryl, NH₂, NHalkyl, N(alkyl)₂, NHaryl or NHacyl; B is an alkyl, aryl, phenyl, benzyl, naphthyl or indole group, or the side chain of a D- or L-amino acid selected from the group consisting of phenylalanine, homophenylalanine, tryptophan, homotryptophan, tyrosine, and homotyrosine; C is the side chain of a D-, L- or homo-amino acid selected from the group consisting of proline, alanine, leucine, valine, isoleucine, arginine, histidine, aspartate, glutamate, glutamine, asparagine, lysine, tyrosine, phenylalanine, cyclohexylalanine, norleucine, tryptophan, cysteine and methionine; D is the side chain of a D- or L-amino acid selected from the group consisting of cyclohexylalanine, homocyclohexylalanine, leucine, norleucine, homoleucine, homonorleucine and tryptophan; E is the side chain of a D- or L-amino acid selected from the group consisting of tryptophan and homotryptophan; F is the side chain of a D- or L-amino acid selected from the group consisting of arginine, homoarginine, lysine and homolysine or is one of the following side-chains 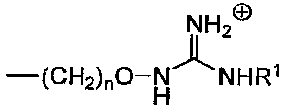 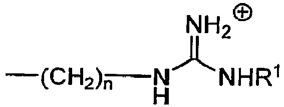 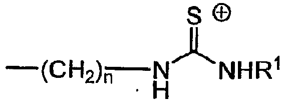 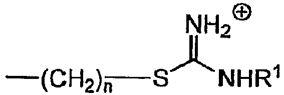 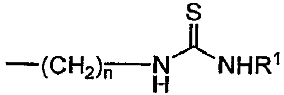 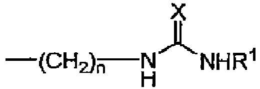 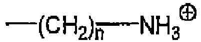 or another mimetic of an arginine side chain, where X is NCN, NNO₂, CHNO₂ or NSO₂NH₂; n is an integer from 1 to 4, and R¹ is H or an alkyl, aryl, CN, NH₂, OH, --CO-CH₂CH₃, --CO--CH₃, --CO--CH₂CH₂CH₃, --CO--CH₂ Ph, or --CO-Ph; and X¹ is --(CH₂)ₙNH-or (CH₂)ₙ -S--, --(CH₂)₂ O--, --(CH₂)₃ O--, --(CH₂)₃ -, --(CH₂)₄ --, or --CH₂ COCHRNH-, where R is the side chain of any common or uncommon amino acid, and where n is an integer of from 1 to 4, e.g., 1, 2, 3, or 4.

In certain compounds, F is one of the following side-chains: 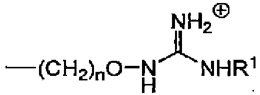 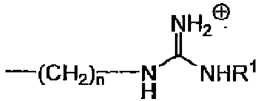 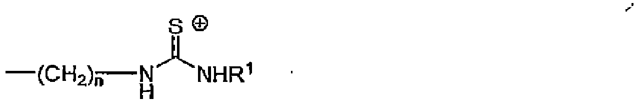 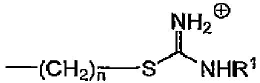 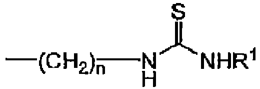 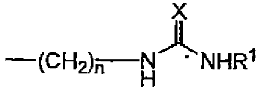 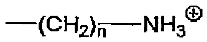 or another mimetic of an arginine side chain; where X is NCN, NNO₂, CHNO₂ or NSO₂NH₂; n is an integer from 1 to 4, and R¹ is H or an alkyl, aryl, CN, NH₂, OH, --CO-CH₂CH₃, --CO-CH₃, --CO--CH₂CH₂CH₃, -CO--CH₂ Ph, or --CO-Ph; B is an indole, indole methyl, benzyl, phenyl, naphthyl, naphthyl methyl, cinnamyl group, or any other derivative of the aromatic group; and C is D- or L-cyclohexcylalanine (Cha), leucine, valine, isoleucine, phenylalanine, tryptophan or methionine. In certain compounds, A is L-arginine. In certain compounds, F is an L-amino acid. In certain compounds, F is L-arginine. In certain compounds, n = 1, 2, 3, or 4.

The compound may be selected from the group consisting of SEQ ID NOs: 11, 12, 13, 14, 15, 16, 17, 18, 19, 20, 21, 22, 23, 24, 25, 26, 27 and 28, as described in U.S. Pat. No. 6,821,950. Other compounds disclosed therein may also be used. For example, A, B, C, D, E, F, and R¹ may be any of the groups mentioned in U.S. Pat. No. 6,821,950. It is noted that the letters A, B, C, D, E, and F in the formulas presented herein are to be given the meanings described herein and in U.S. Pat. No. 6,821,950 and do not stand for chemical elements or isotopes such as boron, carbon, deuterium, or fluorine. The compounds described above will be referred to collectively herein as GPCRA.

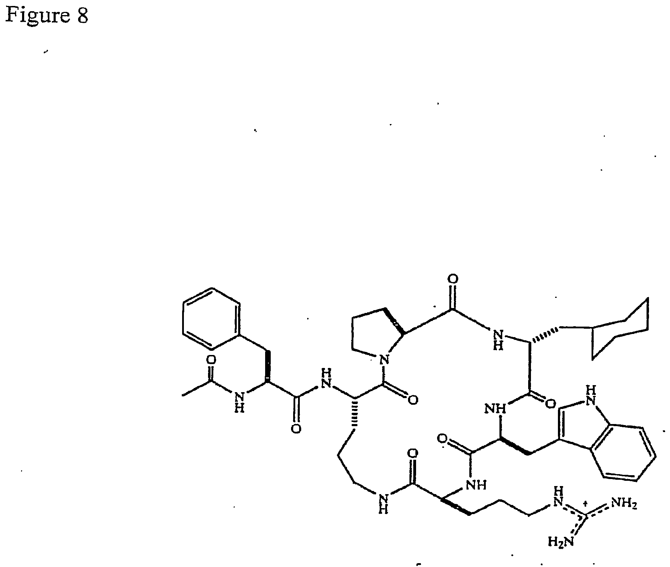

A complement inhibitor may be a C5a receptor inhibitor, e.g., a C5a antagonist. For example, the complement inhibitor may be a peptide having the following sequence: HC-[ORN-PRO-dCHA-TRP-ARG] (SEQ ID NO: 45) where HC = hydrocinnamate, dCHA = d-cyclohexylalaine, ORN = 1-ornithine, and [ ] denotates cyclization through an amide bound. The complement inhibitor may be a peptide having sequence Ac-PHE-[ORN-PRO-dCHA-TRP-ARG] (SEQ ID NO: 46), using the same abbreviations. The complement inhibitor may be the compound depicted in Figure 8 or a C3a receptor inhibitor, e.g., a C3a antagonist.

Methods for making the GPCRA, confirming their structure, and testing their activity as modulators of a GPCR are disclosed in U.S. Pat. No. 6,821,950. Certain of these compounds are available from Promics (Brisbane, Australia). One complement inhibitor is PMX205.

### C. Long-acting Therapeutic Agents

In certain embodiments of the invention at least one of the therapeutic agents is a long-acting agent For example, certain complement inhibitors may intrinsically have a long duration of activity even if not provided as a component of a sustained release formulation. The long-acting therapeutic agent may, for example, have an activity period of at least 3 months, at least 6 months, at least 9 months, or at least 12 months when administered in solution in a liquid medium in medically acceptable quantities. The long-acting therapeutic agent may be administered in solution in a liquid medium or may be a component of a solid or semi-solid formulation which optionally contains one or more additional therapeutically active or inactive components.

In other embodiments a therapeutic agent that is not a long-acting agent is modified such that it becomes long-acting. The modification may, for example, stabilize the agent against the activity of various endogenous molecules such as proteases. Suitable modifications are known in the art and include, for example, pegylation.

In certain embodiments of the invention the long-acting therapeutic agent is administered as a component of a sustained release formulation, e.g., an ocular implant or any sustained release formulation described herein.

### III. Liquid Compositions Comprising a Therapeutic Agent

In certain embodiments of the invention at least one of the therapeutic agents, e.g., any of the therapeutic agents useful in the invention discussed above, is administered in solution in a liquid medium. Suitable preparations, e.g., substantially pure preparations of one or more therapeutic agents may be combined with pharmaceutically acceptable carriers, diluents, solvents, etc., to produce an appropriate pharmaceutical composition, i.e., one that is pharmaceutically acceptable for administration to the eye. The preparation may contain a pharmaceutically acceptable carrier, diluent, etc. Suitable carriers are known in the art and include, for example, sterile water for injection, saline, etc. Additional components may include, but are not limited to, buffers, preservatives, salts, etc.

The therapeutic agents themselves may be provided as pharmaceutically acceptable salts, which include those derived from pharmaceutically acceptable inorganic and organic acids and bases. Examples of suitable acid salts include acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, formate, fumarate, glucoheptanoate, glycerophosphate, glycolate, hemisulfate, heptanoate, hexanoate, hydrochloride, hydrobromide, hydroiodide, 2-hydroxyethanesulfonate, lactate, maleate, malonate, methanesulfonate, 2-naphthalenesulfonate, nicotinate, nitrate, oxalate, palmoate, pectinate, persulfate, 3-phenylpropionate, phosphate, picrate, pivalate, propionate, salicylate, succinate, sulfate, tartrate, thiocyanate, tosylate and undecanoate. Salts derived from appropriate bases include alkali metal (e.g., sodium and potassium), alkaline earth metal (e.g., magnesium), ammonium and N+(C1-4 alkyl)4 salts. This invention also envisions the quaternization of any basic nitrogen-containing groups of the compounds disclosed herein. Water or oil-soluble or dispersible products may be obtained by such quaternization.

Solutions or suspensions can include components such as a sterile diluent such as water for injection, saline solution, or other solvent acceptable for administration to the eye, buffers such as acetates, citrates or phosphates and agents for the adjustment of tonicity such as sodium chloride or dextrose. pH can be adjusted with acids or bases, such as hydrochloric acid or sodium hydroxide. The preparation can be enclosed in ampoules, disposable syringes or single or multiple dose vials made of glass or plastic and provided for commercial sale and/or use in any such manner. The term "suspension" includes a composition comprising particles in a liquid medium. In some embodiments, the particles consist essentially of a therapeutic agent. In other embodiments the particles comprise a drug-releasing component such as a polymer and, optionally, one or more additional components such as an excipient.

In some embodiments of the invention the liquid composition comprises an agent that enhances uptake of the therapeutic agent by cells, enhances bioavailability of the agent at its site of action, or otherwise enhances activity of the therapeutic agent. For example, a variety of delivery vehicles that enhance uptake and/or activity of RNAi agents such as siRNAs are known in the art and may be included in the liquid composition.

Preferred pharmaceutical formulations are stable under the conditions of manufacture and storage and may be preserved against the contaminating action of microorganisms such as bacteria and fungi.

### IV. Sustained Release Formulations

A sustained release formulation of use in the present invention provides a therapeutic concentration of a drug within the eye or a portion or region thereof for a prolonged period of time. The period of time during which a therapeutic level of the drug is present can be, e.g., at least 1, 2, 4, or 6 weeks, at least 1, 2, 3, 4, 6, 8, 10, 12, 15, 18, 24 months, or longer. Release may begin immediately or shortly (e.g., within 24 hours) after administration of the sustained delivery formulation. Alternately, release may be delayed, e.g., it may commence at a time point at least 24 hours following administration. Without limitation, release may occur steadily or may occur intermittently (e.g., in bursts during which a substantial amount of the agent is released), or periods of steady release may alternate with bursts. In certain embodiments the therapeutic agent is released at controlled or predetermined rates when the sustained release formulation is placed in the eye. Such rates may range, for example, from about 0.003 micrograms/day to about 5000 micrograms/day, or between about 01 micrograms/day to about 5 micrograms/day, or between about .05 micrograms to about 1 microgram/day. In some embodiments the rate of release is between 1 µg and 5 µg/day.

A sustained release formulation of use in the present invention typically comprises a therapeutic agent and an additional component, element, or structure that contributes to the sustained release properties of the formulation. The additional component, element, or structure that is effective to provide sustained release is referred to herein as a "drug delivery regulating component". Optionally the drug delivery regulating element is designed to provide control over the kinetics of release. It will be appreciated that the physical nature of the formulation, e.g., the shape and total surface area of any solid or semi-solid constituents, may contribute to its sustained release properties. As another example, tight compression of particles containing an active agent may result in release that takes place over a longer time period than if the particles were not compressed. In some embodiments the structure is provided at least in part by the therapeutic agent itself and, optionally, one or more substances present at the site of administration such as an ion, protein, etc. In some embodiments no additional drug delivery regulating component need be present in the administered composition. For example, a composition comprising a therapeutic agent in a liquid medium may form a structure having properties of a gel following its administration. The therapeutic agent may be released over time, optionally as the structure degrades. The drug delivery regulating component may comprise or consist of a polymer matrix that is physically associated with the therapeutic agent. For example, the therapeutic agent may be entrapped, embedded, or encapsulated by the polymer matrix. A sustained release formulation can be in the form of an individual ocular implant, a plurality of nanoparticles, microparticles, or liposomes, a semi-solid or viscous material such as a gel, etc. The therapeutic agent may preferably be from about 1 % to 90% by weight of the sustained release formulation. More preferably, the therapeutic agent is from about 20% to about 80% by weight of the of the sustained release formulation. In certain embodiments, the therapeutic agent comprises about 40% by weight of the sustained release formulation (e.g., 30%-50%).

A number of polymeric delivery vehicles for providing sustained release have been used in an ocular context and can be used to administer the compositions of the invention. Various polymers, e.g., biocompatible polymers, which may be biodegradable, can be used. The polymers may be homopolymers, copolymers (including block copolymers), straight, branched-chain, or cross-linked. Useful polymers include, but are not limited to, poly-lactic acid (PLA), poly-glycolic acid (PGA), poly-lactide-co-glycolide (PLGA), poly(phosphazine), poly (phosphate ester), polycaprolactones, polyanhydrides, ethylene vinyl acetate, polyorthoesters, polyethers, and poly (beta amino esters). Peptides, proteins such as collagen or albumin, polysaccharides such as chitosan, alginate, hyaluronic acid (or derivatives of any of these) and dendrimers (e.g., PAMAM dendrimers) are also of use. Methods for preparation of such formulations will be apparent to those skilled in the art. Certain of the materials can also be obtained commercially, e.g., from Alza Corporation Any of these polymers, or combinations thereof, can be used in various embodiments of the invention.

Additional exemplary polymers include cellulose derivatives such as carboxymethylcellulose, polycarbamates or polyureas, cross-linked poly(vinyl acetate) and the like, ethylene-vinyl ester copolymers having an ester content of 4 to 80% such as ethylene-vinyl acetate (EVA) copolymer, ethylene-vinyl hexanoate copolymer, ethylene-vinyl propionate copolymer, ethylene-vinyl butyrate copolymer, ethylene-vinyl pentantoate copolymer, ethylene-vinyl trimethyl acetate copolymer, ethylene-vinyl diethyl acetate copolymer, ethylene-vinyl 3-methyl butanoate copolymer, ethylene-vinyl 3-3-dimethyl butanoate copolymer, and ethylene-vinyl benzoate copolymer, or mixtures thereof.

Poly(ortho esters) have been introduced into the eye and demonstrated favorable properties for sustained release ocular drug delivery (Einmahl, S., Invest. Ophthalmol. Vis. Sci., 43(5), 2002). Polylactide particles have been used to target an agent to the retina and RPE following intravitreous injection of a suspension of such particles (Bourges, J-L, et al, Invest. Ophthalmol. Vis. Sci., 44(8), 2003).

Sustained release formulations including various ocular implants and other ocular drug delivery systems that are of use in various embodiments of the invention are described, for example, in U.S. Patent Nos. 6,692,759; 6,331,313; 5,869,079; 5,824,072; and U.S.S.N. 10/918,597 (Pub. No. 20050048099); 10/837,357 (Pub. No. 20050244469); 11/092,122 (Pub. No. 20050244472) and 11/116,698 (Pub. No. 20050281861) as well as a number of other patents and publications referenced in the foregoing.

A method of making a sustained release formulation involves combining or mixing the therapeutic agent with a polymeric component to form a mixture. The mixture may then be extruded, compressed, molded, etc., to form a single composition. Optionally, heat and/or pressure can be used. The single composition may then be processed to form individual implants or particles suitable for placement in an eye of a patient. Additional methods for incorporating therapeutically active agents into polymeric matrices are known in the art. The polymeric matrix can be formed into various shapes such as rods, disks, wafers, etc., which may have a range of different dimensions (e.g., length, width, etc.) and volumes. Exemplary shapes include spherical, cylindrical, helical, coil-shaped or helical, screw-shaped, cubical, conical, ellipsoidical, biconvex, hemispherical or near-hemispherical etc.

In certain embodiments of the invention an ocular implant is so dimensioned and shaped that it fits within the hollow shaft of an injection needle, e.g., a 22, 25, 27, 30, 33, or 35 gauge needle (or needle of any gauge ranging between 22 and 35). Exemplary and nonlimiting dimensions for a cylindrical implant may be about 0.5 to 8 millimeters in length and about 0.1 to 2 millimeters in diameter, e.g., about 0.75 mm to about 1.5 mm in diameter. Implants having other shapes, e.g., other rodlike structures with cross-sections that are rectangular or square in cross-section may have a cross-section in which the two points most distant from each other are separated by at most 0.1 mm to 1 mm. In particular embodiments the intraocular implant may have a length or other longest dimension of between about 5 microns and about 2 mm, or between about 10 microns and about 1 mm for administration with a needle. Alternately, the length or other longest dimension is greater than 1 mm, or greater than 2 mm, such as 3 mm or up to 10 mm. The vitreous chamber in humans is able to accommodate relatively large implants of varying geometries, having lengths of, for example, 1 to 10 mm.

In certain embodiments of the invention the implants may also be at least somewhat flexible, which may facilitate both insertion of the implant in the eye, e.g., in the vitreous, and/or may facilitate accommodation of the implant. The total weight of the implant may be about 250-5000 micrograms, e.g., about 500-1000 micrograms. For example, an implant may be about 500 micrograms or about 1000 micrograms. Larger implants may also be formed and further processed before administration to an eye. In addition, larger implants may be desirable where relatively greater amounts of a therapeutic agent are provided in the implant, as used.

In one embodiment the sustained release formulation is a biocompatible ocular implant comprising a substantially impermeable polymeric outer layer covering a core which comprises the drug to be delivered, wherein said outer layer has one or more orifices, by which is meant one or more openings in the outer layer through which, when the device is in use, body fluids can enter the device and the drug contained in the device (e.g., dissolved, encapsulated, or entrapped within the device) can migrate out of the device. In certain embodiments the orifices in total have a surface area of less than 10 percent of the total surface area of the device. In certain embodiments of the invention the ocular implant comprises an outer coating layer that is permeable to the therapeutic agent, allowing its slow diffusion out of the implant. The composition, structure, and/ or thickness of the coating layer may be selected to provide a particular permeability and diffusion rate.

A drug can be contained in an ocular implant as a dry powder, particles, granules, or as a compressed solid. The drug may also be present as a solution or be dispersed in a polymer matrix. Ocular implants, may be have the active agent or agents homogenously distributed through the polymeric matrix, e.g., they may be monolithic. In other embodiments the active agent(s) are heterogeneously distributed in the polymeric matrix. For example, discrete regions of the implant may contain solid particles of an active agent, or a reservoir of active agent may be encapsulated by the polymeric matrix. The therapeutic agent(s) may be distributed in a non-homogenous pattern in the matrix. For example, an implant may include a portion that has a greater concentration of the therapeutic agent relative to a second portion of the implant. Multilayered structures, with the layers having different compositions and may have different physical characteristics such as density or porosity are another possibility. For example, the layers may contain different therapeutic agents or combinations thereof. In another embodiment, layers that are relatively resistant to degradation are interspersed with layers that degrade more rapidly.

The biodegradable polymeric materials which are included to form the matrix may be subject to enzymatic or hydrolytic instability. Water soluble polymers may be cross-linked with hydrolytic or biodegradable unstable cross-links to provide useful water insoluble polymers. The degree of stability can vary widely, depending, for example, upon the choice of monomer, whether a homopolymer or copolymer or mixture, is employed, and whether the polymer includes terminal acid groups. The biodegradation of the polymer and hence the extended release profile of the sustained release formulation may also influenced by the relative average molecular weight of the polymeric materials employed. Different molecular weights of the same or different polymeric materials may be included in the formulations to modulate the release profile. For example, the average molecular weight of the polymer may range from about 5 to about 500 kD, e.g., from about 10 to 100 kD, or from about 15 to 50 kD.

Nanoparticles or microparticles can be made using any method known in the art including, but not limited to, spray drying, phase separation, single and double emulsion, solvent evaporation, solvent extraction, and simple and complex coacervation. Particulate polymeric compositions can also be made using granulation, extrusion, and/or spheronization. A composition can contain nanoparticles or microparticles having different compositions and/or properties.

The conditions used in preparing the particles may be altered to yield particles of a desired size or property (e.g., hydrophobicity, hydrophilicity, external morphology, "stickiness", shape, etc.). The method of preparing the particle and the conditions (e.g., solvent, temperature, concentration, air flow rate, etc.) used may also depend on the therapeutic agent and/or the composition of the polymer matrix.

Microparticles and nanoparticles of use in the invention can have a range of dimensions. Generally, a microparticle will have a diameter of 500 microns or less, e.g., between 1 and 500 microns, between 50 and 500 microns, between 100 and 250 microns, between 20 and 50 microns, between 1 and 20 microns, between 1 and 10 microns, etc., and a nanoparticle will have a diameter of less than 1 micron, e.g., between 10 nm and 100 nm, between 100 nm and 250 nm, between 100 nm and 500 nm, between 250 nm and 500 nm, between 250 nm and 750 nm, between 500 nm and 750 micron. If the particles prepared by any of the above methods have a size range outside of the desired range, the particles can be sized, for example, using a sieve. Particles can be substantially uniform in size (e.g., diameter) or shape or may be heterogeneous in size and/or shape. They may be substantially spherical or may have other shapes, in which case the relevant dimension will be the longest straight dimension rather than the diameter.

In certain embodiments of the invention a sustained release formulation comprises a therapeutic agent and a gel-forming material. In accordance with certain embodiments of the invention, a solution containing the soluble gel-forming material and a therapeutic agent is prepared by combining the soluble gel-forming material and therapeutic agent in solution using any suitable method, e.g., by adding the therapeutic agent to a solution containing the gel-forming material. The composition is delivered locally to an appropriate location in the eye of a subject. The solution rapidly forms a gel at or close to of the site of administration. The therapeutic agent is entrapped within the gel. The therapeutic agent diffuses out of the gel or is released as the gel degrades overtime, thereby providing a continuous supply of the agent to tissues and structures that are either in direct physical contact with the gel or located nearby. In certain embodiments the solution is administered behind the sclera of the eye. Delivery can be accomplished by injection (e.g., using a 25, 27, or 30 gauge needle or the like), by catheter, etc. In other embodiments the solution is administered intravitreally. In certain embodiments a "gel" is a structure that exhibits properties (e.g., fluidity) intermediate between solid and liquid phases. The structure may be a solid or semisolid colloid comprising a solid continuous phase and a liquid phase. The structure may have an appearance typical of a gel, which appearance is readily recognized by those of skill in the art.

In one embodiment, soluble collagen is used as the gel-forming material. The collagen is initially soluble, e.g., in an aqueous medium, and forms a solution that has a low viscosity but is capable of rapid formation of a gel under appropriate conditions, e.g., conditions encountered upon administration to a mammalian subject. A variety of different collagen preparations can be used in the present invention provided that the collagen is initially soluble and is capable of rapidly forming a gel under appropriate conditions. Suitable collagen preparations, and methods for their manufacture, are described, e.g., in U.S. Pat. Nos. 5,492,135; 5,861,486; 6,197,934; 6,204,365; and WO 00/47130, but the invention is not limited to such preparations or methods. These collagens are prepared in soluble form and rapidly form a gel upon exposure to physiological fluids or other fluids having suitable concentration of ions. In accordance with the present invention, injecting or otherwise introducing the collagen solution to the eye or near the eye results in gel formation, presumably induced by contact with physiological fluids. However it is noted that the invention is in no way limited by the mechanism by which gel formation occurs. In addition, as noted above, the gel can be formed *in vitro* and then implanted at an appropriate location.

Other gel-forming materials of use in the invention include, but are not limited to, hyaluronic acid and modified forms thereof, polysaccharides such as alginate and modified forms thereof, self-assembling peptides, etc. See, e.g., U.S. Pat. Nos. 6,129,761 for further description of alginate and modified forms thereof, hyaluronic acid and modified forms thereof, and additional examples of soluble gel-forming materials that are of use in various embodiments of the present invention. Other polymeric hydrogel precursors include polyethylene oxide-polypropylene glycol block copolymers such as Pluronics ™ or Tetronics™ which are crosslinked by hydrogen bonding and/or by a temperature change, as described in Steinleitner et al., Obstetrics & Gynecology, 77:48-52 (1991); and Steinleitner et al., Fertility and Sterility, 57:305-308 (1992). Other materials which may be utilized include proteins such as fibrin or gelatin. Polymer mixtures also may be utilized. For example, a mixture of polyethylene oxide and polyacrylic acid which gels by hydrogen bonding upon mixing may be utilized.

Typically a gel-forming material of use in the invention is capable of being at least partly dissolved, or in certain embodiments of the invention substantially or fully dissolved, e.g., in an aqueous medium. For example, at least 50%, at least 60%, at least 70%, at least 80%, at least 90%, at least 95%, or more, by weight, of the gel-forming material present in a gel-forming composition may be dissolved. In certain embodiments essentially 100% of the material is dissolved. The aqueous medium can contain one or more liquids in addition to water, e.g., various alcohols. In general, at least 10%, 20%, 30%, 40%, 50%, 60%, 70%, 80%, 90%, 95%, or 100% of the liquid present in the medium is water.

Covalently crosslinkable hydrogel precursors also are useful. For example, a water soluble polyamine, such as chitosan, can be cross-linked with a water soluble diisothiocyanate, such as polyethylene glycol diisothiocyanate. The isothiocyanates will react with the amines to form a chemically crosslinked gel. Aldehyde reactions with amines, e.g., with polyethylene glycol dialdehyde also may be utilized. A hydroxylated water soluble polymer also may be utilized.

In certain embodiments of the invention a therapeutic agent is covalently or noncovalently attached to a drug delivery regulating component such as a polymer via a linking moiety. The linking moiety is cleaved to release the therapeutic agent from the drug delivery regulating component to provide sustained release. For example, the linking moiety may be a peptide containing a site that is cleaved by an endogenous enzyme such as a protease or may contain a labile or hydrolyzable bond, e.g., a disulfide bond, ester moiety, etc.

Cells that express a therapeutic agent that is a biological macromolecule such as a protein or RNAi agent can be implanted into the eye and are of use in certain embodiments of the invention to provide sustained release. U.S. Patent No. 6,436,427 provides a method for delivering biologically active molecules to the eye by implanting biocompatible capsules containing a cellular source of the biologically active molecule.

In certain embodiments of the invention the sustained release formulation comprises liposomes. For example, a liposomal suspension can be administered. Liposomes can be prepared according to methods known to those skilled in the art, for example, as described in U.S. Patent No. 4,522,811 and other references listed herein. Liposomes, including targeted liposomes (e.g., antibody targeted liposomes) and pegylated liposomes have been described (Hansen CB, et al., Biochim Biophys Acta. 1239(2):133-44,1995; Torchilin VP, et al., Biochim Biophys Acta, 1511(2):397-411, 2001; Ishida T, et al., FEBS Lett. 460(1):129-33, 1999).

One of ordinary skill in the art will appreciate that the materials and methods selected for preparation of a sustained release formulation, implant, etc., should be such as to retain activity of the compound. For example, it may be desirable to avoid excessive heating of certain agents such as polypeptides, which could lead to denaturation and loss of activity. Furthermore, it will be appreciated that a sustained release formulation may contain a variety of additional components that lack therapeutic activity and that may or may not contribute to the sustained release features of the formulation. Examples include plasticizing agents, solubilizing agents, solubility decreasing agents, and dispersing agents (see U.S. Pat. No. 6,331,313), provided that such components are compatible with administration to the eye under the conditions used. For example, a sustained release formulation may include a β-cyclodextrin, which is effective in enhancing the solubility of the therapeutic agent. The β-cyclodextrin may be provided in an amount from about 0.5% (w/w) to about 25% (w/w) of the implant. In certain implants, the β-cyclodextrin is provided in an amount from about 5% (w/w) to about 15% (w/w) of the formulation. Other formulations include a gamma-cyclodextrin, and/or cyclodextrin derivatives.

A sustained release formulation herein may include an excipient component, such as effective amounts of buffering agents, preservatives and the like. Suitable water soluble buffering agents include, without limitation, alkali and alkaline earth carbonates, phosphates, bicarbonates, citrates, borates, acetates, succinates and the like, such as sodium phosphate, citrate, borate, acetate, bicarbonate, carbonate and the like. These agents are advantageously present in amounts sufficient to maintain a pH of the system of between about 2 to about 9 and more preferably about 4 to about 8. As such the buffering agent may be as much as about 5% by weight of the total system. Suitable water soluble preservatives include sodium bisulfite, sodium bisulfate, sodium thiosulfate, ascorbate, benzalkonium chloride, chlorobutanol, thimerosal, phenylmercuric acetate, phenylmercuric borate, phenylmercuric nitrate, parabens, methylparaben, polyvinyl alcohol, benzyl alcohol, phenylethanol and the like and mixtures thereof. These agents may be present in amounts of from 0.001 to about 5% by weight and preferably 0.01 to about 2% by weight. These agents may also be used in certain of the liquid compositions described herein.

In some embodiments of the invention the sustained release formulation comprises an agent that enhances uptake of the therapeutic agent by cells, enhances bioavailability of the agent at its site of action, or otherwise enhances activity of the therapeutic agent. For example, a variety of delivery vehicles that enhance uptake and/or activity of RNAi agents such as siRNAs are known in the art and may be included in the sustained release formulation.

If desired, the proportions of therapeutic agent, polymer, and any other modifiers may be empirically determined by formulating several implants, for example, with varying proportions of such ingredients. A USP approved method for dissolution or release test can be used to measure the rate of release (USP 23; NF 18 (1995) pp. 1790-1798). The implants can also be tested *in vivo.*

Included within the scope of the term "sustained release formulation" of a therapeutic agent are devices or "chips" that include one or more reservoirs containing the agent and that release the agent or a portion thereof from the one or more reservoirs into the surrounding environment (see, e.g., U.S. Pat. Nos. 5,797,898 and 6,976,982). Release may occur through a variety of means. For example, the reservoirs may have a biodegradable cap that is impermeable to the agent and degrades over time, so that the therapeutic agent is released once the cap is degraded. Caps of differing thickness will cause release to occur at different times. Mechanical, electrical, or other means may be used to release the agent from a reservoir, optionally using external control means to regulate such release. Release can occur at predetermined times and/or in predetermined amounts. The device may be programmable.

### V. Methods of Administration

A variety of different methods, techniques, and procedures may be used to administer the first and second therapeutic agents. In certain embodiments of the invention administration is performed by intravitreal injection. While it will be appreciated that a certain amount of inter-physician variability exists.A nonlimiting example of an intravitreal procedure may be performed as follows: The tension in the eye is typically measured with a tensiometer and inflammation is graded clinically. The eyes are anesthetized with sodium channel blockers (such as novocaine) and treated with mydriatic drops (either sympathomimetic, anti-parasympathetic or myoplegic in nature), followed by further treatment with anesthetic and antibiotic drops. A speculum is then inserted under the eyelids and the patient is asked to look sideways. A caliper is used to measure a distance of 2 mm from the limbus and determine the injection site (this is done in order to avoid hitting the lens with the needle). An injection syringe (e.g., a 1 ml or 0.5 ml syringe) with a needle (e.g., a 22, 25, 27, or 30 gauge needle) is then loaded with 50-100 microliters of an agent (e.g., either 1mg Avastin or 0.3mg Macugen), or a syringe that is preloaded with the agent is used. The needle is then inserted at the injection site until the middle of the vitreous cavity is reached with the tip of the needle and the drug is slowly injected. The syringe is then slowly retracted and pressure is administered for a few seconds at the site of injection with wet gauze. More antibiotic drops are administered and the speculum is removed. The patient is then typically observed for 10 to 60 min, during which time the intraocular pressure is measured at regular intervals.

Other methods of administration include, e.g., choroidal injection, transscleral injection or placing a scleral patch, selective arterial catheterization, intraocular administration including transretinal, subconjunctival bulbar, intravitreal injection, suprachoroidal injection, subtenon injection, scleral pocket and scleral cutdown injection, by osmotic pump, etc. In choroidal injection and scleral patching, the clinician uses a local approach to the eye after initiation of appropriate anesthesia, including painkillers and ophthalmoplegics. A needle containing the therapeutic compound is directed into the subject's choroid or sclera and inserted under sterile conditions. When the needle is properly positioned the compound is injected into either or both of the choroid or sclera.

Intraocular administration of drugs intended for treatment of macular degeneration and/or other intraocular conditions by a variety of methods is well known in the art, and any suitable method can be used in the present invention. See, e.g., U.S. Patent Nos. 5,632,984 and 5,770,589. U.S. Patent No. 6,378,526 provides methods for intrascleral injection of a therapeutic or diagnostic material at a location overlying the retina, which provide a minimally invasive technique for delivering the agent to the posterior segment of the eye.

Only minor modifications of the foregoing procedures, or no modifications at all, may be needed to administer first and second therapeutic agents in accordance with the present invention. Standard injection times and pressures can be used or appropriately modified. For example, the total injection time may be longer than in the case of injecting a single agent. It will be appreciated that the nature of the modifications, if any, will be dictated at least in part by the particular procedure as well as the nature of the therapeutic agents and their formulation in addition to the manner in which they are provided for use by the clinician. For example, in one embodiment a sustained release formulation containing the second therapeutic agent, e.g., an ocular implant, is loaded into the needle. The needle may be supplied to the clinician having already been preloaded with the sustained release formulation or the clinician may load the needle with the sustained release formulation. The clinician attaches the needle to the syringe. An appropriate volume (containing an appropriate amount) of a solution containing the first therapeutic agent is then drawn up into the syringe and the the intravitreal injection procedure is performed as described above. Depression of the plunger of the syringe will eject both the first therapeutic agent and the sustained release formulation into the vitreous (or elsewhere in the eye if a technique other than intravitreal injection is used). In another embodiment, a syringe containing an appropriate volume of a solution containing the first therapeutic agent is provided to the clinician, optionally together with a needle that is preloaded with the sustained release formulation containing the second therapeutic agent Thus the clinician needs to undertake no measurement, dilution, or other manipulation of the therapeutic agents themselves.

In another embodiment, both therapeutic agents are contained in individual syringes. The injection is performed with a needle and syringe assembly, wherein the syringe contains the first therapeutic agent or, more generally, the syringe contains a composition comprising the first therapeutic agent. After administration of the therapeutic agent contained in the syringe, the syringe is removed and a second syringe, containing the second therapeutic agent, or more generally, a composition comprising the second therapeutic agent, is attached to the needle. The second therapeutic agent (or composition) is then administered. The therapeutic agents can be administered in either order. Any number of therapeutic agents can be administered consecutively, without removing the tip of the needle from the subject's eye.

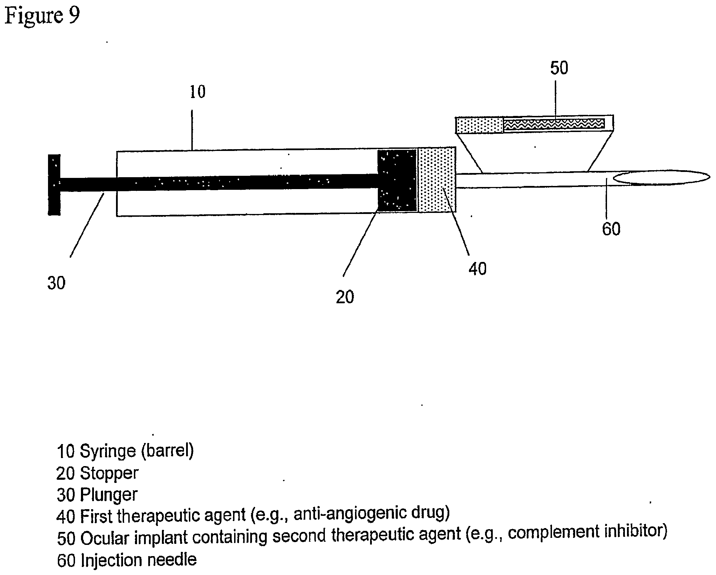

Figure 9 shows an exemplary embodiment of a needle and syringe assembly that may be used to practice the invention. The assembly includes a syringe having a barrel and a plunger with a stopper at its end. The syringe is attached to a needle, typically by means of a threaded tip with an opening (e.g., a Luer lock tip), which is not shown. The portion of the syringe between the stopper and the end of the barrel contains a therapeutic agent, e.g., an angiogenesis inhibitor. The needle contains an additional therapeutic agent, e.g., a sustained release formulation such as an ocular implant containing a therapeutic agent (e.g., a complement inhibitor). For purposes of clarity the implant is depicted outside the needle in Figure 9 though of course it would be located within the shaft of the needle for administration. Exerting pressure on the plunger following introduction of the needle into a subject's eye will eject both the therapeutic agent in the syringe and the ocular implant in the needle into the eye. In other embodiments, the syringe may be provided with additional or alternative means of ejecting the implant

In certain embodiments of the invention the first and second therapeutic agents are delivered to different structures, regions, compartments, or tissues of the eye in a single procedure. For example, a needle or other instrument may pass through different structures, regions, compartments, or tissues of the eye during the process of inserting and withdrawing the needle. The first and second therapeutic agents may be ejected into different structures, regions, compartments, or tissues of the eye in the course of a single injection procedure. For example, in one embodiment one of the therapeutic agents is introduced into the vitreous and one of the therapeutic agents in introduced into a different structure, region, compartment, or tissue of the eye in a single procedure. Either the agent that provides rapid rapid improvement in the condition of the subject's eye or the sustained release formulation of the second therapeutic agent may be introduced into the vitreous.

The volume to be administered will depend on the location within the eye to which the composition is administered. For example, for intravitreal injection volumes of 200 µl, preferably 100 µl or less are generally preferred. In certain embodiments the total volume of liquid injected is 200 µl or less, 100 µl or less, 50 µl or less. In certain embodiments of the invention the total volume of material introduced into the subject's eye (including liquid and any solid or semi-solid components) is 500 µl or less, 400 µl or less, 300 µl or less 200 µl or less, 100 µl or less, 50 µl or less, or 25 µl or less.

### VI. Gene Therapy

Disclosed herein in gene therapy, in which one or more of the therapeutic agents is a nucleic acid that encodes a therapeutic agent such as an siRNA or protein such as a VCCP in operable association with regulatory elements sufficient to direct expression of the nucleic acid is administered to the eye. A composition comprising a nucleic acid therapeutic can consist essentially of the nucleic acid or a gene therapy vector in an acceptable diluent, or can comprise a drug release regulating component such as a polymer matrix with which the nucleic acid or gene therapy vector is physically associated, e.g, with which it is mixed or within which it is encapsulated or embedded. The gene therapy vector can be a plasmid, virus, or other vector. Alternatively, the pharmaceutical composition can comprise one or more cells which produce a therapeutic nucleic acid or polypeptide. Preferably such cells secrete the therapeutic agent into the extracellular space.

Viral vectors that have been used for gene therapy protocols include, but are not limited to, retroviruses, lentiviruses, other RNA viruses such as poliovirus or Sindbis virus, adenovirus, adeno-associated virus, herpes viruses, SV 40, vaccinia and other DNA viruses. Replication-defective murine retroviral or lentiviral vectors are widely utilized gene transfer vectors. Chemical methods of gene therapy involve carrier-mediated gene transfer through the use of fusogenic lipid vesicles such as liposomes or other vesicles for membrane fusion. A carrier harboring a nucleic acid of interest can be conveniently introduced into the eye or into body fluids or the bloodstream. The carrier can be site specifically directed to the target organ or tissue in the body. Cell or tissue specific DNA-carrying liposomes, for example, can be used and the foreign nucleic acid carried by the liposome absorbed by those specific cells. Gene transfer may also involve the use of lipid-based compounds which are not liposomes. For example, lipofectins and cytofectins are lipid-based compounds containing positive ions that bind to negatively charged nucleic acids and form a complex that can ferry the nucleic acid across a cell membrane.

Certain cationic polymers spontaneously bind to and condense nucleic acids such as DNA into nanoparticles. For example, naturally occurring proteins, peptides, or derivatives thereof have been used. Synthetic cationic polymers such as polyethylenimine (PEI), polylysine (PLL) etc. condense DNA and are useful delivery vehicles. Dendrimers can also be used. Many useful polymers contain both chargeable amino groups, to allow for ionic interaction with the negatively charged DNA phosphate, and a degradable region, such as a hydrolyzable ester linkage. Examples include poly(alpha-(4-aminobutyl)-L-glycolic acid), network poly(amino ester), and poly (beta-amino esters). These complexation agents can protect nucleic acids against degradation, e.g., by nucleases, serum components, etc., and create a less negative surface charge, which may facilitate passage through hydrophobic membranes (e.g., cytoplasmic, lysosomal, endosomal, nuclear) of the cell. Certain complexation agents facilitate intracellular trafficking events such as endosomal escape, cytoplasmic transport, and nuclear entry, and can dissociate from the nucleic acid.

### VII. Articles of manufacture

In another aspect of the invention, an article of manufacture, which may be referred to as a pharmaceutical pack or kit, comprising the first and second therapeutic agents and a needle containing the second therapeutic agent, and optionally additional material or items useful for treating the disorders described above is provided. In another aspect, an article of manufacture comprises the first and second therapeutic agents of the first aspect, wherein each therapeutic agent is contained in an individual syringe and wherein the article of manufacture optionally further comprises a needle is provided. The article of manufacture can comprise a container and a label or package insert on or associated with the container. Suitable containers include, for example, bottles, vials, syringes, etc. The containers may be formed from a variety of materials such as glass or plastic. The container holds a composition which is, either alone or together with another composition effective for treating the condition. Optionally the container may have a sterile access port (for example the container may be a vial having a stopper pierceable by a hypodermic injection needle). The label or package insert may indicate that the composition is used for treating one or more conditions of choice, e.g., an eye disorder such as macular degeneration. The article of manufacture may comprise (a) a first container with a composition contained therein, wherein the composition comprises a first therapeutic agent; and (b) a second container with a composition contained therein, wherein the composition comprises a sustained release formulation of a second therapeutic agent. The article of manufacture in this embodiment of the invention may further comprise a package insert indicating that the first and second compositions can be used to treat a particular eye disorder, e.g., exudative ARMD. Alternatively, or additionally, the article of manufacture may further comprise a second (or third) container comprising a pharmaceutically acceptable liquid, such as bacteriostatic water for injection (BWFI), phosphate-buffered saline, Ringer's solution and dextrose solution.

In certain embodiments of the invention the article of manufacture may further comprise one or more devices or instruments for administering a therapeutic agent to the eye. For example, the article of manufacture may include one or more needles (e.g., a 22, 25, 27, or 30 gauge needle) and/or one or more syringes (e.g., 0.3, 0.5, or 1.0 ml syringes). Either a needle or a syringe, or both, may contain one or more compositions comprising a unit dosage form of a therapeutic agent. For example, the article of manufacture may include a needle or syringe that contains a predetermined volume and/or amount of a composition comprising a therapeutic agent. The article of manufacture may contain a needle or syringe that contains a sustained release formulation of a therapeutic agent, e.g., an ocular implant. The needle and syringe may, but need not be, attached to one another. The needle and/or syringe may be provided with a removable cap. Providing one or more of the compositions already loaded into the device that will be used to administer the agent(s) may provide increased reliability, safety, and convenience. The article of manufacture may include a plurality of syringes, each of which optionally contains a unit doseage form of a different therapeutic agent. For example, a first syringe containing a first therapeutic agent and a second syringe containing a third therapeutic agent may be included. In one embodiment a first syringe containing an angiogenesis inhibitor and a second syringe containing a complement inhibitor are provided. Either or both of the agents may be in a liquid composition. Either or both of the agents may be a component of a sustained release formulation. Each syringe may contain a composition containing a single therapeutic agent and optionally other components such as a pharmaceutically acceptable carrier. Alternately, one or more of the syringes may contain a composition containing a plurality of different therapeutic agents and optionally other components such as a pharmaceutically acceptable carrier.

The individual components described above may be packaged together in a larger container, e.g., a box, foil, styrofoam, or plastic wrapper, or other container, which may optionally contain additional packaging material. Care should be taken to use materials that will, if necessary or desirable, protect the therapeutic agent(s) from light and/or other environmental conditions and will not adversely affect them. The article of manufacture may include instructions, e.g., in the form of a package insert, that instruct the clinician as to methods by which the compositions should be administered including, if appropriate, instructions for assembling, diluting, or otherwise manipulating any individual components. In one embodiment each article of manufacture contains appropriate amounts of first and second compositions comprising first and second therapeutic agents for performing a single procedure (i.e., a single administration of first and second therapeutic agents to the eye). Optionally included are devices or instruments such as a needle and syringe for performing the procedure. The needle, syringe, or both, may be preloaded with a composition comprising a therapeutic agent. Articles of manufacture that contain one or more of any of the therapeutic agents, sustained release formulations thereof, and/or devices or instruments for administering a therapeutic agent to the eye, and any combinations thereof, are within the scope of the invention.

Preferably any composition to be administered to the eye is sterile. The composition can be made from sterile components, or sterilization can be performed after manufacture. Methods of sterilization include irradiation, heat, etc. Preferably, the sterilization method used does not substantially reduce the activity or biological or therapeutic activity of the therapeutic agents. Devices and instruments to be used for administration to the eye are also preferably sterile, at least to the extent they will enter the eye.

### VIII. Testing in Animal Models

Animal models that replicate one or more features of macular degeneration, diabetic retinopathy, CNV, inflammation, or other ocular conditions are known in the art. A compound of the invention can be administered in various doses to mice, rats, dogs, primates, etc. that have spontaneous macular degeneration and/or CNV or in which macular degeneration and/or CNV have been induced by a treatment. The ability of the compound to prevent or treat one or more signs or symptoms of macular degeneration (e.g. CNV, accumulation of lipofuscin in and/or drusen beneath the RPE, photoreceptor atrophy or hypertrophy, altered RPE pigmentation, photoreceptor loss, altered electroretinogram, etc.) is assessed. Visual examination, photography, histopathology, immunohistology, etc., can be used.

Useful models include animals (e.g., mice,Yucatan pigs, monkeys, etc.) in which CNV is induced by laser treatment (see, e.g., Bora, P.S., et al., Proc. Natl. Acad. Sci. 100(5): 2679-2684, 2003; Zacks, DN, et al., Invest Ophthalmol Vis Sci. 243(7):2384-91, 2002). Other models include animals that have been treated with a variety of agents such as lipid hydroperoxide (Tamai, K., et al., Exp Eye Res. 74(2):301-8, 2002), pellets comprising growth factors, etc. Animals genetically engineered to overexpress or underexpress one or more genes are also useful. For example, transgenic mice (mcd/mcd mice) that express a mutated form of cathepsin D that is enzymatically inactive display features associated with geographic atrophy (Rakoczy, PE, et al, Am. J. Path., 161(4), 1515-1524, 2002). Adeno-associated virus (AAV) mediated expression of vascular endothelial growth factor induces CNV in rats (Wang, F., et al., Invest Ophthalmol Vis Sci. 44(2):781-90, 2003). One animal model is a transgenic mouse deficient in either monocyte chemoattractant protein (Ccl-2) or its cognate chemokine receptor (Ccr-2) (Ambati, J., et al., Nat Med. 9(11):1390-7, 2003; U.S.S.N. 10/685,705 - U.S. Pat. Pub. No. 20040177387). Aged mice with a deficiency in either of these proteins exhibit a number of features of ARMD including accumulation of lipofuscin in and drusen beneath the RPE, photoreceptor atrophy, and CNV. Methods for testing the efficacy of a candidate agent using this mouse model are disclosed in U.S. Pat. Pub. No. 20040177387. In general, a candidate agent is administered to the mouse either before or after development of features of ARMD, and at least one eye is monitored for development or regression of drusen and/or lipofuscin accumulation therein, for effect of the candidate agent on Bruch's membrane, effect on retinal degeneration, and/or for effect on CNV.

The therapeutic agents are administered as described herein. The eye can be- analyzed by ophthalmoscopy (e.g., indirect ophthalmoscopy, slit lamp assessment), angiography (e.g., fluorescein angiography), histopathology, optical coherence tomography (OCT), fundus photography, or a combination thereof. Any of these methods can be used to assess efficacy in any animal model or in humans. Compounds that show promising results in animal studies are tested in humans, e.g., using standard protocols and endpoints for clinical trials for therapies for ARMD or diabetic retinopathy but it will be appreciated that agents may be administered to humans without evidence of efficacy in animal models.

### IX. Identifying Subjects and Assessing Response

The invention may include providing a subject to which a composition of the invention is to be administered. The subject is typically suffering from an eye disorder characterized by macular degeneration, CNV, or RNV. The compositions are administered to the subject according to the invention with the intent of treating or preventing such condition. Thus the subject will typically have been identified as being at risk of or suffering from such a condition. Methods for diagnosis of macular degeneration, CNV, and RNV etc., and for assessing response to therapy are known in the art.

Any suitable tests and criteria can be used to identify a subject at risk of or suffering from a macular degeneration related condition, diabetic retinopathy, or CNV and/or to evaluate the condition of a subject's eye either prior to or following therapy (e.g., to determine whether the subject is in need of therapy or has responded to therapy). Visual acuity can be measured using, for example, a Snellen chart, a Bailey-Lovie chart, a decimal progression chart, a Freiburg visual acuity test, a measurement of minimum angle of resolution (MAR) etc. Metamorphopsia (visual distortion) may be measured using an Amsler chart. Contrast sensitivity may be measured using a Pelli-Robson chart. Diagnostic studies include, but are not limited to, standard ophthalmologic examination of the fundus, stereo biomicroscopic examination of the macula, intravenous fundus fluorescein angiography, fundus photography, indocyanine green video-angiography, and optical coherence tomography (OCT). OCT may be of particular use for measuring macular thickness, an indicator of macular edema. A subject displaying an abnormality on one or more of these diagnostic studies (e.g., a subject that falls outside a range that is considered normal for a healthy eye) may be treated in accordance with the present invention.

Subjects may be classified as having early, intermediate, or advanced ARMD in accordance with the classification scheme used in the Age-Related Eye Diseases Study (AREDS), which is set forth in guidelines developed American Academy of Ophthalmology (American Academy of Ophthalmology, Age Related Macular Degeneration Preferred Practice Pattern™, 2003; available for download at URL www.aao.org/aao/education/library/ppp/amd_new.cfm). A subject falling into any of these categories may be treated in accordance with the present invention. If the subject has already developed CNV, the subject may have classic CNV, occult CNV, or a mixture of the two. Of course alternate classification schemes, of which a variety is described in the literature, could be used.

ARMD is known to have a genetic component, based on studies showing an increased incidence of ARMD in individuals with relatives suffering from ARMD (e.g., twin studies). Therefore, a subject may be considered at risk of developing ARMD if he or she has one or more close relatives (e.g., parent, grandparent, sibling, cousin, uncle, aunt), who has received a diagnosis of ARMD. Individuals who have certain polymorphic variants of genes encoding certain complement components, such as the factor H gene, are particularly prone to develop ARMD (see, e.g., Klein RJ, et al., Zeiss C, Science, 308(5720):385-9, 2005; Edwards AO, et al., Science, 308(5720):421-4, 2005; Haines JL, et al., Science, 308(5720):419-21, 2005). In one embodiment the method comprises providing or ascertaining a genotype of a subject, wherein the genotype includes information as to the presence of one or more polymorphisms predisposing to ARMD. Optionally the gene encodes a complement component, e.g., factor H or factor B.

Individuals who smoke and/or consume a high fat diet are also at increased risk. The incidence of ARMD increases with age. Therefore, an individual over approximately 50 years of age, generally at least 60 or at least 70 years of age may be considered at increased risk. An individual having drusen and one or more additional risk factors may be at particular risk for developing ARMD. An individual with multiple drusen, particularly if large and with indistinct borders, may be at particular risk. An individual with RPE hyperpigmentation or hypopigmentation or geographic atrophy may be at particular risk. Specific genetic mutations are associated with various less common macular degeneration related conditions. A subject who has received a diagnosis of diabetes is at risk of developing diabetic retinopathy. In addition, a subject who has developed ARMD in one eye is at increased risk of developing the disorder in the other eye.

Response to therapy can be assessed by any of the methods mentioned above. Numerous studies have been conducted to assess the efficacy of a variety of different therapies in restoring vision, preventing visual loss, and/or resulting in improvement or slowing progression of ARMD or choroidal neovascularization as judged by diagnostic tests such as those described above. One of ordinary skill in the art will be able to select appropriate criteria by which to judge the efficacy of therapy.

Rapid improvement in the condition of the subject's eye may be, for example, any clinically significant improvement in a sign or symptom associated with the eye disorder. For example, the improvement may be an improvement in visual acuity (e.g., best corrected visual acuity) such as gaining 1, 2, 3, or more lines on an eye chart. The improvement may be a decrease in macular thickness, e.g., a decrease by at least 50 µm, at least 100 µm, at least 150 µm, etc. The improvement can be a decrease in the area of exudate evident in or on the retina.

The improvement can be an increase of at least 20%, 30%, 40%, 50%, 60%, 70%, 80%, 90%, 100%, 200%, 300%, or more, in any quantitative measure of the condition of the subject's eye, where an increase in the quantitative measure indicates improvement in the condition of the subject's eye. The improvement can be a decrease of at least 20%, 30%, 40%, 50%, 60%, 70%, 80%, 90%, or more, in any quantitative measure of the condition of the subject's eye, where a decrease in the quantitative measure indicates improvement in the condition of the subject's eye. If a scoring system is used as an indication of the condition of the subject's eye, (e.g., a scoring system including scores ranging from 1-3, 1-5, 1-10, etc.), the improvement may be, e.g., an increase of at least 1, 2, 3, or more units, where an increase in the score indicates improvement in the condition of the subject's eye. Alternately, if a scoring system in which a decrease in the score indicates an improvement in the condition of the subject's eye is used, the improvement may be a decrease of at least 1, 2, 3, or more units. One of ordinary skill in the art will appreciate that a variety of scoring systems may be used. For example, scores may be expressed in terms of units such as "+" or "-" rather than with integers. Of course it will be understood that individual responses will vary, and not all sujbects will respond. In animal studies and in clinical trials, changes in the condition of a subject's eye may be expressed in terms of an average or mean change in the population studied and/or using appropriate statistical tests.

In one example, subjects with exudative ARMD are divided into two groups. A variety of parameters, for example visual acuity (e.g., best corrected visual acuity), contrast sensitivity, visual distortion, retinal hemorrhage number or area, macular thickness, etc., are evaluated to provide baseline indication of the condition of the subject's eye. One group receives a single intravitreal injection of a first therapeutic agent, e.g., an angiogenesis inhibitor such as an anti-VEGF agent, e.g., Lucentis, Avastin, or Macugen. The other group receives the same dose of the first therapeutic agent and also receives a sustained release formulation of a second therapeutic agent (e.g., a complement inhibitor such as compstatin or a derivative thereof), with both agents being administered together by a single intravitreal injection. The sustained release formulation may be, e.g., a cylindrical or screw-shaped ocular implant comprising the second therapeutic agent, a plurality of particles comprising the agent, a composition that forms a discrete solid or semi-solid structure such as a gel following administration, etc. The groups are monitored over time. Parameters such as visual acuity (e.g., best corrected visual acuity), contrast sensitivity, visual distortion, retinal hemorrhage number or area, macular thickness, etc., are evaluated, preferably using the same methods and metrics as were used in the initial evaluation to determine baseline values. For example, resolution of macular edema may be monitored by OCT. Evaluations to determine the number of subjects who experience rapid improvement in the condition of a treated eye can take place, e.g., 1 week, 10 days, or 2 weeks following treatment. The number of subjects that experience rapid improvement in the condition of a treated eye (e.g., rapid decrease in macular edema and its associated visual disturbances) is compared between the two groups. Also monitored is the average time to destabilization, e.g., the average time before an acute deterioration in one or more of the foregoing parameters occurs. Also monitored (e.g., using fluorescein angiography and/or ophthalmologic examination) is the extent of neovascularization and/or vessel leakage at various time points following treatment, e.g., at 30, 60, 90, 120, 150, and 180 day time points and at 30 day intervals thereafter (or an appropriate subset of these time points). The change from baseline in a retinal thickness score may be evaluated and compared between the two groups. A greater mean decrease in retinal thickness at one or more of the foregoing time points in the group that received the combined therapy of the present invention is indicative that the combined therapy of the present invention provides a therapeutic advantage for treating the eye disorder. The change from baseline in fluorescein leakage score (where the fluorescein leakage score provides an indication of neovascularization and/or vessel leakage and a higher score indicates a greater amount of neovascularization and/or vessel leakage) may be evaluated. A greater mean decrease in fluorescein leakage score from baseline in the in the group that received the combined therapy of the present invention is indicative that the combined therapy of the present invention provides a therapeutic advantage for treating the eye disorder.

Each of the above examples is repeated except that the first and second therapeutic agents are both complement inhibitors or combinations thereof. For example, the first therapeutic agent is a VCCP and the second therapeutic agent is a GPCRA. Alternately, the first therapeutic agent is compstatin or a derivative thereof and the second therapeutic agent is a VCCP.

Each of the above examples is repeated except that one of the groups receives, by a single intravitreal injection, a composition comprising multiple therapeutic agents in a liquid medium and a sustained release formulation containing at least one therapeutic agent The multiple therapeutic agents can be, for example, different angiogenesis inhibitors. Alternately, the multiple therapeutic agents can include a complement inhibitor and an angiogenesis inhibitor. The sustained release formulation may contain, for example, two or more different complement inhibitors or a complement inhibitor and an angiogenesis inhibitor. For example, in one embodiment the sustained release formulation contains a compstatin analog and Lucentis.

Each of the above examples is repeated except that at least one therapeutic agent is an RNAi agent. For example, the first or second therapeutic agent is an siRNA that inhibits expression of one or more endogenous pro-angiogenic molecules such as one or more VEGF isoforms, one or more VEGF receptors, one or more complement components, etc. In one embodiment the sustained release formulation contains at least two RNAi agents, each of which inhibits expression of a different pro-angiogenic molecule. For example, the sustained release formulation may contain Cand5 and Sima-027.

Each of the above examples is repeated in subjects suffering from diabetic retinopathy.

In another example the ability of the invention to inhibit progression of early ARMD (AREDS 2) to intermediate ARMD (AREDS 3) is assessed. Subjects with early ARMD are divided into two groups, one of which receives an inventive combination of agents as described in either of the two examples above while the other receives either no therapy or an alternative therapy such as therapy with a single agent, e.g., Lucentis, Avastin, or Macugen as described in either of the two examples above. The groups are monitored for a period of time (e.g., as described above). In addition the percentage of subjects that progress from early to intermediate ARMD is determined. A lower proportion of subjects that progress to intermediate ARMD in the group that receives the combined therapy of the present invention is indicative that the combined therapy of the present invention provides a therapeutic advantage for treating the eye disorder.

In another example the ability of the invention to inhibit progression of intermediate ARMD (AREDS 3) to advanced ARMD (AREDS 4) is assessed. Subjects with intermediate ARMD are divided into two groups, one of which receives an inventive combination of agents as described in either of the two examples above while the other receives either no therapy or an alternative therapy such as Lucentis, Avastin, Macugen as described in either of the two examples above. The groups are monitored for a period of time (e.g., as described above). The percentage of subjects that progress from intermediate to advanced ARMD is determined. A lower proportion of subjects that progress to advanced ARMD in the group that receives the combined therapy of the present invention is indicative that the combined therapy of the present invention provides a therapeutic advantage for treating the eye disorder.

In addition to monitoring progression of ARMD, the incidence of side effects and complications may also be monitored. Consideration of side effects is an important aspect when evaluating the overall outcome and risk/benefit ratio of a therapy. For example, if two therapies are equally efficacious in terms of inhibiting progression of or treating ARMD, the therapy with a lower incidence of side effects (e.g., severe complications such as those mentioned above) is typically preferred for most subjects. In certain embodiments of the invention therapy of a disorder such as ARMD, or a disorder featuring CNV or RNV from any cause, using the methods and compositions of the invention is associated with fewer total side effects, e.g., severe complications, over time (e.g., over a 1-2 year period) than therapy in which multiple agents are administered individually or therapy in which only a single therapeutic agent is used.

### Equivalents and Scope

In the claims and elsewhere in the specification, articles such as "a,", "an" and "the" may mean one or more than one unless indicated to the contrary or otherwise evident from the context. For example, the indefinite articles "a" and "an", as used herein in the specification and in the claims, unless clearly indicated to the contrary, should be understood to mean "at least one". Claims or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention also includes embodiments in which more than one, or all of the group members are present in, employed in, or otherwise relevant to a given product or process. Furthermore, it is to be understood that the invention encompasses all variations, combinations, and permutations in which one or more limitations, elements, clauses, descriptive terms, etc., from one or more of the listed claims (or from the portion of the specification relevant to such claim or claim element) is introduced into another claim. For example, and without limitation, any claim that is dependent on another claim can be modified to include one or more elements or limitations found in any other claim (or from the portion of the specification relevant to such claim or claim element) that is dependent on the same base claim. Furthermore, where the claims or description recite a composition, it is to be understood that methods of administering the composition according to any of the methods disclosed herein, and methods of using the composition for any of the purposes disclosed herein are included, and methods of making the composition according to any of the methods of making disclosed herein are included, unless otherwise indicated or unless it would be evident to one of ordinary skill in the art that a contradiction or inconsistency would arise. The invention encompasses all variations, combinations, and permutations in which one or more elements, clauses, descriptive terms, etc., from one or more of the listed claims is introduced into another claim dependent on the same base claim unless otherwise indicated or unless it would be evident to one of ordinary skill in the art that a contradiction or inconsistency would arise.

Where elements are presented as lists, e.g., in Markush group format or the like, it is to be understood that each subgroup of the elements is also disclosed, and any element(s) can be removed from the group. It should it be understood that, in general, where the invention, or aspects of the invention, is/are referred to as comprising particular elements, features, etc., certain embodiments of the invention or aspects of the invention consist, or consist essentially of, such elements, features, etc. For purposes of simplicity those embodiments have not been specifically set forth ***in haec verba*** herein in all cases.

The inclusion of a "providing a subject..." step in certain embodiments of the invention is intended to indicate that the composition is administered to treat an eye disorder. Thus the subject will have or be at risk of an eye disorder and the composition is administered to treat the disorder, typically upon the sound recommendation of a medical or surgical practitioner, e.g., an ophthalmologist, who may or may not be the same individual who administers the composition. The invention includes embodiments in which a step of providing is not explicitly included and embodiments in which a step of providing is included. The invention also includes embodiments in which a step of identifying the subject as being at risk of or suffering from a eye disorder characterized by macular degeneration, CNV, or RNV, is included.

Where ranges are given, endpoints are included and the invention includes embodiments in which either or both endpoints are excluded. Furthermore, it is to be understood that unless otherwise indicated or otherwise evident from the context and understanding of one of ordinary skill in the art, values expressed as ranges can assume any specific value or subrange within the stated ranges in different embodiments of the invention, to the tenth of the unit of the lower limit of the range, unless the context clearly dictates otherwise.

## Claims

1. First and second therapeutic agents for use in treating an eye disorder **characterized by** macular degeneration, choroidal neovascularisation or retinal neovascularisation, wherein
effective amounts of the first and second therapeutic agents are administered to the subject's eye, optionally in a single procedure;
the first therapeutic agent is an anti-VEGF agent selected from the group consisting of antibodies or antibody fragments that bind to VEGF, a fusion protein containing extracellular domains of two VEGF receptors connected to the Fc region of an antibody, and nucleic acids that bind to VEGF; and
the second therapeutic agent is selected from the group consisting of a compstatin analog having at least 5-fold higher complement inhibiting activity than compstatin, a monoclonal antibody or monoclonal antibody fragment that binds to C3 and an antibody or antibody fragment that binds to C5, factor B or factor D.

2. The first and second therapeutic agents for use according to claim 1, wherein the compstatin analog is
a) a compound that comprises a cyclic peptide having a core sequence of X'aa-Gln - Asp - Xaa - Gly (SEQ ID NO: 3), where X'aa and Xaa are selected from Trp and analogs of Trp, or
b) a compound that comprises a cyclic peptide having a core sequence of X'aa-Gln - Asp - Xaa - Gly-X"aa (SEQ ID NO: 4), where X'aa and Xaa are each independently selected from Trp and analogs of Trp and X"aa is selected from His, Ala, single methyl unbranched amino acids, Phe, Trp, and analogs of Trp, or
c) a compound that comprises a cyclic peptide having a sequence of X'aa1 - X'aa2 - X'aa3 - X'aa4-Gln-Asp-Xaa-Gly- X"aa1- X"aa2- X"aa3- X"aa4- X"aa5 (SEQ ID NO: 5), where X'aa4 and Xaa are selected from Trp and analogs of Trp, wherein X'aa1, X'aa2, X'aa3, X"aa1, X"aa2, X"aa3, X"aa4, and X"aa5 are independently selected from among amino acids and amino acid analogs, and the peptide is cyclized via a bond between X'aa2 and X"aa4.

3. The first and second therapeutic agents for use according to claim 1, wherein the procedure is an injection procedure, such as an injection procedure in which the first and second therapeutic agents are injected into the vitreous of the subject's eye and/or an injection procedure in which, prior to administration, the first therapeutic agent is contained in a syringe and the second therapeutic agent is contained in a needle attached to the syringe and wherein, preferably, the first therapeutic agent is dissolved in a liquid medium located in the syringe and the second therapeutic agent comprises an ocular implant located in the needle.

4. The first and second therapeutic agents for use according to any preceding claim, wherein the first therapeutic agent or composition is selected from the group consisting of bevacizumab, ranibizumab, pegaptanib and VEGF-Trap.

5. The first and second therapeutic agents for use according to claim 1, wherein the first therapeutic agent or composition is dissolved or suspended in a liquid medium prior to administration and/or the second therapeutic agent comprises an ocular implant, wherein optionally the second therapeutic agent is released from the ocular implant so as to maintain a therapeutic level in the subject's eye over a period of at least three months.

6. The first and second therapeutic agents for use according to claim 1, wherein the second therapeutic agent is administered as a sustained release formulation which comprises a polymeric material such as polymeric material selected from the group consisting of: poly-lactic acid (PLA), poly-glycolic acid (PGA), poly-lactide-co- glycolide (PLGA)5 poly(phosphazine), poly (phosphate ester), polycaprolactones, polyanhydrides, ethylene vinyl acetate, polyorthoesters, polyethers, poly (beta amino esters), copolymers containing monomeric subunits found in any of the foregoing polymers, collagen, albumin, chitosan, alginate, hyaluronic acid, and mixtures of any of the foregoing polymers and preferably the polymeric material is biodegradable.

7. The first and second therapeutic agents for use according to claim 1, wherein the second therapeutic agent is administered as a sustained release formulation which comprises nanoparticles, microparticles, dendrimers, or liposomes comprising the second therapeutic agent, a solid or semi-solid material that entraps or encapsulates the second therapeutic agent, or an inactive material to which the second therapeutic agent is covalently attached.

8. The first and second therapeutic agents for use according to claim 1, wherein the first therapeutic agent is administered in soluble or particulate form in a liquid medium and the second therapeutic agent is administered in or attached to a solid or semi-solid matrix and/or wherein the second therapeutic agent, when administered as a component of a sustained release formulation, has an activity period greater than that of the first therapeutic agent.

9. The first and second therapeutic agents for use according to claim 1, wherein administering the second therapeutic agent prolongs the time interval during which the subject experiences improvement in the condition of the subject's eye relative to the time interval during which the subject would have experienced improvement if the first therapeutic agent had been administered as sole therapy.

10. The first and second therapeutic agents for use according to any preceding claim, wherein the eye disorder is exudative age-related macular degeneration.

11. The first and second therapeutic agents for use according to claim 1, wherein the subject has experienced a perceptible deterioration in the condition of the subject's eye within the two weeks preceding administration of the first and second therapeutic agents.

12. The first and second therapeutic agents for use according to claim 1, wherein the procedure is performed one or more additional times at time intervals greater than the activity period of the first therapeutic agent.

13. An article of manufacture comprising the first and second therapeutic agents of claim 1, wherein the article of manufacture comprises a needle containing the second therapeutic agent, wherein the article of manufacture optionally further comprises a syringe, such as a syringe that contains the first therapeutic agent and/or wherein the article of manufacture contains a unit dosage form of the first therapeutic agent and/or a unit dosage form of the second therapeutic agent.

14. The article of manufacture of claim 13, further comprising a syringe, wherein the article of manufacture contains at least one compartment and the syringe and needle are housed in a single compartment of the article of manufacture and/or wherein the syringe and needle are attached to one another or wherein the article of manufacture contains at least two compartments, and wherein the syringe and needle are housed in individual compartments.

15. An article of manufacture comprising the first and second therapeutic agents of claim 1, wherein each therapeutic agent is contained in an individual syringe and wherein the article of manufacture optionally further comprises a needle.

## Patentansprüche

1. Erstes und zweites therapeutisches Mittel zur Verwendung bei der Behandlung einer Augenkrankheit, **gekennzeichnet durch** Makuladegeneration, choroidale Neovaskularisation oder retinale Neovaskularisation, wobei
wirksame Mengen des ersten und des zweiten therapeutischen Mittels an das Auge des Patienten verabreicht werden, optional in einer einzigen Prozedur;
das erste therapeutische Mittel ein Anti-VEGF-Mittel ist, das ausgewählt ist aus der Gruppe bestehend aus Antikörpern oder Antikörper-Fragmenten, die an VEGF binden, einem Fusionsprotein, das extrazelluläre Domänen zweier VEGF-Rezeptoren, die gebunden sind an die Fc-Region eines Antikörpers, enthält, und Nukleinsäuren, die an VEGF binden; und
das zweite therapeutische Mittel ausgewählt ist aus der Gruppe bestehend aus einem Compstatin-Analogon mit mindestens 5-fach höherer Komplement-inhibierender Aktivität als Compstatin, einem monoklonalen Antikörper oder monoklonalen Antikörper-Fragment, der/das an C3 bindet, und einem Antikörper oder Antikörper-Fragment, der/das an C5, Faktor B oder Faktor D bindet.

2. Das erste und das zweite therapeutische Mittel zur Verwendung gemäß Anspruch 1, worin das Compstatin-Analogon ist
a) eine Verbindung, die ein cyclisches Peptid mit X'aa - Gln - Asp - Xaa - Gly (SEQ ID NO: 3) als Kernsequenz umfasst, wobei X'aa und Xaa ausgewählt sind aus Trp und Analoga von Trp, oder
b) eine Verbindung, die ein cyclisches Peptid mit X'aa - Gln - Asp - Xaa - Gly - X"aa (SEQ ID NO: 4) als Kernsequenz umfasst, wobei X'aa und Xaa jeweils unabhängig ausgewählt sind aus Trp und Analoga von Trp, und X"aa ausgewählt ist aus His, Ala, unverzweigten Aminosäuren mit einer Methylgruppe, Phe, Trp und Analoga von Trp, oder
c) eine Verbindung, die ein cyclisches Peptid mit X'aa1 - X'aa2 - X'aa3 - X'aa4 - Gln - Asp - Xaa - Gly - X"aa1 - X"aa2 - X"aa3 - X"aa4 - X"aa5 (SEQ ID NO: 5) als Sequenz umfasst, wobei X'aa4 und Xaa ausgewählt sind aus Trp und Analoga von Trp, wobei X'aa1, X'aa2, X'aa3, X"aa1, X"aa2, X"aa3, X"aa4 und X"aa5 unabhängig ausgewählt sind aus Aminosäuren und Aminosäure-Analoga, und das Peptid über eine Bindung zwischen X'aa2 und X"aa4 cyclisiert ist.

3. Das erste und das zweite therapeutische Mittel zur Verwendung gemäß Anspruch 1, wobei die Prozedur eine Injektionsprozedur ist, wie etwa eine Injektionsprozedur, bei welcher das erste und das zweite therapeutische Mittel in den Glaskörper des Patientenauges injiziert werden und/oder eine Injektionsprozedur, bei welcher, vor der Verabreichung, das erste therapeutische Mittel in einer Spritze enthalten ist und das zweite therapeutische Mittel in einer an der Spritze befestigten Nadel enthalten ist und wobei, vorzugsweise, das erste therapeutische Mittel in einem in der Spritze befindlichen Flüssigmedium gelöst wird und das zweite therapeutische Mittel ein in der Nadel befindliches Augenimplantat umfasst.

4. Das erste und das zweite therapeutische Mittel zur Verwendung gemäß jedem vorangehenden Anspruch, wobei das/die erste therapeutische Mittel oder Zusammensetzung ausgewählt ist aus der Gruppe bestehend aus Bevacizumab, Ranibizumab, Pegaptanib und VEGF-Trap.

5. Das erste und das zweite therapeutische Mittel zur Verwendung gemäß Anspruch 1, wobei das/die erste therapeutische Mittel oder Zusammensetzung in einem Flüssigmedium vor der Verabreichung gelöst oder suspendiert wird und/oder das zweite therapeutische Mittel ein Augenimplantat umfasst, wobei optional das zweite therapeutische Mittel aus dem Augenimplantat freigesetzt wird, um einen therapeutischen Spiegel in dem Patientenauge über einen Zeitraum von mindestens drei Monaten aufrechtzuerhalten.

6. Das erste und das zweite therapeutische Mittel zur Verwendung gemäß Anspruch 1, wobei das zweite therapeutische Mittel als einer anhaltend freisetzenden Formulierung verabreicht wird, welche ein polymeres Material umfasst, wie ein polymeres Material, das ausgewählt ist aus der Gruppe bestehend aus: Polymilchsäure (PLA), Polyglykolsäure (PGA), Poly-(lactid-co-glycolid) (PLGA), Polyphosphazin, Polyphosphatester, Polycaprolaktonen, Polyanhydriden, Ethylenvinylacetat, Polyorthoestern, Polyethern, Poly-beta-Aminoestern, Copolymeren, die in irgendeinem der vorangegangenen Polymere zu findende monomere Untereinheiten enthalten, Kollagen, Albumin, Chitosan, Alginat, Hyaluronsäure, und Gemische aus Beliebigen der vorangegangenen Polymere, und das polymere Material vorzugsweise biologisch abbaubar ist.

7. Das erste und das zweite therapeutische Mittel zur Verwendung gemäß Anspruch 1, wobei das zweite therapeutische Mittel als einer anhaltend freisetzenden Formulierung verabreicht wird, welche Nanopartikel, Mikropartikel, Dendrimere oder Liposome, umfassend das zweite therapeutische Mittel, ein festes oder halbfestes Material, welches das zweite therapeutische Mittel einschließt oder einkapselt, oder ein inaktives Material, an das das zweite therapeutische Mittel kovalent gebunden ist, umfasst.

8. Das erste und das zweite therapeutische Mittel zur Verwendung gemäß Anspruch 1, wobei das erste therapeutische Mittel in löslicher oder partikulärer Form in einem Flüssigmedium verabreicht wird und das zweite therapeutische Mittel in einer festen oder halbfesten Matrix oder daran angelagert verabreicht wird und/oder wobei das zweite therapeutische Mittel, wenn verabreicht als einer Komponente einer anhaltend freisetzenden Formulierung, eine Aktivitätsperiode aufweist, die größer ist als die des ersten therapeutischen Mittels.

9. Das erste und das zweite therapeutische Mittel zur Verwendung gemäß Anspruch 1, wobei die Verabreichung des zweiten therapeutischen Mittels das Zeitintervall verlängert, während dessen das Individuum eine Verbesserung im Zustand des Patientenauges erfährt, relativ zu dem Zeitintervall, während dessen das Individuum eine Verbesserung erfahren hätte, wenn das erste therapeutische Mittel als einzige Therapie verabreicht worden wäre.

10. Das erste und das zweite therapeutische Mittel zur Verwendung gemäß jedem vorangehenden Anspruch, wobei die Augenkrankheit eine exsudative altersbezogene Makuladegeneration ist.

11. Das erste und das zweite therapeutische Mittel zur Verwendung gemäß Anspruch 1, wobei das Individuum eine merkliche Verschlechterung im Zustand des Patientenauges innerhalb der zwei der Verabreichung des ersten und des zweiten therapeutischen Mittels vorausgehenden Wochen erfahren hat.

12. Das erste und das zweite therapeutische Mittel zur Verwendung gemäß Anspruch 1, wobei die Prozedur ein oder mehrere zusätzliche Male in Zeitintervallen von größer als der Aktivitätsperiode des ersten therapeutischen Mittels vorgenommen wird.

13. Herstellungsgegenstand, umfassend das erste und das zweite therapeutische Mittel nach Anspruch 1, wobei der Herstellungsgegenstand eine Nadel umfasst, die das zweite therapeutische Mittel enthält, wobei der Herstellungsgegenstand optional außerdem eine Spritze umfasst, wie eine Spritze, die das erste therapeutische Mittel enthält, und/oder wobei der Herstellungsgegenstand eine Einheitendosierungsform des ersten therapeutischen Mittels und/oder eine Einheitendosierungsform des zweiten therapeutischen Mittels enthält.

14. Der Herstellungsgegenstand nach Anspruch 13, außerdem umfassend eine Spritze, wobei der Herstellungsgegenstand mindestens ein Kompartiment enthält und die Spritze und Nadel in einem einzigen Kompartiment des Herstellungsgegenstands untergebracht sind und/oder wobei die Spritze und Nadel miteinander verbunden sind oder wobei der Herstellungsgegenstand mindestens zwei Kompartimente enthält und wobei die Spritze und Nadel in individuellen Kompartiments untergebracht sind.

15. Herstellungsgegenstand, umfassend das erste und das zweite therapeutische Mittel nach Anspruch 1, wobei jedes therapeutische Mittel in einer individuellen Spritze enthalten ist und wobei der Herstellungsgegenstand optional außerdem eine Nadel umfasst.

## Revendications

1. Premier et second agents thérapeutiques destinés à être utilisés dans le traitement d'un trouble oculaire **caractérisé par** une dégénérescence maculaire, une néovascularisation choroïdienne ou une néovascularisation rétinienne, où :
des quantités efficaces du premier et du second agents thérapeutiques sont administrées à l'oeil du sujet, facultativement en une procédure unique ;
le premier agent thérapeutique est un agent anti-VEGF choisi dans le groupe constitué d'anticorps ou de fragments d'anticorps qui se lient au VEGF, d'une protéine de fusion contenant des domaines extracellulaires de deux récepteurs du VEGF connectés à la région Fc d'un anticorps, et d'acides nucléiques qui se lient au VEGF ; et
le second agent thérapeutique est choisi dans le groupe constitué d'un analogue de la compstatine, ayant une activité inhibitrice du complément au moins 5 fois plus forte que la compstatine, d'un anticorps monoclonal ou d'un fragment d'anticorps monoclonal qui se lie à C3, ou d'un anticorps ou d'un fragment d'anticorps qui se lie à C5, au facteur B ou au facteur D.

2. Premier et second agents thérapeutiques destiné à être utilisés selon la revendication 1, où l'analogue de la compstatine est :
a) un composé qui comprend un peptide cyclique ayant une séquence fondamentale X'aa-Gln-Asp-Xaa-Gly (SEQ ID n°3), où X'aa et Xaa sont choisis parmi Trp et des analogues de Trp, ou
b) un composé qui comprend un peptide cyclique ayant une séquence fondamentale X'aa-Gln-Asp-Xaa-Gly-X"aa (SEQ ID n°4), où X'aa et Xaa sont choisis chacun indépendamment parmi Trp et des analogues de Trp, et X"aa est choisi parmi His, Ala, des acides aminés non-ramifiés mono-méthyle, Phe, Trp et des analogues de Trp, ou
c) un composé qui comprend un peptide cyclique ayant une séquence fondamentale X'aa1-X'aa2-X'aa3-X'aa4-Gln-Asp-Xaa-Gly-X"aa1-X"aa2-X"aa3-X"aa4-X"aa5 (SEQ ID n°5), où X'aa4 et Xaa sont choisis parmi Trp et des analogues de Trp, où X'aa1, X'aa2, X'aa3, X"aa1, X"aa2, X"aa3, X"aa4 et X"aa5 sont choisis indépendamment parmi des acides aminés et des analogues d'acides aminés, et le peptide est cyclisé par une liaison entre X'aa2 et X"aa4.

3. Premier et second agents thérapeutiques destiné à être utilisés selon la revendication 1, où la procédure est une procédure d'injection, comme une procédure d'injection dans laquelle le premier et le second agents thérapeutiques sont injectés dans l'humeur vitrée de l'oeil du sujet et/ou une procédure d'injection dans laquelle, avant administration, le premier agent thérapeutique est contenu dans une seringue et le second agent thérapeutique est contenu dans une aiguille attachée à la seringue, et où, de préférence, le premier agent thérapeutique est dissout dans un milieu liquide situé dans la seringue et le second agent thérapeutique comprend un implant oculaire situé dans l'aiguille.

4. Premier et second agents thérapeutiques destiné à être utilisés selon l'une quelconque des revendications précédentes, le premier agent thérapeutique ou la composition étant choisi dans le groupe constitué du bévacizumab, du ranibizumab, du pégaptanib et du VEGF-Trap.

5. Premier et second agents thérapeutiques destiné à être utilisés selon la revendication 1, où le premier agent thérapeutique ou composition est dissout ou mis en suspension dans un milieu liquide avant administration et/ou le second agent thérapeutique est libéré de l'implant oculaire de façon à maintenir un niveau thérapeutique dans l'oeil du sujet sur une période d'au moins trois mois.

6. Premier et second agents thérapeutiques destiné à être utilisés selon la revendication 1, où le second agent thérapeutique est administré sous forme d'une formulation à libération retardée qui comprend un matériau polymère, comme un matériau polymère choisi dans le groupe constitué de : acide polylactique (PLA), acide polyglycolique (PGA), polylactide coglycolique (PLGA), polyphosphazine, polyester phosphorique, polycaprolactones, polyanhydrides, éthylène vinylacétate, polyorthoesters, polyéthers, poly(β-amino esters), copolymères contenant des sous-unités monomères que l'on trouve dans n'importe lequel des polymères ci-dessus : collagène, albumine, chitosan, alginate, acide hyaluronique, et leurs mélanges, ledit matériau polymère étant de préférence biodégradable.

7. Premier et second agents thérapeutiques destiné à être utilisés selon la revendication 1, où le second agent thérapeutique est administré sous forme d'une formulation à libération retardée qui comprend des nanoparticules, des microparticules, des dendrimères ou des liposomes contenant le second agent thérapeutique, un matériau solide ou semi-solide qui piège ou encapsule le second agent thérapeutique, ou un matériau inactif auquel le second agent thérapeutique est lié de façon covalente.

8. Premier et second agents thérapeutiques destiné à être utilisés selon la revendication 1, où le premier agent thérapeutique est administré sous forme soluble ou particulaire dans un milieu liquide, et le second agent thérapeutique est administré dans ou attaché à une matrice solide ou semi-solide, et/ou le second agent thérapeutique, quand on l'administre comme composant d'une formulation à libération prolongée, a une période d'activité supérieure à celle du premier agent thérapeutique.

9. Premier et second agents thérapeutiques destiné à être utilisés selon la revendication 1, où l'administration du second agent thérapeutique prolonge l'intervalle de temps pendant lequel le sujet connaît une amélioration de l'état de son oeil par rapport à l'intervalle de temps où il aurait connu une amélioration si le premier agent thérapeutique avait été administré comme seule thérapie.

10. Premier et second agents thérapeutiques destiné à être utilisés selon l'une quelconque des revendications précédentes, le trouble oculaire étant une dégénérescence maculaire liée à l'âge exsudative.

11. Premier et second agents thérapeutiques destiné à être utilisés selon la revendication 1, où le sujet a connu une amélioration perceptible de l'état de son oeil dans les deux semaines qui précèdent l'administration du premier et du second agents thérapeutiques.

12. Premier et second agents thérapeutiques destiné à être utilisés selon la revendication 1, où la procédure est effectuée au moins une fois de plus à intervalles de temps supérieurs à la période d'activité du premier agent thérapeutique.

13. Article manufacturé comprenant les premier et second agents thérapeutiques selon la revendication 1, l'article manufacturé comprenant une aiguille contenant le second agent thérapeutique, l'article manufacturé comprenant facultativement une seringue, comme une seringue contenant le premier agent thérapeutique, et/ou l'article manufacturé contenant une forme de dosage unitaire du premier agent thérapeutique et/ou une forme de dosage unitaire du second agent thérapeutique.

14. Article manufacturé selon la revendication 13, comprenant en outre une seringue, où l'article manufacturé comprend au moins un compartiment, la seringue et l'aiguille étant logées dans un unique compartiment de l'article manufacturé et/ou la seringue et l'aiguille étant attachées l'une à l'autre, ou où l'article manufacturé contient au moins deux compartiments, la seringue et l'aiguille étant logées dans des compartiments individuels.

15. Article manufacturé comprenant les premier et second agents thérapeutiques selon la revendication 1, dans lequel chaque agent thérapeutique est contenu dans une seringue individuelle et dans lequel l'article manufacturé comprend en plus, facultativement, une aiguille.
